# EUROPEAN PATENT APPLICATION

(11) **EP 2 620 499 A1**
(43) Date of publication of application: **31.07.2013**
(21) Application number: 11826890.3
(22) Date of filing: 22.09.2011
(51) Int. Cl.: C12N 15/29, A01H 1/00, A01H 5/00, C12N 5/10, C12Q 1/68

(54) **METHOD FOR PRODUCING CRUCIFEROUS PLANT RESISTANT TO CLUBROOT**

(30) Priority: 22.09.2010 JP 2010211689
(71) Applicant: Incorporated Administrative Agency National Agriculture And Food Research Organization, Ibaraki 305-8517 (JP)
(72) Inventor: MATSUMOTO, Satoru, Tsu-shi Mie 514-2392 (JP); HATAKEYAMA, Katsunori, Tsu-shi Mie 514-2392 (JP); FUKINO, Nobuko, Tsu-shi Mie 514-2392 (JP)
(74) Representative: Bösl, Raphael Konrad
(86) International application number: PCT/JP2011/071554
(87) International publication number: WO 2012/039445

(57) **Abstract**

Successfully produced are cruciferous plants resistant to clubroot by introducing the clubroot resistance gene (Crr1) isolated by map-based cloning into cruciferous plants and expressing the gene.

SEQUENCE LISTING

<110>  INCORPORATED ADMINISTRATIVE AGENCY NATIONAL AGRICULTURE AND
       FOOD RESEARCH ORGANIZATION

<120>  METHOD OF PRODUCING CLUBROOT RESISTANCE CRUCIFEROUS PLANTS

<130>  ARO-A1001P

<150>  JP 2010-211689
<151>  2010-09-22

<160>  13

<170>  PatentIn version 3.4

<210>  1
<211>  3749
<212>  DNA
<213>  Brassica rapa

<220>
<221>  CDS
<222>  (1)..(3675)

<400>  1
atg aaa ttt caa tcg ttt ttg aaa gaa att aag aaa cag aga ggg aag        48
Met Lys Phe Gln Ser Phe Leu Lys Glu Ile Lys Lys Gln Arg Gly Lys
1               5                   10                  15

agg aaa aga gat gaa ctt tcg aat cca gca aga aag aag atc cga gtt        96
Arg Lys Arg Asp Glu Leu Ser Asn Pro Ala Arg Lys Lys Ile Arg Val
            20                  25                  30

cag aac ctg agc caa atc caa gta cca tca tca ccc cca cct tct cct       144
Gln Asn Leu Ser Gln Ile Gln Val Pro Ser Ser Pro Pro Pro Ser Pro
        35                  40                  45

tca tca ctt cca tcc tct ttg tct ctt tca tca gct cca tct tct tcg       192
Ser Ser Leu Pro Ser Ser Leu Ser Leu Ser Ser Ala Pro Ser Ser Ser
    50                  55                  60

tct cat aac tgg aca cac gat gtc ttt cca agc ttc cgc ggg gaa gat       240
Ser His Asn Trp Thr His Asp Val Phe Pro Ser Phe Arg Gly Glu Asp
65                  70                  75                  80

gtc cgc ata ggg ttt cta agc cac att caa aag gag ttt aaa aga aaa       288
Val Arg Ile Gly Phe Leu Ser His Ile Gln Lys Glu Phe Lys Arg Lys
                85                  90                  95

gga atc aca cca ttc atc gac aat gag atc agg aga gga gaa tcc atc       336
Gly Ile Thr Pro Phe Ile Asp Asn Glu Ile Arg Arg Gly Glu Ser Ile
            100                 105                 110

ggt cca gaa ctc ata cgg gcc att aga gga tct aaa atc gcc atc gtc       384
Gly Pro Glu Leu Ile Arg Ala Ile Arg Gly Ser Lys Ile Ala Ile Val
        115                 120                 125

ttg ctc tcg agg aac tat gct tct tca aag tgg tgt ctt gat gag ttg       432
Leu Leu Ser Arg Asn Tyr Ala Ser Ser Lys Trp Cys Leu Asp Glu Leu
    130                 135                 140

gtg gag gtt atg aag tgc aaa gaa gag tta ggc caa acc gtg atc ccc       480
Val Glu Val Met Lys Cys Lys Glu Glu Leu Gly Gln Thr Val Ile Pro

145                 150                 155                 160

gtt ttc tat aaa gta gat cct tct cat gta aag aag ctg aga gga tat       528
Val Phe Tyr Lys Val Asp Pro Ser His Val Lys Lys Leu Arg Gly Tyr
                165                 170                 175

ttt ggg aaa gtt ttc gaa aaa act tgc gag ggt aaa agt aag gag gat       576
Phe Gly Lys Val Phe Glu Lys Thr Cys Glu Gly Lys Ser Lys Glu Asp
            180                 185                 190

act gag aaa tgg aga cat gct ttg gag aag gtg gcc aca att gct ggt       624
Thr Glu Lys Trp Arg His Ala Leu Glu Lys Val Ala Thr Ile Ala Gly
        195                 200                 205

tac gat tca agc acc tgg gat aat gaa gcg gcc atg att gag caa ata       672
Tyr Asp Ser Ser Thr Trp Asp Asn Glu Ala Ala Met Ile Glu Gln Ile
    210                 215                 220

gcc aca gat gtt tca aac aag ctg att agt tct gtt cca tca agt gat       720
Ala Thr Asp Val Ser Asn Lys Leu Ile Ser Ser Val Pro Ser Ser Asp
225                 230                 235                 240

ttc aac agc tta gtt ggg atg aga gct cac atg aaa agt atg gaa ctg       768
Phe Asn Ser Leu Val Gly Met Arg Ala His Met Lys Ser Met Glu Leu
                245                 250                 255

ctc tta cgc ttg gac tcc gat gaa gtg agg atg ata ggg att tgg ggt       816
Leu Leu Arg Leu Asp Ser Asp Glu Val Arg Met Ile Gly Ile Trp Gly
            260                 265                 270

ccg tct gga att ggt aag agc acc atc gcc aga tct ctc ttt agc caa       864
Pro Ser Gly Ile Gly Lys Ser Thr Ile Ala Arg Ser Leu Phe Ser Gln
        275                 280                 285

cac tct cct gac ttt caa ctt agc gtc ttc atg gag aat atc aaa aga       912
His Ser Pro Asp Phe Gln Leu Ser Val Phe Met Glu Asn Ile Lys Arg
    290                 295                 300

gag tat ccg aga cct tgt ttc gat aga tac agc gca caa gtg caa tta       960
Glu Tyr Pro Arg Pro Cys Phe Asp Arg Tyr Ser Ala Gln Val Gln Leu
305                 310                 315                 320

caa aat aag ttc ttg tct cta ata ctc aat cag aat gat gtc gct atc      1008
Gln Asn Lys Phe Leu Ser Leu Ile Leu Asn Gln Asn Asp Val Ala Ile
                325                 330                 335

cat cat tta gga gtt gca caa gac cgg cta aaa aat aag aaa gtg tta      1056
His His Leu Gly Val Ala Gln Asp Arg Leu Lys Asn Lys Lys Val Leu
            340                 345                 350

gtt gtt ctc gat gac gtg gat cac tca gcg caa cta gat gcc ttg gcg      1104
Val Val Leu Asp Asp Val Asp His Ser Ala Gln Leu Asp Ala Leu Ala
        355                 360                 365

aaa gaa act tgc tgg ttt ggc agt gga agt cgg att atc gtc acg acg      1152
Lys Glu Thr Cys Trp Phe Gly Ser Gly Ser Arg Ile Ile Val Thr Thr
    370                 375                 380

caa gat aag aaa att ttg aat gca cat cgg atc aat cat att tac gag      1200
Gln Asp Lys Lys Ile Leu Asn Ala His Arg Ile Asn His Ile Tyr Glu
385                 390                 395                 400

gtt ggt ttt cca cat gat gat gaa gct ctt gaa atc ttc tgc ata aat      1248
Val Gly Phe Pro His Asp Asp Glu Ala Leu Glu Ile Phe Cys Ile Asn
                405                 410                 415

gct ttt ggt caa aaa tcc cca tat gat ggt ttt ggg gac ctt gct cgg 1296
Ala Phe Gly Gln Lys Ser Pro Tyr Asp Gly Phe Gly Asp Leu Ala Arg
            420                 425                 430

gaa gtt aca agg ctt gtg ggt aac ctc cct ttg gga cta agt gtt atg 1344
Glu Val Thr Arg Leu Val Gly Asn Leu Pro Leu Gly Leu Ser Val Met
        435                 440                 445

gga tct tat ttc aaa ggc ttg tcc aag gag gtg tgg gaa cgt gag tta 1392
Gly Ser Tyr Phe Lys Gly Leu Ser Lys Glu Val Trp Glu Arg Glu Leu
    450                 455                 460

cca agg tta aga act aga ctt gac ggt gaa aca gaa agt att tta aag 1440
Pro Arg Leu Arg Thr Arg Leu Asp Gly Glu Thr Glu Ser Ile Leu Lys
465                 470                 475                 480

ttc agc tat gat gcc tta tgc gat gaa gat caa gct ttg ttt ctt cac 1488
Phe Ser Tyr Asp Ala Leu Cys Asp Glu Asp Gln Ala Leu Phe Leu His
                485                 490                 495

ata gcc tgc ttt ttc aac ggt gaa cgg act gac aaa gta gaa gag ttt 1536
Ile Ala Cys Phe Phe Asn Gly Glu Arg Thr Asp Lys Val Glu Glu Phe
            500                 505                 510

ctt gca gag aaa ttt gtt gct gtg gaa ggt cgt ctt cgt gtt tta gct 1584
Leu Ala Glu Lys Phe Val Ala Val Glu Gly Arg Leu Arg Val Leu Ala
        515                 520                 525

gag aaa tct ctc ata tcc gtc ggc tca gaa gga tat ata agg atg cat 1632
Glu Lys Ser Leu Ile Ser Val Gly Ser Glu Gly Tyr Ile Arg Met His
    530                 535                 540

gat ttg cta gca cgt ttg ggt agg gaa att gtt cgt aaa caa tcc cct 1680
Asp Leu Leu Ala Arg Leu Gly Arg Glu Ile Val Arg Lys Gln Ser Pro
545                 550                 555                 560

aac gaa cct ggg cag cgt cag ttt ttg gtt gat gat gga gat ata cgc 1728
Asn Glu Pro Gly Gln Arg Gln Phe Leu Val Asp Asp Gly Asp Ile Arg
                565                 570                 575

caa gta cta cgt gat gat aca ctt ggt agt cga agt gtt ata gga ata 1776
Gln Val Leu Arg Asp Asp Thr Leu Gly Ser Arg Ser Val Ile Gly Ile
            580                 585                 590

aat ttt ttg ttg aag aag aaa ttg aag ata agt gac caa gcc ttt gaa 1824
Asn Phe Leu Leu Lys Lys Lys Leu Lys Ile Ser Asp Gln Ala Phe Glu
        595                 600                 605

aga atg tcc aat ctc caa ttc tta aga ctt gat agt cag tat ttt gcc 1872
Arg Met Ser Asn Leu Gln Phe Leu Arg Leu Asp Ser Gln Tyr Phe Ala
    610                 615                 620

cag att cta ttc gaa gga aaa agt agt caa tac ata tta gaa agt gtg 1920
Gln Ile Leu Phe Glu Gly Lys Ser Ser Gln Tyr Ile Leu Glu Ser Val
625                 630                 635                 640

aac tgt cta cct cga gaa gtt aga tta cta gat tgg aga aca ttc ccg 1968
Asn Cys Leu Pro Arg Glu Val Arg Leu Leu Asp Trp Arg Thr Phe Pro
                645                 650                 655

atg aca tgt ctg cct tct gat ttt aat cca gag ctc cta atg gaa ata 2016
Met Thr Cys Leu Pro Ser Asp Phe Asn Pro Glu Leu Leu Met Glu Ile
            660                 665                 670

aaa atg att tgt agc aac ctt gag aaa ttg tgg gaa gga aat aaa acg     2064
Lys Met Ile Cys Ser Asn Leu Glu Lys Leu Trp Glu Gly Asn Lys Thr
        675                 680                 685

att aga aat ctc aag tgg atg gat ctg tct cat tcc aaa aat cta aag     2112
Ile Arg Asn Leu Lys Trp Met Asp Leu Ser His Ser Lys Asn Leu Lys
    690                 695                 700

gag ctt cct aat ctt tca act gcc aca aat ctc cga gaa ctg aat ctc     2160
Glu Leu Pro Asn Leu Ser Thr Ala Thr Asn Leu Arg Glu Leu Asn Leu
705                 710                 715                 720

ttt gga tgt tca agt ctt atg gag ctt cct tct tct att ggg aat ttg     2208
Phe Gly Cys Ser Ser Leu Met Glu Leu Pro Ser Ser Ile Gly Asn Leu
                725                 730                 735

act aat ctc aag aaa ttg aat ctc aag ctg tgc tca agc ctt atg gaa     2256
Thr Asn Leu Lys Lys Leu Asn Leu Lys Leu Cys Ser Ser Leu Met Glu
            740                 745                 750

ctc ccc tct tct att ggt aac atg act aat ctt gag aat ttg aat ctt     2304
Leu Pro Ser Ser Ile Gly Asn Met Thr Asn Leu Glu Asn Leu Asn Leu
        755                 760                 765

tct gga tgc tct agc ctt gtg gag ctc ccc tct tct att agt aat atg     2352
Ser Gly Cys Ser Ser Leu Val Glu Leu Pro Ser Ser Ile Ser Asn Met
    770                 775                 780

act aat ctt gag aat ttt aat ctc tcc caa tgc tca agc gtt gta cgg     2400
Thr Asn Leu Glu Asn Phe Asn Leu Ser Gln Cys Ser Ser Val Val Arg
785                 790                 795                 800

ctc tct ttt tct att gga aat atg acc aat ctc aag gaa ttg gag ctc     2448
Leu Ser Phe Ser Ile Gly Asn Met Thr Asn Leu Lys Glu Leu Glu Leu
                805                 810                 815

aat gaa tgc tca agc ctt gtg gag ctc acc ttt ggg aat atg act aat     2496
Asn Glu Cys Ser Ser Leu Val Glu Leu Thr Phe Gly Asn Met Thr Asn
            820                 825                 830

ctc aag aat ttg gat ccc aat aga tgc tca agc ctt gtg gag att tcc     2544
Leu Lys Asn Leu Asp Pro Asn Arg Cys Ser Ser Leu Val Glu Ile Ser
        835                 840                 845

tct tct att ggg aat atg act aat ctc gtg agg ttg gat ctc acc gga     2592
Ser Ser Ile Gly Asn Met Thr Asn Leu Val Arg Leu Asp Leu Thr Gly
    850                 855                 860

tgc tca agc ctt gtg gag ctc ccc tat tct att ggg aat atg act aat     2640
Cys Ser Ser Leu Val Glu Leu Pro Tyr Ser Ile Gly Asn Met Thr Asn
865                 870                 875                 880

ctt gaa act ttg gaa ctc tct gga tgt tca agc ctt gtg gag ctc ccc     2688
Leu Glu Thr Leu Glu Leu Ser Gly Cys Ser Ser Leu Val Glu Leu Pro
                885                 890                 895

tct tct att ggg aat ctt cat aat ttg aag cgg ttg aat ctc aga aat     2736
Ser Ser Ile Gly Asn Leu His Asn Leu Lys Arg Leu Asn Leu Arg Asn
            900                 905                 910

tgc tca acg cta atg gcc ctt cca gtg aac atc aac atg aaa tct ctt     2784
Cys Ser Thr Leu Met Ala Leu Pro Val Asn Ile Asn Met Lys Ser Leu
        915                 920                 925

gat ttt ctt gat ctc agt tac tgc tcg gtg ttg aaa agc ttt cct gag     2832

Asp Phe Leu Asp Leu Ser Tyr Cys Ser Val Leu Lys Ser Phe Pro Glu
    930                 935                 940

att tcc aca aac att atc ttt cta gga atc aaa gga act gcc att gaa      2880
Ile Ser Thr Asn Ile Ile Phe Leu Gly Ile Lys Gly Thr Ala Ile Glu
945                 950                 955                 960

gaa att cca aca tca atc agg tca tgg tct cgt ctt gat acg tta gat      2928
Glu Ile Pro Thr Ser Ile Arg Ser Trp Ser Arg Leu Asp Thr Leu Asp
                965                 970                 975

atg tca tac agt gaa aac cta agg aaa tcg cat cat gct ttt gac ctc      2976
Met Ser Tyr Ser Glu Asn Leu Arg Lys Ser His His Ala Phe Asp Leu
            980                 985                 990

atc acg aat ctt cac ttg agc gac  aca gga att caa gaa  att tct cca    3024
Ile Thr Asn Leu His Leu Ser Asp  Thr Gly Ile Gln Glu  Ile Ser Pro
        995                 1000                1005

tgg gta  aaa gaa atg tct cgt  cta cgg gaa ctt gta  atc aat gga      3069
Trp Val  Lys Glu Met Ser Arg  Leu Arg Glu Leu Val  Ile Asn Gly
    1010                1015                1020

tgc aca  aag ctg gtt tcg ctc  cca cag ctt cca gat  tca tta gaa      3114
Cys Thr  Lys Leu Val Ser Leu  Pro Gln Leu Pro Asp  Ser Leu Glu
    1025                1030                1035

ttc atg  cat gta gaa aac tgc  gag tcc ctc gag aga  cta gat agt      3159
Phe Met  His Val Glu Asn Cys  Glu Ser Leu Glu Arg  Leu Asp Ser
    1040                1045                1050

cta gat  tgc tct ttt tac agg  aca aag ttg act gat  ctt cgc ttt      3204
Leu Asp  Cys Ser Phe Tyr Arg  Thr Lys Leu Thr Asp  Leu Arg Phe
    1055                1060                1065

gtt aac  tgc ctc aaa ctg aat  cga gaa gca gta gac  ctt att ctc      3249
Val Asn  Cys Leu Lys Leu Asn  Arg Glu Ala Val Asp  Leu Ile Leu
    1070                1075                1080

aag aca  tcg aca aaa ata tgg  gcg atc ttt ccc gga  gaa tcg gtg      3294
Lys Thr  Ser Thr Lys Ile Trp  Ala Ile Phe Pro Gly  Glu Ser Val
    1085                1090                1095

cct gca  tat ttc agt tac aga  gcc acg ggg agt tca  gtg tca atg      3339
Pro Ala  Tyr Phe Ser Tyr Arg  Ala Thr Gly Ser Ser  Val Ser Met
    1100                1105                1110

aaa ctg  aat aga ttc gat aca  cgt ttt cct aca tcc  ttg aga ttt      3384
Lys Leu  Asn Arg Phe Asp Thr  Arg Phe Pro Thr Ser  Leu Arg Phe
    1115                1120                1125

aaa gct  tgc atc ttg ctg gtt  act aac cct gac gac  gtt gag cct      3429
Lys Ala  Cys Ile Leu Leu Val  Thr Asn Pro Asp Asp  Val Glu Pro
    1130                1135                1140

gct gct  tgg tac agg tcg gat  atg tct tat tgc atc  aat ggc aaa      3474
Ala Ala  Trp Tyr Arg Ser Asp  Met Ser Tyr Cys Ile  Asn Gly Lys
    1145                1150                1155

ctg agg  gat gcc ggt gtt ttc  cta gca tat act cat  ata tgg gac      3519
Leu Arg  Asp Ala Gly Val Phe  Leu Ala Tyr Thr His  Ile Trp Asp
    1160                1165                1170

cca ctc  cgt cca cgt tct gag  cat ctg gtc gta atc  gaa ttt gaa      3564
Pro Leu  Arg Pro Arg Ser Glu  His Leu Val Val Ile  Glu Phe Glu

1175                1180                1185

gaa act gtg act tcc ccc gaa tta gtc ttt gag ttc agg ttc gaa      3609
Glu Thr Val Thr Ser Pro Glu Leu Val Phe Glu Phe Arg Phe Glu
    1190                1195                1200

aaa gaa aac tgg gag att aaa gaa tgc gga cta cgt cct cta gaa      3654
Lys Glu Asn Trp Glu Ile Lys Glu Cys Gly Leu Arg Pro Leu Glu
    1205                1210                1215

agc tta gct ctc tca tgt taa tggaagctga agaaacattt cgcatgtgca      3705
Ser Leu Ala Leu Ser Cys
    1220

taaaatatag taatgcattc cgttgtttat cttaaatatc aagg                  3749

<210>  2
<211>  1224
<212>  PRT
<213>  Brassica rapa

<400>  2

Met Lys Phe Gln Ser Phe Leu Lys Glu Ile Lys Lys Gln Arg Gly Lys
1               5                   10                  15

Arg Lys Arg Asp Glu Leu Ser Asn Pro Ala Arg Lys Lys Ile Arg Val
            20                  25                  30

Gln Asn Leu Ser Gln Ile Gln Val Pro Ser Ser Pro Pro Pro Ser Pro
        35                  40                  45

Ser Ser Leu Pro Ser Ser Leu Ser Leu Ser Ser Ala Pro Ser Ser Ser
    50                  55                  60

Ser His Asn Trp Thr His Asp Val Phe Pro Ser Phe Arg Gly Glu Asp
65                  70                  75                  80

Val Arg Ile Gly Phe Leu Ser His Ile Gln Lys Glu Phe Lys Arg Lys
                85                  90                  95

Gly Ile Thr Pro Phe Ile Asp Asn Glu Ile Arg Arg Gly Glu Ser Ile
            100                 105                 110

Gly Pro Glu Leu Ile Arg Ala Ile Arg Gly Ser Lys Ile Ala Ile Val
        115                 120                 125

Leu Leu Ser Arg Asn Tyr Ala Ser Ser Lys Trp Cys Leu Asp Glu Leu
    130                 135                 140

Val Glu Val Met Lys Cys Lys Glu Glu Leu Gly Gln Thr Val Ile Pro
145                 150                 155                 160

Val Phe Tyr Lys Val Asp Pro Ser His Val Lys Lys Leu Arg Gly Tyr

                165                 170                 175

Phe Gly Lys Val Phe Glu Lys Thr Cys Glu Gly Lys Ser Lys Glu Asp
            180                 185                 190

Thr Glu Lys Trp Arg His Ala Leu Glu Lys Val Ala Thr Ile Ala Gly
        195                 200                 205

Tyr Asp Ser Ser Thr Trp Asp Asn Glu Ala Ala Met Ile Glu Gln Ile
    210                 215                 220

Ala Thr Asp Val Ser Asn Lys Leu Ile Ser Ser Val Pro Ser Ser Asp
225                 230                 235                 240

Phe Asn Ser Leu Val Gly Met Arg Ala His Met Lys Ser Met Glu Leu
                245                 250                 255

Leu Leu Arg Leu Asp Ser Asp Glu Val Arg Met Ile Gly Ile Trp Gly
            260                 265                 270

Pro Ser Gly Ile Gly Lys Ser Thr Ile Ala Arg Ser Leu Phe Ser Gln
        275                 280                 285

His Ser Pro Asp Phe Gln Leu Ser Val Phe Met Glu Asn Ile Lys Arg
    290                 295                 300

Glu Tyr Pro Arg Pro Cys Phe Asp Arg Tyr Ser Ala Gln Val Gln Leu
305                 310                 315                 320

Gln Asn Lys Phe Leu Ser Leu Ile Leu Asn Gln Asn Asp Val Ala Ile
                325                 330                 335

His His Leu Gly Val Ala Gln Asp Arg Leu Lys Asn Lys Lys Val Leu
            340                 345                 350

Val Val Leu Asp Asp Val Asp His Ser Ala Gln Leu Asp Ala Leu Ala
        355                 360                 365

Lys Glu Thr Cys Trp Phe Gly Ser Gly Ser Arg Ile Ile Val Thr Thr
    370                 375                 380

Gln Asp Lys Lys Ile Leu Asn Ala His Arg Ile Asn His Ile Tyr Glu
385                 390                 395                 400

Val Gly Phe Pro His Asp Asp Glu Ala Leu Glu Ile Phe Cys Ile Asn
                405                 410                 415

Ala Phe Gly Gln Lys Ser Pro Tyr Asp Gly Phe Gly Asp Leu Ala Arg
            420                 425                 430

Glu Val Thr Arg Leu Val Gly Asn Leu Pro Leu Gly Leu Ser Val Met
        435                 440                 445

Gly Ser Tyr Phe Lys Gly Leu Ser Lys Glu Val Trp Glu Arg Glu Leu
    450                 455                 460

Pro Arg Leu Arg Thr Arg Leu Asp Gly Glu Thr Glu Ser Ile Leu Lys
465                 470                 475                 480

Phe Ser Tyr Asp Ala Leu Cys Asp Glu Asp Gln Ala Leu Phe Leu His
                485                 490                 495

Ile Ala Cys Phe Phe Asn Gly Glu Arg Thr Asp Lys Val Glu Glu Phe
            500                 505                 510

Leu Ala Glu Lys Phe Val Ala Val Glu Gly Arg Leu Arg Val Leu Ala
        515                 520                 525

Glu Lys Ser Leu Ile Ser Val Gly Ser Glu Gly Tyr Ile Arg Met His
    530                 535                 540

Asp Leu Leu Ala Arg Leu Gly Arg Glu Ile Val Arg Lys Gln Ser Pro
545                 550                 555                 560

Asn Glu Pro Gly Gln Arg Gln Phe Leu Val Asp Asp Gly Asp Ile Arg
                565                 570                 575

Gln Val Leu Arg Asp Asp Thr Leu Gly Ser Arg Ser Val Ile Gly Ile
            580                 585                 590

Asn Phe Leu Leu Lys Lys Lys Leu Lys Ile Ser Asp Gln Ala Phe Glu
        595                 600                 605

Arg Met Ser Asn Leu Gln Phe Leu Arg Leu Asp Ser Gln Tyr Phe Ala
    610                 615                 620

Gln Ile Leu Phe Glu Gly Lys Ser Ser Gln Tyr Ile Leu Glu Ser Val
625                 630                 635                 640

Asn Cys Leu Pro Arg Glu Val Arg Leu Leu Asp Trp Arg Thr Phe Pro
                645                 650                 655

Met Thr Cys Leu Pro Ser Asp Phe Asn Pro Glu Leu Leu Met Glu Ile
            660                 665                 670

Lys Met Ile Cys Ser Asn Leu Glu Lys Leu Trp Glu Gly Asn Lys Thr
        675                 680                 685

Ile Arg Asn Leu Lys Trp Met Asp Leu Ser His Ser Lys Asn Leu Lys
    690                 695                 700

Glu Leu Pro Asn Leu Ser Thr Ala Thr Asn Leu Arg Glu Leu Asn Leu
705                 710                 715                 720

Phe Gly Cys Ser Ser Leu Met Glu Leu Pro Ser Ser Ile Gly Asn Leu
                725                 730                 735

Thr Asn Leu Lys Lys Leu Asn Leu Lys Leu Cys Ser Ser Leu Met Glu
            740                 745                 750

Leu Pro Ser Ser Ile Gly Asn Met Thr Asn Leu Glu Asn Leu Asn Leu
        755                 760                 765

Ser Gly Cys Ser Ser Leu Val Glu Leu Pro Ser Ser Ile Ser Asn Met
    770                 775                 780

Thr Asn Leu Glu Asn Phe Asn Leu Ser Gln Cys Ser Ser Val Val Arg
785                 790                 795                 800

Leu Ser Phe Ser Ile Gly Asn Met Thr Asn Leu Lys Glu Leu Glu Leu
                805                 810                 815

Asn Glu Cys Ser Ser Leu Val Glu Leu Thr Phe Gly Asn Met Thr Asn
            820                 825                 830

Leu Lys Asn Leu Asp Pro Asn Arg Cys Ser Ser Leu Val Glu Ile Ser
        835                 840                 845

Ser Ser Ile Gly Asn Met Thr Asn Leu Val Arg Leu Asp Leu Thr Gly
    850                 855                 860

Cys Ser Ser Leu Val Glu Leu Pro Tyr Ser Ile Gly Asn Met Thr Asn
865                 870                 875                 880

Leu Glu Thr Leu Glu Leu Ser Gly Cys Ser Ser Leu Val Glu Leu Pro
                885                 890                 895

Ser Ser Ile Gly Asn Leu His Asn Leu Lys Arg Leu Asn Leu Arg Asn
            900                 905                 910

Cys Ser Thr Leu Met Ala Leu Pro Val Asn Ile Asn Met Lys Ser Leu
        915                 920                 925

Asp Phe Leu Asp Leu Ser Tyr Cys Ser Val Leu Lys Ser Phe Pro Glu
    930                 935                 940

Ile Ser Thr Asn Ile Ile Phe Leu Gly Ile Lys Gly Thr Ala Ile Glu
945                 950                 955                 960

Glu Ile Pro Thr Ser Ile Arg Ser Trp Ser Arg Leu Asp Thr Leu Asp
                965                 970                 975

Met Ser Tyr Ser Glu Asn Leu Arg Lys Ser His His Ala Phe Asp Leu
            980                 985                 990

Ile Thr Asn Leu His Leu Ser Asp  Thr Gly Ile Gln Glu  Ile Ser Pro
        995                 1000                 1005

Trp Val  Lys Glu Met Ser Arg  Leu Arg Glu Leu Val  Ile Asn Gly
    1010                 1015                 1020

Cys Thr  Lys Leu Val Ser Leu  Pro Gln Leu Pro Asp  Ser Leu Glu
    1025                 1030                 1035

Phe Met  His Val Glu Asn Cys  Glu Ser Leu Glu Arg  Leu Asp Ser
    1040                 1045                 1050

Leu Asp  Cys Ser Phe Tyr Arg  Thr Lys Leu Thr Asp  Leu Arg Phe
    1055                 1060                 1065

Val Asn  Cys Leu Lys Leu Asn  Arg Glu Ala Val Asp  Leu Ile Leu
    1070                 1075                 1080

Lys Thr  Ser Thr Lys Ile Trp  Ala Ile Phe Pro Gly  Glu Ser Val
    1085                 1090                 1095

Pro Ala  Tyr Phe Ser Tyr Arg  Ala Thr Gly Ser Ser  Val Ser Met
    1100                 1105                 1110

Lys Leu  Asn Arg Phe Asp Thr  Arg Phe Pro Thr Ser  Leu Arg Phe
    1115                 1120                 1125

Lys Ala  Cys Ile Leu Leu Val  Thr Asn Pro Asp Asp  Val Glu Pro
    1130                 1135                 1140

Ala Ala  Trp Tyr Arg Ser Asp  Met Ser Tyr Cys Ile  Asn Gly Lys
    1145                 1150                 1155

Leu Arg  Asp Ala Gly Val Phe  Leu Ala Tyr Thr His  Ile Trp Asp
    1160                 1165                 1170

Pro Leu  Arg Pro Arg Ser Glu  His Leu Val Val Ile  Glu Phe Glu
    1175                 1180                 1185

Glu Thr  Val Thr Ser Pro Glu  Leu Val Phe Glu Phe  Arg Phe Glu

1190                 1195                 1200

Lys Glu  Asn Trp Glu Ile Lys  Glu Cys Gly Leu Arg  Pro Leu Glu
    1205                 1210                 1215

Ser Leu  Ala Leu Ser Cys
    1220

<210>  3
<211>  7995
<212>  DNA
<213>  Brassica rapa

<220>
<221>  exon
<222>  (2525)..(3165)

<220>
<221>  Intron
<222>  (3166)..(4652)

<220>
<221>  exon
<222>  (4653)..(5763)

<220>
<221>  Intron
<222>  (5764)..(5856)

<220>
<221>  exon
<222>  (5857)..(6165)

<220>
<221>  Intron
<222>  (6166)..(6264)

<220>
<221>  exon
<222>  (6265)..(7875)

<400>  3
accaaagaga ttgtgctgca gagattcttt tcttgggatc cttactgagc gtttttcata      60

acaaggtcat ggttggaagg tccagcgata tccctgtaag cttcatatca agcatatgaa     120

cacccgagat tattatcaac taagactacc ataattgaga atctgttttc tttttttcag     180

tggagcagga tatgtttctc ttattgagta tgctcattta actggaagac ctttctgact     240

tattgagtac atgtttcctg atgctgttga gatccaagct ttggttagga acaaggtgtt     300

tgtgtctctt tctctcaaga tggtcaccaa gagtgttgcc tatccgaaaa gtgagtccgc     360

tgaggcgtgc cttaaagggt ttatttaaag ttgattttag catcacttta tcatcgatag     420

tttcttgcgg caccatctca ttctaatgga agctgaaaaa aacaattcgc agtttgctgt     480

atctgtgtaa actactttct gtaaaacatt tcccccggtt taggacagca atacttctac     540

ttccactata acttctagaa taatgttttt ggatcaagtt taattttatg ttcctccttt     600

tcgatttatc aatgaatgga atggaacaag ttttcccttt tgtggctgcg tggtaccagt 660
atcacttcat catggcacag aattcttcat tgttaatctc aacgttggct cataggtttt 720
tttcatgcta tgtaatgtag tgggaaacat aacttgtaat tatctgtatc tacttcctct 780
atagcttctt tgatgatagc ttcatctccc ataccatact atttcatggc taactccaac 840
tcatccattg ttgataaacc tgtgtaagcc aattccatct cagtgtctcg gaatattgtt 900
ttaacttctc attctatagt tttctctgtt ccaccaaaga gaacttacca gctcttgtct 960
ttgtcaaagt attggaagtc tttgaacaag tgctcctctc aatccagtct gtatctctag 1020
taataacttc catgagttgg ttaacttcat ctacagagag tctagaaccg tgtttagcca 1080
ccctggtttt cagttctttg taattaaggg aggtacacca caaagcaaga tgtatagaat 1140
aatctctgca ctccaaatat ctctgtaaac ttattgaagc ccataaaaaa gacccattga 1200
atgatccatt aagaagcttt tgtaaccatg taactagggt ttatagacca ttataaatat 1260
gtccttagat gttctttggt tgtaactttg gctgctataa tagcagcctc tccctttcca 1320
ttatatgaaa tcaaatccaa cgtttatctc ctggtgacaa cttaatctct tcctctcttt 1380
agtttctagc atttcaataa agctttctta agctatcttt catgtgtcaa taaagcttag 1440
caaagctata atcttttatg tttctcaaac gttatcaaaa ctttccctgc aatttgtttt 1500
tcagtttata aacaacacat aaagcttatg aatacacagt tatcaactaa caaaaaagaa 1560
aacacaaggt ctacacaagt aattactcac attgccaact tgcaaagcct ctttttgaag 1620
attaccacta tgtactgaac taataagact ctcaagttat atatggatca aacctgaaaa 1680
taaacattgg ttataatctt aagaaatcta aaacatagca tttacatata tcttataacc 1740
agaagagttg tatgtatagc gttctcaact tctccaatga aaacagacaa tccgaagtca 1800
gtagctttga gcatagcgtt ctcatcttta ctaaagagca agaagttctc aggcttgatc 1860
tacaatccta tcggacaatt cacttccacc accacacatt tccattacaa gacgaatgga 1920
ctgtctagat gatgatgacc agatgttcat gaacattatt ttttatgaag aatgtacttt 1980
ttgaatgaat atgaaaacca aacgaagccc aaacgtttta aaataaaaga ttggtatgag 2040
taggaggaga aatatataaa aaagcttcta gagaagattg aatgatctct ttgctgtgaa 2100
attttcaagt gcgaactcat cagttctgat cactgcttct ttgaaattgc ttgtgctctt 2160
ccttgcattt tccttcttag aatagtataa gccaaaaggg aatggtggga ctttatgcac 2220
cttatttttc gttggtgaca tatttttcca caaaagtctt tgtaagtcgg ccgagtcaac 2280
gttttatgac atatacgcaa ttacgcatac aaaattgtca caagtctctt tggttcatta 2340
ttgcgttaca tatcttcttt agaaatattc ttggtccctc tctctctacc acacagattg 2400
attatccctt ctaatcatcc cctctacaat tcttgctgta tgcttcagat tccatcaaga 2460
aaaccaagaa actgattttc tttttttttc aacttcggta aaaaaaaaag gcagcttttt 2520

gaaa atg aaa ttt caa tcg ttt ttg aaa gaa att aag aaa cag aga ggg 2569
Met Lys Phe Gln Ser Phe Leu Lys Glu Ile Lys Lys Gln Arg Gly
1               5                   10                  15

aag agg aaa aga gat gaa ctt tcg aat cca gca aga aag aag atc cga 2617
Lys Arg Lys Arg Asp Glu Leu Ser Asn Pro Ala Arg Lys Lys Ile Arg
                20                  25                  30

gtt cag aac ctg agc caa atc caa gta cca tca tca ccc cca cct tct 2665
Val Gln Asn Leu Ser Gln Ile Gln Val Pro Ser Ser Pro Pro Pro Ser
            35                  40                  45

cct tca tca ctt cca tcc tct ttg tct ctt tca tca gct cca tct tct 2713
Pro Ser Ser Leu Pro Ser Ser Leu Ser Leu Ser Ser Ala Pro Ser Ser
        50                  55                  60

tcg tct cat aac tgg aca cac gat gtc ttt cca agc ttc cgc ggg gaa 2761
Ser Ser His Asn Trp Thr His Asp Val Phe Pro Ser Phe Arg Gly Glu
    65                  70                  75

gat gtc cgc ata ggg ttt cta agc cac att caa aag gag ttt aaa aga 2809
Asp Val Arg Ile Gly Phe Leu Ser His Ile Gln Lys Glu Phe Lys Arg
80                  85                  90                  95

aaa gga atc aca cca ttc atc gac aat gag atc agg aga gga gaa tcc 2857
Lys Gly Ile Thr Pro Phe Ile Asp Asn Glu Ile Arg Arg Gly Glu Ser
                100                 105                 110

atc ggt cca gaa ctc ata cgg gcc att aga gga tct aaa atc gcc atc 2905
Ile Gly Pro Glu Leu Ile Arg Ala Ile Arg Gly Ser Lys Ile Ala Ile
            115                 120                 125

gtc ttg ctc tcg agg aac tat gct tct tca aag tgg tgt ctt gat gag 2953
Val Leu Leu Ser Arg Asn Tyr Ala Ser Ser Lys Trp Cys Leu Asp Glu
        130                 135                 140

ttg gtg gag gtt atg aag tgc aaa gaa gag tta ggc caa acc gtg atc 3001
Leu Val Glu Val Met Lys Cys Lys Glu Glu Leu Gly Gln Thr Val Ile
    145                 150                 155

ccc gtt ttc tat aaa gta gat cct tct cat gta aag aag ctg aga gga 3049
Pro Val Phe Tyr Lys Val Asp Pro Ser His Val Lys Lys Leu Arg Gly
160                 165                 170                 175

tat ttt ggg aaa gtt ttc gaa aaa act tgc gag ggt aaa agt aag gag 3097
Tyr Phe Gly Lys Val Phe Glu Lys Thr Cys Glu Gly Lys Ser Lys Glu
                180                 185                 190

gat act gag aaa tgg aga cat gct ttg gag aag gtg gcc aca att gct 3145
Asp Thr Glu Lys Trp Arg His Ala Leu Glu Lys Val Ala Thr Ile Ala
            195                 200                 205

ggt tac gat tca agc acc tg gtttgtcctt ttctattttt ctttttattt 3195
Gly Tyr Asp Ser Ser Thr Trp
        210

ctaatctttc aaactcatac aaatctaact caaaaatacc aaatgatcac agttttttcc 3255

tcttaatttt tacttgctct ttatatgcat caatatataa tatcaatgtt aaatctcaaa 3315

atataacaaa aaggtgttag ttccaaaaat tcaaaaactt gtgaaaactt atacggaaaa 3375

aagtttccta gctacacgaa gtttacacta atatggtgga aaccatccag tgtatgagaa 3435

tgtgtcaaca actacacgaa gtttatgtta atacatgtca catgtacgaa ctaaataata 3495

atatggtggc aacatcacaa tcggttttaa ttgattttaa actaagttaa tggggtcaat 3555

ccgtaattta ctaaacttaa taaatttaat attttttttt aattttaata aacttaattg 3615

attttgttaa taaacttaat aattaattaa acttacttaa taaacttaat aaatttacta 3675

aacttataaa aattaataaa tttattaaac ttataaaaat taataaattt attaaactta 3735

ataaaattaa tcatttatta ataaacttaa tcatttatta ataaatttaa taaaattaat 3795

catttattaa taaacttaat catttaataa acttaataaa cctaataaat ttgttaaatt 3855

tatcaagttt aataagttta ttaagtttaa taaatttatt tttattaagt ttaagtttat 3915

taagtttatt aaattattaa gtttattaag tttaatgaat ttattaagta taataaatta 3975

ttaagtttat taagttgaat tttattaaat taaaaagttt attaataaaa tcaattaagt 4035

ttattaaact taataaacca ttaagtttat taagtttatt aaattataaa gtttattaag 4095

tttaataaat ttattaagta taataaatta ttaagttttt taagttaaag tttattaaat 4155

tactaagttt attaataaaa tcaatttaag tttattaaac ttaataaatc attaagttta 4215

ttaagtttag taaattacag attgacccca ttaacttagc ttataatcaa ttaaacccga 4275

ttgtgatgtt gccaccatat tattatttac ttcgtacatg taacatgtat taacataaac 4335

ttcgtgtagt tgttgacaca ttttcatata ttggatagtt tccaccatat tagtgtaaac 4395

ttcgtgtagc tatgaaactt ttttccaact tataccattc tctaaatttt gaacatgttt 4455

ggttatttaa tgaagagata ttgggagcgt gaattcaaat agagttgctt aatttttaat 4515

ttaagagttt tgtgtattta acatcatcca aaaacatgca atgtgacatt cttgagaatt 4575

gcacacagca tatttccagc caaacaattc ttatttggaa accgtttgag ccgatgtatg 4635

ttctttcctc tgtttag g gat aat gaa gcg gcc atg att gag caa ata gcc 4686
Asp Asn Glu Ala Ala Met Ile Glu Gln Ile Ala
215                 220                 225

aca gat gtt tca aac aag ctg att agt tct gtt cca tca agt gat ttc 4734
Thr Asp Val Ser Asn Lys Leu Ile Ser Ser Val Pro Ser Ser Asp Phe
                230                 235                 240

aac agc tta gtt ggg atg aga gct cac atg aaa agt atg gaa ctg ctc 4782
Asn Ser Leu Val Gly Met Arg Ala His Met Lys Ser Met Glu Leu Leu
            245                 250                 255

tta cgc ttg gac tcc gat gaa gtg agg atg ata ggg att tgg ggt ccg 4830
Leu Arg Leu Asp Ser Asp Glu Val Arg Met Ile Gly Ile Trp Gly Pro
        260                 265                 270

tct gga att ggt aag agc acc atc gcc aga tct ctc ttt agc caa cac 4878
Ser Gly Ile Gly Lys Ser Thr Ile Ala Arg Ser Leu Phe Ser Gln His
    275                 280                 285

tct cct gac ttt caa ctt agc gtc ttc atg gag aat atc aaa aga gag 4926
Ser Pro Asp Phe Gln Leu Ser Val Phe Met Glu Asn Ile Lys Arg Glu
290                 295                 300                 305

tat ccg aga cct tgt ttc gat aga tac agc gca caa gtg caa tta caa 4974
Tyr Pro Arg Pro Cys Phe Asp Arg Tyr Ser Ala Gln Val Gln Leu Gln

                310                 315                 320

aat aag ttc ttg tct cta ata ctc aat cag aat gat gtc gct atc cat 5022
Asn Lys Phe Leu Ser Leu Ile Leu Asn Gln Asn Asp Val Ala Ile His
            325                 330                 335

cat tta gga gtt gca caa gac cgg cta aaa aat aag aaa gtg tta gtt 5070
His Leu Gly Val Ala Gln Asp Arg Leu Lys Asn Lys Lys Val Leu Val
        340                 345                 350

gtt ctc gat gac gtg gat cac tca gcg caa cta gat gcc ttg gcg aaa 5118
Val Leu Asp Asp Val Asp His Ser Ala Gln Leu Asp Ala Leu Ala Lys
    355                 360                 365

gaa act tgc tgg ttt ggc agt gga agt cgg att atc gtc acg acg caa 5166
Glu Thr Cys Trp Phe Gly Ser Gly Ser Arg Ile Ile Val Thr Thr Gln
370                 375                 380                 385

gat aag aaa att ttg aat gca cat cgg atc aat cat att tac gag gtt 5214
Asp Lys Lys Ile Leu Asn Ala His Arg Ile Asn His Ile Tyr Glu Val
                390                 395                 400

ggt ttt cca cat gat gat gaa gct ctt gaa atc ttc tgc ata aat gct 5262
Gly Phe Pro His Asp Asp Glu Ala Leu Glu Ile Phe Cys Ile Asn Ala
            405                 410                 415

ttt ggt caa aaa tcc cca tat gat ggt ttt ggg gac ctt gct cgg gaa 5310
Phe Gly Gln Lys Ser Pro Tyr Asp Gly Phe Gly Asp Leu Ala Arg Glu
        420                 425                 430

gtt aca agg ctt gtg ggt aac ctc cct ttg gga cta agt gtt atg gga 5358
Val Thr Arg Leu Val Gly Asn Leu Pro Leu Gly Leu Ser Val Met Gly
    435                 440                 445

tct tat ttc aaa ggc ttg tcc aag gag gtg tgg gaa cgt gag tta cca 5406
Ser Tyr Phe Lys Gly Leu Ser Lys Glu Val Trp Glu Arg Glu Leu Pro
450                 455                 460                 465

agg tta aga act aga ctt gac ggt gaa aca gaa agt att tta aag ttc 5454
Arg Leu Arg Thr Arg Leu Asp Gly Glu Thr Glu Ser Ile Leu Lys Phe
                470                 475                 480

agc tat gat gcc tta tgc gat gaa gat caa gct ttg ttt ctt cac ata 5502
Ser Tyr Asp Ala Leu Cys Asp Glu Asp Gln Ala Leu Phe Leu His Ile
            485                 490                 495

gcc tgc ttt ttc aac ggt gaa cgg act gac aaa gta gaa gag ttt ctt 5550
Ala Cys Phe Phe Asn Gly Glu Arg Thr Asp Lys Val Glu Glu Phe Leu
        500                 505                 510

gca gag aaa ttt gtt gct gtg gaa ggt cgt ctt cgt gtt tta gct gag 5598
Ala Glu Lys Phe Val Ala Val Glu Gly Arg Leu Arg Val Leu Ala Glu
    515                 520                 525

aaa tct ctc ata tcc gtc ggc tca gaa gga tat ata agg atg cat gat 5646
Lys Ser Leu Ile Ser Val Gly Ser Glu Gly Tyr Ile Arg Met His Asp
530                 535                 540                 545

ttg cta gca cgt ttg ggt agg gaa att gtt cgt aaa caa tcc cct aac 5694
Leu Leu Ala Arg Leu Gly Arg Glu Ile Val Arg Lys Gln Ser Pro Asn
                550                 555                 560

gaa cct ggg cag cgt cag ttt ttg gtt gat gat gga gat ata cgc caa 5742
Glu Pro Gly Gln Arg Gln Phe Leu Val Asp Asp Gly Asp Ile Arg Gln
            565                 570                 575

gta cta cgt gat gat aca ctt gtaagttttc acgttggttg ttctcagcat 5793
Val Leu Arg Asp Asp Thr Leu
        580

ttctcctaga aaatgttata acaaatcatg tttgcttatg tatctttggg tttttttttt 5853

cag ggt agt cga agt gtt ata gga ata aat ttt ttg ttg aag aag aaa 5901
Gly Ser Arg Ser Val Ile Gly Ile Asn Phe Leu Leu Lys Lys Lys
585                 590                 595

ttg aag ata agt gac caa gcc ttt gaa aga atg tcc aat ctc caa ttc 5949
Leu Lys Ile Ser Asp Gln Ala Phe Glu Arg Met Ser Asn Leu Gln Phe
600                 605                 610                 615

tta aga ctt gat agt cag tat ttt gcc cag att cta ttc gaa gga aaa 5997
Leu Arg Leu Asp Ser Gln Tyr Phe Ala Gln Ile Leu Phe Glu Gly Lys
                620                 625                 630

agt agt caa tac ata tta gaa agt gtg aac tgt cta cct cga gaa gtt 6045
Ser Ser Gln Tyr Ile Leu Glu Ser Val Asn Cys Leu Pro Arg Glu Val
            635                 640                 645

aga tta cta gat tgg aga aca ttc ccg atg aca tgt ctg cct tct gat 6093
Arg Leu Leu Asp Trp Arg Thr Phe Pro Met Thr Cys Leu Pro Ser Asp
        650                 655                 660

ttt aat cca gag ctc cta atg gaa ata aaa atg att tgt agc aac ctt 6141
Phe Asn Pro Glu Leu Leu Met Glu Ile Lys Met Ile Cys Ser Asn Leu
    665                 670                 675

gag aaa ttg tgg gaa gga aat aaa gtaagtacag atatatatat atatatatat 6195
Glu Lys Leu Trp Glu Gly Asn Lys
680                 685

atttcaaaat aatatgcttt agctgaggat cgtctttttc ttaatgttgc attttgttta 6255

tctgtacag acg att aga aat ctc aag tgg atg gat ctg tct cat tcc aaa 6306
Thr Ile Arg Asn Leu Lys Trp Met Asp Leu Ser His Ser Lys
        690                 695                 700

aat cta aag gag ctt cct aat ctt tca act gcc aca aat ctc cga gaa 6354
Asn Leu Lys Glu Leu Pro Asn Leu Ser Thr Ala Thr Asn Leu Arg Glu
            705                 710                 715

ctg aat ctc ttt gga tgt tca agt ctt atg gag ctt cct tct tct att 6402
Leu Asn Leu Phe Gly Cys Ser Ser Leu Met Glu Leu Pro Ser Ser Ile
        720                 725                 730

ggg aat ttg act aat ctc aag aaa ttg aat ctc aag ctg tgc tca agc 6450
Gly Asn Leu Thr Asn Leu Lys Lys Leu Asn Leu Lys Leu Cys Ser Ser
    735                 740                 745

ctt atg gaa ctc ccc tct tct att ggt aac atg act aat ctt gag aat 6498
Leu Met Glu Leu Pro Ser Ser Ile Gly Asn Met Thr Asn Leu Glu Asn
750                 755                 760                 765

ttg aat ctt tct gga tgc tct agc ctt gtg gag ctc ccc tct tct att 6546
Leu Asn Leu Ser Gly Cys Ser Ser Leu Val Glu Leu Pro Ser Ser Ile
                770                 775                 780

agt aat atg act aat ctt gag aat ttt aat ctc tcc caa tgc tca agc 6594
Ser Asn Met Thr Asn Leu Glu Asn Phe Asn Leu Ser Gln Cys Ser Ser
            785                 790                 795

gtt gta cgg ctc tct ttt tct att gga aat atg acc aat ctc aag gaa     6642
Val Val Arg Leu Ser Phe Ser Ile Gly Asn Met Thr Asn Leu Lys Glu
        800                 805                 810

ttg gag ctc aat gaa tgc tca agc ctt gtg gag ctc acc ttt ggg aat     6690
Leu Glu Leu Asn Glu Cys Ser Ser Leu Val Glu Leu Thr Phe Gly Asn
    815                 820                 825

atg act aat ctc aag aat ttg gat ccc aat aga tgc tca agc ctt gtg     6738
Met Thr Asn Leu Lys Asn Leu Asp Pro Asn Arg Cys Ser Ser Leu Val
830                 835                 840                 845

gag att tcc tct tct att ggg aat atg act aat ctc gtg agg ttg gat     6786
Glu Ile Ser Ser Ser Ile Gly Asn Met Thr Asn Leu Val Arg Leu Asp
                850                 855                 860

ctc acc gga tgc tca agc ctt gtg gag ctc ccc tat tct att ggg aat     6834
Leu Thr Gly Cys Ser Ser Leu Val Glu Leu Pro Tyr Ser Ile Gly Asn
            865                 870                 875

atg act aat ctt gaa act ttg gaa ctc tct gga tgt tca agc ctt gtg     6882
Met Thr Asn Leu Glu Thr Leu Glu Leu Ser Gly Cys Ser Ser Leu Val
        880                 885                 890

gag ctc ccc tct tct att ggg aat ctt cat aat ttg aag cgg ttg aat     6930
Glu Leu Pro Ser Ser Ile Gly Asn Leu His Asn Leu Lys Arg Leu Asn
    895                 900                 905

ctc aga aat tgc tca acg cta atg gcc ctt cca gtg aac atc aac atg     6978
Leu Arg Asn Cys Ser Thr Leu Met Ala Leu Pro Val Asn Ile Asn Met
910                 915                 920                 925

aaa tct ctt gat ttt ctt gat ctc agt tac tgc tcg gtg ttg aaa agc     7026
Lys Ser Leu Asp Phe Leu Asp Leu Ser Tyr Cys Ser Val Leu Lys Ser
                930                 935                 940

ttt cct gag att tcc aca aac att atc ttt cta gga atc aaa gga act     7074
Phe Pro Glu Ile Ser Thr Asn Ile Ile Phe Leu Gly Ile Lys Gly Thr
            945                 950                 955

gcc att gaa gaa att cca aca tca atc agg tca tgg tct cgt ctt gat     7122
Ala Ile Glu Glu Ile Pro Thr Ser Ile Arg Ser Trp Ser Arg Leu Asp
        960                 965                 970

acg tta gat atg tca tac agt gaa aac cta agg aaa tcg cat cat gct     7170
Thr Leu Asp Met Ser Tyr Ser Glu Asn Leu Arg Lys Ser His His Ala
    975                 980                 985

ttt gac ctc atc acg aat ctt cac ttg agc gac  aca gga att caa gaa     7218
Phe Asp Leu Ile Thr Asn Leu His Leu Ser Asp  Thr Gly Ile Gln Glu
990                 995                 1000                1005

att tct cca tgg gta  aaa gaa atg tct cgt  cta cgg gaa ctt gta     7263
Ile Ser Pro Trp Val  Lys Glu Met Ser Arg  Leu Arg Glu Leu Val
                1010                1015                1020

atc aat gga tgc aca  aag ctg gtt tcg ctc  cca cag ctt cca gat     7308
Ile Asn Gly Cys Thr  Lys Leu Val Ser Leu  Pro Gln Leu Pro Asp
                1025                1030                1035

tca tta gaa ttc atg  cat gta gaa aac tgc  gag tcc ctc gag aga     7353
Ser Leu Glu Phe Met  His Val Glu Asn Cys  Glu Ser Leu Glu Arg
                1040                1045                1050

cta gat agt cta gat  tgc tct ttt tac agg  aca aag ttg act gat     7398

Leu Asp Ser Leu Asp Cys Ser Phe Tyr Arg Thr Lys Leu Thr Asp
                1055                1060                1065

ctt cgc ttt gtt aac tgc ctc aaa ctg aat cga gaa gca gta gac 7443
Leu Arg Phe Val Asn Cys Leu Lys Leu Asn Arg Glu Ala Val Asp
                1070                1075                1080

ctt att ctc aag aca tcg aca aaa ata tgg gcg atc ttt ccc gga 7488
Leu Ile Leu Lys Thr Ser Thr Lys Ile Trp Ala Ile Phe Pro Gly
                1085                1090                1095

gaa tcg gtg cct gca tat ttc agt tac aga gcc acg ggg agt tca 7533
Glu Ser Val Pro Ala Tyr Phe Ser Tyr Arg Ala Thr Gly Ser Ser
                1100                1105                1110

gtg tca atg aaa ctg aat aga ttc gat aca cgt ttt cct aca tcc 7578
Val Ser Met Lys Leu Asn Arg Phe Asp Thr Arg Phe Pro Thr Ser
                1115                1120                1125

ttg aga ttt aaa gct tgc atc ttg ctg gtt act aac cct gac gac 7623
Leu Arg Phe Lys Ala Cys Ile Leu Leu Val Thr Asn Pro Asp Asp
                1130                1135                1140

gtt gag cct gct gct tgg tac agg tcg gat atg tct tat tgc atc 7668
Val Glu Pro Ala Ala Trp Tyr Arg Ser Asp Met Ser Tyr Cys Ile
                1145                1150                1155

aat ggc aaa ctg agg gat gcc ggt gtt ttc cta gca tat act cat 7713
Asn Gly Lys Leu Arg Asp Ala Gly Val Phe Leu Ala Tyr Thr His
                1160                1165                1170

ata tgg gac cca ctc cgt cca cgt tct gag cat ctg gtc gta atc 7758
Ile Trp Asp Pro Leu Arg Pro Arg Ser Glu His Leu Val Val Ile
                1175                1180                1185

gaa ttt gaa gaa act gtg act tcc ccc gaa tta gtc ttt gag ttc 7803
Glu Phe Glu Glu Thr Val Thr Ser Pro Glu Leu Val Phe Glu Phe
                1190                1195                1200

agg ttc gaa aaa gaa aac tgg gag att aaa gaa tgc gga cta cgt 7848
Arg Phe Glu Lys Glu Asn Trp Glu Ile Lys Glu Cys Gly Leu Arg
                1205                1210                1215

cct cta gaa agc tta gct ctc tca tgt taatggaagc tgaagaaaca 7895
Pro Leu Glu Ser Leu Ala Leu Ser Cys
                1220

tttcgcatgt gcataaaata tagtaatgca ttccgttgtt tatcttaaat atcaaggaaa 7955

caatttttat tagtaatctt tgtttcctac tcgttgagtg 7995

<210> 4
<211> 214
<212> PRT
<213> Brassica rapa

<400> 4

Met Lys Phe Gln Ser Phe Leu Lys Glu Ile Lys Lys Gln Arg Gly Lys
1               5                   10                  15

Arg Lys Arg Asp Glu Leu Ser Asn Pro Ala Arg Lys Lys Ile Arg Val
            20                  25                  30

Gln Asn Leu Ser Gln Ile Gln Val Pro Ser Ser Pro Pro Pro Ser Pro
        35                  40                  45

Ser Ser Leu Pro Ser Ser Leu Ser Leu Ser Ser Ala Pro Ser Ser Ser
    50                  55                  60

Ser His Asn Trp Thr His Asp Val Phe Pro Ser Phe Arg Gly Glu Asp
65                  70                  75                  80

Val Arg Ile Gly Phe Leu Ser His Ile Gln Lys Glu Phe Lys Arg Lys
                85                  90                  95

Gly Ile Thr Pro Phe Ile Asp Asn Glu Ile Arg Arg Gly Glu Ser Ile
            100                 105                 110

Gly Pro Glu Leu Ile Arg Ala Ile Arg Gly Ser Lys Ile Ala Ile Val
        115                 120                 125

Leu Leu Ser Arg Asn Tyr Ala Ser Ser Lys Trp Cys Leu Asp Glu Leu
    130                 135                 140

Val Glu Val Met Lys Cys Lys Glu Glu Leu Gly Gln Thr Val Ile Pro
145                 150                 155                 160

Val Phe Tyr Lys Val Asp Pro Ser His Val Lys Lys Leu Arg Gly Tyr
                165                 170                 175

Phe Gly Lys Val Phe Glu Lys Thr Cys Glu Gly Lys Ser Lys Glu Asp
            180                 185                 190

Thr Glu Lys Trp Arg His Ala Leu Glu Lys Val Ala Thr Ile Ala Gly
        195                 200                 205

Tyr Asp Ser Ser Thr Trp
    210

<210>  5
<211>  341
<212>  PRT
<213>  Brassica rapa

<400>  5

Asp Asn Glu Ala Ala Met Ile Glu Gln Ile Ala Thr Asp Val Ser Asn
1               5                   10                  15

Lys Leu Ile Ser Ser Val Pro Ser Ser Asp Phe Asn Ser Leu Val Gly
            20                  25                  30

Met Arg Ala His Met Lys Ser Met Glu Leu Leu Leu Arg Leu Asp Ser
        35                  40                  45

Asp Glu Val Arg Met Ile Gly Ile Trp Gly Pro Ser Gly Ile Gly Lys
    50                  55                  60

Ser Thr Ile Ala Arg Ser Leu Phe Ser Gln His Ser Pro Asp Phe Gln
65                  70                  75                  80

Leu Ser Val Phe Met Glu Asn Ile Lys Arg Glu Tyr Pro Arg Pro Cys
                85                  90                  95

Phe Asp Arg Tyr Ser Ala Gln Val Gln Leu Gln Asn Lys Phe Leu Ser
            100                 105                 110

Leu Ile Leu Asn Gln Asn Asp Val Ala Ile His His Leu Gly Val Ala
        115                 120                 125

Gln Asp Arg Leu Lys Asn Lys Lys Val Leu Val Val Leu Asp Asp Val
    130                 135                 140

Asp His Ser Ala Gln Leu Asp Ala Leu Ala Lys Glu Thr Cys Trp Phe
145                 150                 155                 160

Gly Ser Gly Ser Arg Ile Ile Val Thr Thr Gln Asp Lys Lys Ile Leu
                165                 170                 175

Asn Ala His Arg Ile Asn His Ile Tyr Glu Val Gly Phe Pro His Asp
            180                 185                 190

Asp Glu Ala Leu Glu Ile Phe Cys Ile Asn Ala Phe Gly Gln Lys Ser
        195                 200                 205

Pro Tyr Asp Gly Phe Gly Asp Leu Ala Arg Glu Val Thr Arg Leu Val
    210                 215                 220

Gly Asn Leu Pro Leu Gly Leu Ser Val Met Gly Ser Tyr Phe Lys Gly
225                 230                 235                 240

Leu Ser Lys Glu Val Trp Glu Arg Glu Leu Pro Arg Leu Arg Thr Arg
                245                 250                 255

Leu Asp Gly Glu Thr Glu Ser Ile Leu Lys Phe Ser Tyr Asp Ala Leu
            260                 265                 270

Cys Asp Glu Asp Gln Ala Leu Phe Leu His Ile Ala Cys Phe Phe Asn
        275                 280                 285

Gly Glu Arg Thr Asp Lys Val Glu Glu Phe Leu Ala Glu Lys Phe Val

290                 295                 300

Ala Val Glu Gly Arg Leu Arg Val Leu Ala Glu Lys Ser Leu Ile Ser
305                 310                 315                 320

Val Gly Ser Glu Gly Tyr Ile Arg Met His Asp Leu Leu Ala Arg Leu
                325                 330                 335

Gly Arg Glu Ile Val
            340

<210>  6
<211>  106
<212>  PRT
<213>  Brassica rapa

<400>  6

Arg Lys Gln Ser Pro Asn Glu Pro Gly Gln Arg Gln Phe Leu Val Asp
1               5                   10                  15

Asp Gly Asp Ile Arg Gln Val Leu Arg Asp Asp Thr Leu Gly Ser Arg
            20                  25                  30

Ser Val Ile Gly Ile Asn Phe Leu Leu Lys Lys Lys Leu Lys Ile Ser
        35                  40                  45

Asp Gln Ala Phe Glu Arg Met Ser Asn Leu Gln Phe Leu Arg Leu Asp
    50                  55                  60

Ser Gln Tyr Phe Ala Gln Ile Leu Phe Glu Gly Lys Ser Ser Gln Tyr
65                  70                  75                  80

Ile Leu Glu Ser Val Asn Cys Leu Pro Arg Glu Val Arg Leu Leu Asp
                85                  90                  95

Trp Arg Thr Phe Pro Met Thr Cys Leu Pro
            100                 105

<210>  7
<211>  284
<212>  PRT
<213>  Brassica rapa

<400>  7

Ser Asp Phe Asn Pro Glu Leu Leu Met Glu Ile Lys Met Ile Cys Ser
1               5                   10                  15

Asn Leu Glu Lys Leu Trp Glu Gly Asn Lys Thr Ile Arg Asn Leu Lys
            20                  25                  30

Trp Met Asp Leu Ser His Ser Lys Asn Leu Lys Glu Leu Pro Asn Leu
        35                  40                  45

Ser Thr Ala Thr Asn Leu Arg Glu Leu Asn Leu Phe Gly Cys Ser Ser
    50                  55                  60

Leu Met Glu Leu Pro Ser Ser Ile Gly Asn Leu Thr Asn Leu Lys Lys
65                  70                  75                  80

Leu Asn Leu Lys Leu Cys Ser Ser Leu Met Glu Leu Pro Ser Ser Ile
                85                  90                  95

Gly Asn Met Thr Asn Leu Glu Asn Leu Asn Leu Ser Gly Cys Ser Ser
            100                 105                 110

Leu Val Glu Leu Pro Ser Ser Ile Ser Asn Met Thr Asn Leu Glu Asn
        115                 120                 125

Phe Asn Leu Ser Gln Cys Ser Ser Val Val Arg Leu Ser Phe Ser Ile
    130                 135                 140

Gly Asn Met Thr Asn Leu Lys Glu Leu Glu Leu Asn Glu Cys Ser Ser
145                 150                 155                 160

Leu Val Glu Leu Thr Phe Gly Asn Met Thr Asn Leu Lys Asn Leu Asp
                165                 170                 175

Pro Asn Arg Cys Ser Ser Leu Val Glu Ile Ser Ser Ser Ile Gly Asn
            180                 185                 190

Met Thr Asn Leu Val Arg Leu Asp Leu Thr Gly Cys Ser Ser Leu Val
        195                 200                 205

Glu Leu Pro Tyr Ser Ile Gly Asn Met Thr Asn Leu Glu Thr Leu Glu
    210                 215                 220

Leu Ser Gly Cys Ser Ser Leu Val Glu Leu Pro Ser Ser Ile Gly Asn
225                 230                 235                 240

Leu His Asn Leu Lys Arg Leu Asn Leu Arg Asn Cys Ser Thr Leu Met
                245                 250                 255

Ala Leu Pro Val Asn Ile Asn Met Lys Ser Leu Asp Phe Leu Asp Leu
            260                 265                 270

Ser Tyr Cys Ser Val Leu Lys Ser Phe Pro Glu Ile
        275                 280

<210>  8

<211>  279
<212>  PRT
<213>  Brassica rapa

<400>  8

Ser Thr Asn Ile Ile Phe Leu Gly Ile Lys Gly Thr Ala Ile Glu Glu
1               5                   10                  15

Ile Pro Thr Ser Ile Arg Ser Trp Ser Arg Leu Asp Thr Leu Asp Met
            20                  25                  30

Ser Tyr Ser Glu Asn Leu Arg Lys Ser His His Ala Phe Asp Leu Ile
        35                  40                  45

Thr Asn Leu His Leu Ser Asp Thr Gly Ile Gln Glu Ile Ser Pro Trp
    50                  55                  60

Val Lys Glu Met Ser Arg Leu Arg Glu Leu Val Ile Asn Gly Cys Thr
65                  70                  75                  80

Lys Leu Val Ser Leu Pro Gln Leu Pro Asp Ser Leu Glu Phe Met His
                85                  90                  95

Val Glu Asn Cys Glu Ser Leu Glu Arg Leu Asp Ser Leu Asp Cys Ser
            100                 105                 110

Phe Tyr Arg Thr Lys Leu Thr Asp Leu Arg Phe Val Asn Cys Leu Lys
        115                 120                 125

Leu Asn Arg Glu Ala Val Asp Leu Ile Leu Lys Thr Ser Thr Lys Ile
    130                 135                 140

Trp Ala Ile Phe Pro Gly Glu Ser Val Pro Ala Tyr Phe Ser Tyr Arg
145                 150                 155                 160

Ala Thr Gly Ser Ser Val Ser Met Lys Leu Asn Arg Phe Asp Thr Arg
                165                 170                 175

Phe Pro Thr Ser Leu Arg Phe Lys Ala Cys Ile Leu Leu Val Thr Asn
            180                 185                 190

Pro Asp Asp Val Glu Pro Ala Ala Trp Tyr Arg Ser Asp Met Ser Tyr
        195                 200                 205

Cys Ile Asn Gly Lys Leu Arg Asp Ala Gly Val Phe Leu Ala Tyr Thr
    210                 215                 220

His Ile Trp Asp Pro Leu Arg Pro Arg Ser Glu His Leu Val Val Ile
225                 230                 235                 240

Glu Phe Glu Glu Thr Val Thr Ser Pro Glu Leu Val Phe Glu Phe Arg
                245                 250                 255

Phe Glu Lys Glu Asn Trp Glu Ile Lys Glu Cys Gly Leu Arg Pro Leu
            260                 265                 270

Glu Ser Leu Ala Leu Ser Cys
        275

<210>  9
<211>  33
<212>  DNA
<213>  Artificial

<220>
<223>  artificially synthesized primer sequence

<400>  9
tcccccggga aaatgaaatt tcaatcgttt ttg                                    33

<210>  10
<211>  29
<212>  DNA
<213>  Artificial

<220>
<223>  artificially synthesized primer sequence

<400>  10
ccttgatatt taagataaac aacggaatg                                         29

<210>  11
<211>  24
<212>  DNA
<213>  Artificial

<220>
<223>  artificially synthesized primer sequence

<400>  11
ctctctcatg ttaatggaag ctga                                              24

<210>  12
<211>  24
<212>  DNA
<213>  Artificial

<220>
<223>  artificially synthesized primer sequence

<400>  12
cactcaacga gtaggaaaca aaga                                              24

<210>  13
<211>  12737
<212>  DNA
<213>  Brassica rapa

<400> 13
accaaagaga ttgtgctgca gagattcttt tcttgggatc cttactgagc gtttttcata 60
acaaggtcat ggttggaagg tccagcgata tccctgtaag cttcatatca agcatatgaa 120
cacccgagat tattatcaac taagactacc ataattgaga atctgttttc tttttttcag 180
tggagcagga tatgtttctc ttattgagta tgctcattta actggaagac ctttctgact 240
tattgagtac atgtttcctg atgctgtgct ttggttagga acaaggtgtt tgttctcttt 300
ctctcaagat ggtcaccaag agtgttgcct atccgaaaag tgagtcctct gaggcgtgcc 360
ttaaaggatt tatttaaagt tgaaccttaa caaatgttca agccttgtgg agcttccctc 420
ttctattgag aatatgacta atctccaaaa tttatatctc aacggatgct caggtctaga 480
gaagctcccc tcttctattg ggaatcttta ttgtttgcaa agattgcaaa tgaaaggttg 540
ctcaaagcct aaattccctt ccgattacca tcaatttgaa atctctttgg gagctagatc 600
tcagatagtg ctcgtcgttg aaaaggtttc ctgagatttc cataaatatt tatagatcta 660
aagcttaaag gagctgcact agaagaattt ccatgatttt agcatcactt tatcatcgat 720
agtttcttgc ggcaccatct cattctatgg gagctgaaaa aaacaattcg cagtttgctg 780
tatctgtgta cactactttc tgtaaaacat ttcccccggt ttaggacagc aatacttcta 840
cttccactat aacttctaga ataatgtttt tggatcaagt ttaattttat gttcctcctt 900
ttcgatttat caatgaatgg aatggaacaa gttttccctt ttgtggctgc gtggtaccag 960
tatcacttca tcatggcaca gaattcttca ttgttaatct caacgttggc tcataggttt 1020
ttttcatgct atgtagtggg aaacataact tgtaattatc tgtatctact tcctctatag 1080
cttctttgat gatagcttca tctcccatac catactattt catggctaac tccaactcat 1140
ccattgttga taaacctgtg taagccaatt ccatctcagt gtcttggaat attgttttaa 1200
cttctcattc tatagttttc tctgttccac caaagagaac ttaccagctc ttgtctttgt 1260
caaagtattg gaagtctttg aacaagtgct cctctcaatc cagtctgtat ctatagtaat 1320
aacttccatg agttggttaa cttcatctac agagagtcta gaaccgtgtt tagccaccct 1380
ggttttcagt tctttgtaat taagggaggt acaccacaaa gcaagatgta tagaataatc 1440
tctgcactcc aaatatctct gtaaacttat tgaagcccat aagaaagacc cattgaatga 1500
tccattaaga atcttttgta accatgtaac tagggtttat agaccattat aaatatgtcg 1560
ttagatgttc tttggttgta agtctttggc tggtataata gcagcctctc cctttccaat 1620
atatgaaatc aaatccaacg tttatctctt ggtgacaact taatctcttc ctctctttag 1680
tttctagcat ttcaataaag ctttcgtaag ctatctttca tgtgtcaata aagcttagca 1740
aagctataat cttttatgtt tctcaaacgt tatcaaaact ttccctgcaa ttttgttttc 1800
agtttataaa acaacacata aagcttatga atacacagtt atcaactaac taaactgata 1860
gtaagattag gaaaaaagaa aacaaaaggt ctacacaagt aattactcac attcccaact 1920

tgcaaagcct ttttttgaag attaccacta tgtactgaac taataagact ctcaagttat 1980
atatggatca cacctgaaaa taaacattgg ttataatctt aagaaatcta aaacatagca 2040
tttacatata tcttataact agaagagttg tatgtatagc gttctcaact tctccaatga 2100
aagaaaagat tggtatgagt aggaggagaa atatataaaa aagcttctag agaagattga 2160
atgatctctt tgctgtgaaa ttttcaagtg cgaactcatc agttctgttc actgcttctt 2220
tgaaattgct tgtgctcttc cttgcatttt ccttcttaga atagtataag ccaaaaggga 2280
atggtgggac tttatgcacc ttatttttcg ttggtgacat atttttccac aaaagtcttt 2340
gtaagtcggc cgagtcaacg ttttatgaca tatacgcaat tacgcataca aaattgtcac 2400
aagtctcttt ggttcattat tgcgttacat atcttcttta gaaatattct tggtccctct 2460
ctctctacca cacagattga ttatcccttc taatcatccc ctctacaatt cttgctgtat 2520
gcttcagatt ccatcaagaa aaccaagaaa ctgattttct tttttttcaa cttcggtaaa 2580
aaaaaaggca gctttttgaa aatgaaattt caatcgtttt tgaaagaaat taagaaatgt 2640
gaatcacaag agacttattg aagaagaaag agttttgact gcaatatttt tatatctttc 2700
aacttggtta taaagataca actctaactc tatttatatg ccaagcgcaa cctcaaaacc 2760
ctaatgaacc tctacacgtg tttggaggac attgaatcta ttctttgttg caaggtggtg 2820
agctgtacgc ggtgccaaag tggtcctgct cgtcgtacgg tttggctctg caatatgaac 2880
gttgagaggt tgagctaaag caggctttgt cttcatcttt gactggtctc cgaaactcat 2940
atcattgggc ttgggctttg ttgttgtctt gggctcagcc tgactaataa gaaacagaga 3000
gggaagagga aaagagatga actttcgaat ccagcaagaa agaagatccg agttcagaac 3060
ctgagccaaa tccaagtacc atcatcaccc ccaccttctc cttcatcact tccatcctct 3120
ttgtctcttt catcagctcc atcttcttcg tctcataact ggacacacga tgtctttcca 3180
agcttccgcg gggaagatgt ccgcataggg tttctaagcc acattcaaaa ggagtttaaa 3240
agaaaaggaa tcacaccatt catcgacaat gagatcagga gaggagaatc catcggtcca 3300
gaactcatac gggccattag aggatctaaa atcgccatca tcttgctctc gaggaactat 3360
gcttcttcaa agtggtgcct tgacgagttg gtggagatta tgaagtgcaa agaagagtta 3420
ggccaaaccg taatccccgt tttctataaa gtagatccat ctgatgtaaa gaagctgaga 3480
ggatattttg ggaaagtttt cgaaaaaact tgcgagggta aaagtaagga ggatactgag 3540
aaatggagac atgctttgga gaaggtggcc acaattgctg gttacgattc aagaacctgg 3600
tttgtccttt tctatttctt tttgtttcta atctttcaaa ctcatacaaa tctaactcaa 3660
aaattccaaa tgatcacagt tttttcctct taatttttac ttgctcttta tatgcatcaa 3720
tatataatat caatgttaat ctcaaaattt aacaaaaggt gttagttcca aaaattcaaa 3780
aacttgtgaa aacatatacc attctctaaa ttttgaacat gtttggttat ttaatgaaga 3840

gatattggga gcgtgaattc aaatagagtt gcttaatttt taatttaaga gttttgtgta 3900

tttaacatca tccaaaaaca tgcaatgtga cattcttgag aattgcacac agcatatttc 3960

cagccaaaca attcttattt ggaaaccgtt tgagccgatg tatgttattt cctctgttta 4020

gggataatga agcggccatg attgaggaaa tagccacaga tgtttcaaac aagctgatta 4080

gttctgttcc atcaagtgat ttcaacagct tagttgggat gagagctcac atgaaaagta 4140

tggaactgct cttacgcttg gattccaatg aagtgaggat gatagggatt tggggtcctt 4200

ctggaattgg taagagcacc atcgccagat ctctctttag ccaacactct cctgactttc 4260

aacttagcgt cttcatggag aatatcaaaa gagagtatcc aagaccttgt tttgatagat 4320

acagcgcaca actgcaatta caaaagaagt tcttgtctct aatactcaat cagaatgatg 4380

tcgctatcca tcacttagga gttgcacaag accggctaaa aaataagaaa gtgttagttg 4440

ttctcgatga cgtggatcac tcagcgcaac tagatgcctt ggcgaaagta ccctcatggt 4500

ttggtcctgg aagtaggatt atcgtcacga cgcaagataa gaaaattttg aatgcgcatc 4560

ggatcaatca tatttacgag gttggttttc cacatgatga tgaagctctt gaaatcttct 4620

gcataaatgc ttttggtcaa aaatccccat atgatggttt tcgaaacctt gctcgggaag 4680

ttacaaggct tgtgggtaaa ctccctttgg gactaagtgt tatgggatct tatttcaaag 4740

gcttgtccaa ggaggtgtgg gaacgtgagt taccaaggtt aagaactaga cttgacggtg 4800

aaacagaaag tattttaaag ttcagctatg atgcgttatg cgatgaagat caagctttgt 4860

ttcttcacat agcctgcttt ttcaacggtg aacggattga caaagtagaa gagtttcttg 4920

cagagaaatt tgttgctgtg gaaggtcgtc ttcgtgtttt agctgagaaa tctctcatat 4980

ccgtagactc agaaggatat ataaggatgc atgatttgct agcacgtttg ggtagggaaa 5040

ttgttcgtaa acaatctcct aacgaacctg ggcagcgtca gtttttggtt gatgatggag 5100

atatacgcca agtactacgt gatgatacac ttgtaagttt tcacgttggt tgttctcagc 5160

atttctccta gaaaatgtta taacaaatca tgtttgctta tgtatctttg ggttcttttt 5220

cagggtagtc gaagtgttat aggcataaaa tttgagttgg ggaagaagga gttgaagata 5280

agtgatggag cctttgaaag aatgtccaat gtccaattct taagacttga tagtgattta 5340

tttgaccata ttctactcgt acgaacaaat agccaataca tattagaaag tgtgaactgt 5400

ctacctcgag aagttagatt actgcattgg agcacattcc cgatgacatg tctgccttct 5460

gattttaatc cagagctcct aatggaaata aaaatgagat gtagcaacct tgagaaattg 5520

tgggaaggaa ataaagtaag tacaatatat atacatattt caaaattata tgctttagct 5580

gaggatcgtc tttttcttaa tgatgcattt tgtttatctg tacagacgat tagaaatctc 5640

aagtggatgg atttgtctta ttcaaaatat ctaaaggagc ttccaaatct ttcaactgcc 5700

acaaatctcc gagaactgga tctcgatatt tgctcaagtc ttgtggagct tccttcttct 5760

attgggaatt tgactaatct caagaaattg aatctcgagc tgtgctcaag ccttatggaa 5820

ctcccctctt ctattggtaa catgactaat cttgagaatt tgaatctttc tggatgctct 5880

agccttgtgg agctcccctc ttctattgga aatatgacca atctcaagga attggatctc 5940

agtgaatgct caagccttgt ggagctcacc tttgggaata tgaccaatct caaggatttg 6000

gatctcaatg gatgctcaag ccttgtggag atttcctctt ctattgggaa tatgactaat 6060

ctcgtgaaat tggatctctc cagatgctca agccttgagg aactcccttc ttctattggg 6120

aatatgacta atcttgagaa tttgaatctc tccggatgct caaagctaaa agcccttccg 6180

atcaacatta acatgaaatc tcttgatgag cttgatctca catactgctc ctcgatgaaa 6240

aggtttcctg agatttccac aaacattagc gttctaaaga ttgatggaac tgctataaaa 6300

gaaattcctg catcaatcag ttcatggtct cgtcttgata ggttacatgt gtcatacagt 6360

gaaaacctcg ggagatcccg acatgttttt gaccgcatca ggaagctgga cttgaacgac 6420

acaggattac aagaaattgc tccatgggtc aaggaaatgt cttgtctaga gacactagta 6480

atccacggat gctcaaatct acaaaagctc cgctcttcca ttggaaattt gactaatctt 6540

gagaatttgg atctcaaagg atgctcaagc cttgtggagc tcccctcttc tattgggaat 6600

cttcataatt tgaagcagtt gaatctcgga aattgctcaa agctaatgtc ccttccagtg 6660

aacatcaaca tgaaatctct tgatgagctt catctcaggg actgctcgtc attgaaaagc 6720

tttccggaga tttccacaaa cattggagtt ctaaagctca acggaactgc tattgaagaa 6780

attcctcaac caatcaggtc atggtctcgt cttgaaaggt tacatatgtc atacagtgaa 6840

aacctcggga aatcccagca tgcttttgac ctcatcacag agcttcactt gagcgacaca 6900

cgaatacaag aagttgctcc atgggtcaag gaaatgtctc gtctacataa acttgtaacg 6960

aagggatgca caaagctggt ttcgctccca cagcttccac attcattaga attcatgcat 7020

gtagaaaact gcgagtccct ggagagacta gattgctctt tttacaggac aaagttgagt 7080

gatctttgct ttgttaactg cctcaaactg aatcgagaag cagtagacct tattctcaag 7140

acatcgacaa aaggatgggt gatctttccc ggagaaacgg tgcctgcata tttcagttac 7200

agagccacgg ggagttcagt gtcaatgaaa ctggatggat tcgatacacg ttttcctaca 7260

tccttgagat ttaaagcttg catcttgctt gttactaacc ctgacgacgt tgagcctgct 7320

gcttggtaca ggtcggatat atcttattgc atcaatggca aactgaggga tgtcggtgtt 7380

ttactattat tttctcatat atgggaccca ctattaatgt atagagtctg gtttagtccg 7440

gtttagttga ataggtatta tgattctcat ttggtttaca ggattagtca agtgtatata 7500

tatcttgtac caaactcatt ttgtcagttg agaaataaga gatcatttta catacgattc 7560

atctttactc atgatctcta ttatggtatc agagctcttt tagctcgttt ttcttcgatt 7620

cctcttcgtt ttcttcccaa tcatcctccg atcgttggtt cttcactcta ttcctcaatt 7680

tctgatcgta tcgagctcag atctggtgaa aaatggtgaa gacaggggga aagatgagaa 7740

ctcgaagcgc gatatcggaa gacgaagatt ccacgagcag atttcgtcag gatttccaga 7800
atctcgatga acctctcact gcgatgacga aaactcaaca gaatcgccaa tcgatgactc 7860
cagggggaaa cggatctatg cgaaagccat cagaagcgta cgatagcatc cacagtccgt 7920
tcttcctcca ttctgcggat catccaggtt tgacgattgt tgttcatact cttgacgggt 7980
tcaaactaca acagctggtc gattgcgatg aaaataagct tagatgcgaa gaataagctt 8040
agctttgttg atggatcgct tcctaggccg ttagtggatg ataactcgtt caagatatgg 8100
agtcgttgca atagtatggt gaaatcgtgg attctgaatg tcgtgagtaa aggagatcta 8160
tgatagtatc ttgtattatc aggatgctac tgagatgtgg gatgatctgt ttcgaagatt 8220
caaagtgtgt aatctgccac ggaaatatca attggagcaa gcggtgatga ctctcaaaca 8280
aggagatctt gatttgtcaa cctatttcac aaagaagaag acactgtggg aacaactagc 8340
taacacgaag tctagcacgg tgaagcgttg tgattgtgat caagtcaaag aacttctgga 8400
ggaagcagag acaagccgta tcatccaatt cttgatggga ttgaatgata atttcaataa 8460
cattcgtggt cagattctta acatgaagcc aaggccggga ttgaatgaca tctacaatat 8520
gcttgatcaa gacgagagcc agcgagtagt aggaggacct tcggtcatga agcctactcc 8580
atcagcgttt cagaatcaag cacagtttgc agatcagaat caaaatccgg ttctcatggc 8640
acatggtggt ggttatcaga aaccaaaatg ctcacattgc ttccgcattg gacatacagt 8700
tgacaagtgc tacaaagtcc atggttatcc accaggacat ccacgtgcaa agaagagtaa 8760
cacgattggt aatactaact tggctgcaac gatgactgtg tcacagaacc agaatgagca 8820
aggttttgat gagttaagca gtaatatgtc taaggaacag ctccagtaga tgattgcgtt 8880
cttcagttcc aagctccatt ccccgagtgt tgctccatgc tcagataaat cagtagcctc 8940
aacttcaact tctgtaccag ttatatctca aatttctggt actttcctca ctctctataa 9000
taactcttac tatgacatgc tgacttgttc tgtatctaaa gaaactgagc tgtctcttag 9060
tgcttggatc atagattccg gggctagtca ccatgttact catactaggg aattatatag 9120
agagtataga agtctagaaa acaccttcgt tacccttcct aatggttata cagttaagat 9180
agcagggact ggttacattc aactcactga tgctttgtct ctccataatg tgttacatat 9240
tcctgaattc aaattcaatt tgctgagtgt tagtgttcta actaagactt tacattctca 9300
agtttgtttc acttctgatg cttgttttat tcaggctctt actcaggagt tgatgattgg 9360
tcaaggtagt caagtcgcaa atttatatgt cttagatcaa gagaattcgc gtattaatct 9420
cattgctcca ggtaacttct ctgtttgctc caacctggta gttgattctc tgacttggca 9480
tagaagatta ggacatccgt ctatgaagaa aattgaatct ctttccacaa ttttaaattt 9540
ccctaaggat aaacatgtta aacattctga tgcgtgtcat gtttgtcatt tgtctaaaca 9600
aaagcggtta ccctttataa accgaaatca tctttgtaat aaaccatttg agttaataca 9660
tatagatact tggggtccat tctctgttcc tactgttgat ggttacaagt atttcttaac 9720

aattgttgat gattttagta gagctacttg ggtttacttg cttagacaga aatctgatgt 9780

gttacatgtt tttccgggtt ttattcaaat gattgagaat caatttcaca tgaatgtgag 9840

tgcaattaga tctgataatg caaatgaatt gaatttcact aatctatatt tgaataaagg 9900

aataaaagct taccattctt gccctgaaac tcctgaacaa aattctgtgg ttgagagaaa 9960

gcatcagcac ctgttaaatg tagctagagc ccttatgttc cagtcaggta tgcctttaga 10020

atattggggg gattgtgtat taacagcagt ttttcttatt aatcgtttac catctcctgt 10080

cttagagaat aagacaccct atgagcgttt aacctctcaa gttcctgatt atcattccct 10140

taagactttt ggttgccttt gttatgtgtc tacgtcaccc aagtctaaga ataagtttga 10200

acctagagct aaagcctgtg tgttcctagg ataccctgct ggttacaaag gatacaagct 10260

tttagatata gagactcgct cagtttcaat ttctaggcat gtcattttct atgaggatac 10320

tttccctttt gcttcttcaa acattcctga agatgtcaaa agtttctttc ctcatcttca 10380

tttccctgca caaactgatg tattgccttc tatgcaaaca tcttctgata ctcatcatcc 10440

ttgtgatgag tcatcttctc cggcttttgt tccctctgaa ccgaagtctg ctaggcaacg 10500

caaaccgcct tctcatttgc aggattttca ttgctatact actaataaca ctactcctat 10560

aaatacaact ccatatccat tacaaaattt tatttcttat tcttatttag ctgagccttt 10620

tagtgctttc ataaatgcca ttacttcttt caaaattcct caaagacttt ccgaggctct 10680

tgaagataaa gtatggcgtg actcgatggg attggaaatt ggagcttttg atcgtactga 10740

aacgtggagt gttgttgaat tgcctccagg caaaactgct ataggttgta agtggcttca 10800

tactgtgaaa tttaaccctg atggtacagt tgagagagtt aaatcgcggc ttgtaggtaa 10860

aggttataca caacaggagg gcattgattt tctagataca ttttctcctg tcgctaagat 10920

gactacagtg cgtttgatct ttgctttagc tgctaaatta aagtggcatt tacatcaatt 10980

ggatatttcc aatgctttcc tcaatggtga tttggatgaa gaattataca tgaagcttcc 11040

accggggtat gctgagatta agggggaaca aatctcacca actgcagtgt gcaaattgca 11100

taagtctatc tatggcttaa agcaagcgtc gaggcagtgg tttttaaaat tctctgcaac 11160

tctgttgggt tttggctttg agcaatgtca tggagatcac actctgttca tcaaagaact 11220

tgcggggcat ttcttgatag tcagtgtgta tgtagatgac atactcattg cgagtacatc 11280

tctggaggga gtgagtgagt tgatcagcag tttaagtgca gttttcaagt tacgtgattt 11340

gggtatacct aagtattttc ttggcattga aattgcaaga acagctgaag gcatttattt 11400

atgccagagg aagtatgtgt tggatctatt ggaggcatcg ggattttctg attgtaaacc 11460

ttcttccata cctatggaac cgaatcagaa attatctaag gaggatggag ttttgattga 11520

agatgttaag cagtatcgaa gattggttgg gaagcttcag tatcttacaa ataccagacc 11580

tgacatcgcg tttgcagtat ccaagcttgc tcagtattct tgtgctccaa cagatattca 11640

tttgaaggct gttcataagg tgttgaggta tctcaaaggt actattggac agggtgtgtt 11700

ctatggagtg gaggataatt ttgatttgcg agggttctct gattctgact ggggaggttg 11760

tcctgatgat aggagatcag tcacaggtta tgcgatgttt ctgggcagtt ctttggtatc 11820

ttggagatca cagaagcaag acatagtatc gatgagtaca gctgaggcgg agtatcgtgc 11880

tatgagcgtg gctactaagg agattatgtg gtattgtggt gttctcaagt ctgtccgagt 11940

gccgttttct cctccagctt acttatattg cgataacact gctgctcttt acattgctac 12000

taattctgtt tttcatgagc gaaccaagca tgtggagttt gattgccata aggtgagaga 12060

gtgcatcgtc cgtggcatct tgaagacaat gtatgttcgt accgacaatc aactggccga 12120

tgtgttgact aaggcattgt acccagctcc atttcgagac attatcagca agatgggtgt 12180

ttttaacctt tatgcacctt catcttgagg gggaatatta atgtatagag tctggtttag 12240

tccggtttag ttgaataggt attatgattc tcatttggtt tacaggatta gtcaagtgta 12300

tatatatctt gtaccaaact cattttgtca gttgagaaat aagagatcat tttacatacg 12360

attcatcttt actcatgatc tctattaccc actctgtcca cgttctgagc atctggtcgt 12420

aatcgaattt gaagaaactg tgacttcccc cgaattagtc ttcgagttca ggttcaaaaa 12480

agaaaactgg gagattaaag aatgcggact acgtcctcta gaaagcttag ctctctcatg 12540

ttgatggaag ctgaagaaac attttgcagt atgtgtgtaa gattatcatt gtgtgttatg 12600

tttggtttga gtaaaatgta acggcaacta tatttatgta cgcatgtgca taaaatatag 12660

taatgcattc cgttgtttat cttaaatatc aaggaaacaa tttttattag taatctttgt 12720

ttcctactcg ttgagtg 12737

## Description

### Technical Field

The present invention relates to clubroot resistance genes and methods for producing cruciferous plants that are resistant to clubroot by using these genes.

### Background Art

Clubroot is caused by *Plasmodiophora brassicae.* It is a soil-borne disease and is difficult to prevent; and it affects cruciferous vegetables such as Chinese cabbage (Hakusai, *Brassica rapa* L. Pekinensis group), turnip, Nabana *(Brassica napus L., Brassica rapa L.* Oleifera Group), Nozawa-na (*Brassica rapa* L. Hakabura Group), Tsukena (*Brassica rapa L.* Perviridis Group), cabbage, and broccoli in Japan, and rapeseeds abroad. Since the roots of the diseased lines enlarge in the form of a club, they pose problems in nutrient and water absorption, and cause significant delay in growth, or in some cases, cause plant death. Once this disease occurs, a large number of resting spores are released into the soil from the infected lines. Since the resting spores exist in the soil for a long period of time and maintain their ability to germinate, their effects cannot be expected to be reduced by crop rotation; and in a continuously cropped field, the fungus density increases year after year, and cultural control is difficult. Therefore, it becomes necessary to cultivate dependently on chemosynthetic agrochemicals or change crops to vegetables other than cruciferous plants.

Clubroot resistance genes were not found in the genetic resource of headed Chinese cabbage belonging to *Brassica rapa*; and diligent research by Yoshikawa at the National Research Institute of Vegetables, Ornamental Plants and Tea proved that European fodder turnips such as Siloga, Gerlia, Millan white, and 77b are promising resistant materials (Non-Patent Document 1). Furthermore, a number of resistance genes have been found in these European fodder turnips. The pathogenicity of clubroot fungi, *plasmodiophora brassicae,* is diverse, and as a method to identify them, the European Clubroot Differential (ECD) method, the Williams method (Non-Patent Document 2), and such have been proposed. In Japan, indicators for assessing the pathogenicity of domestically emerged strains (Non-Patent Document 3), and classification methods using readily available cultivars (Non-Patent Documents 4 and 5) have been reported.

With regard to the cultivation of resistant cultivars of Chinese cabbage (Clubroot Resistance; CR cultivars), a major resistance gene was found in *Brassica rapa,* and by devising high-precision resistance assays and establishing methods capable of distinguishing differences in pathogenicity, six parental lines of Chinese cabbage were reported from the National Research Institute of Vegetables, Ornamental Plants and Tea, and clubroot-resistant cultivars of Chinese cabbage were cultivated in private seed companies. On the other hand, resistance breeding could not catch up with the speed of race differentiation and spreading of clubroot fungi, so that the resistance of the clubroot-resistant cultivars was lost and reports of infected cases have increased. Among the clubroot fungal isolates, isolates that damage many resistant cultivars and have a wide host range became noticeable. Therefore, chemosynthetic agrochemicals and materials promoting decrease of clubroot are being used even when cultivating resistant cultivars. Use of chemosynthetic agrochemicals such as Nebijin or Furonsaido imposes great burden on the farmers in terms of cost and labor. What is desired is the development of CR cultivars that can cope with a wide range of clubroot fungi races, or specifically, accumulation of a plurality of the introduced resistance genes. However, there are problems in terms of precision and efficiency by conventional selection according to phenotypes such as clubroot resistance. Furthermore, there are few major genes in *B. oleracea* to which cabbage and broccoli belong; and since a number of resistance genes need to be brought together to exert resistance, breeding and cultivation of resistant cultivars are considered to be extremely difficult work.

Cultivating resistant cultivars that can be grown without the use of chemosynthetic agrochemicals is required at the social level. However, as described above, there are many technical issues that have to be solved to accomplish this objective.

To date there have been no articles reporting the isolation of resistance genes for clubroot in cruciferous plants. Meanwhile, a research group at Kyoto Prefectural University is in progress of isolating Crr3 derived from a European fodder turnip "Milan White", and at present they are known to be confirming the work by transformation. The only case in which clubroot resistance was conferred by genetic recombination was generation of a resistant individual by linking an antifungal peptide (Scarvaecin derived from Taiwanese unicorn beetle) gene to a nitrilase promoter and introducing it into broccoli (Patent Document 1). The current method for developing resistant cultivars is mainly through breeding by crossing and selection.

In the development of markers for efficient selective breeding in *B. rapa,* many gene loci such as RA1275 (Non-Patent Document 6), Crr1, Crr2 (Non-Patent Document 7), Crr3 (Non-Patent Documents 8 and 9), Crr4 (Non-Patent Document 7), CRa (Non-Patent Documents 10 and 11), CRb (Non-Patent Document 12), CRc, and CRk (Non-Patent Document 13) have been reported. Outside of the National Institute of Vegetable and Tea Science, patent applications and such have been submitted for markers linked to rutabaga-derived resistance (Patent Document 2).

At the National Institute of Vegetable and Tea Science, as DNA markers that are linked to Crr1 and Crr2, BRMS-173 and BRMS-088 (for Crr1 above), and BRMS-096 and BRMS-100 (for Crr2 above) were developed, and a patent has been acquired for their use (Patent Document 3). In the development of markers related to *B. oleracea,* gene loci that have a relatively small contribution rate as compared to major genes have been reported; and while QTL such as pb-Anju-01 have been reported recently by Nagaoka *et al.* (2010) (Non-Patent Document 14), their use in actual breeding is presumed to be low.

Furthermore, there is one past example of cultivation of plants that were conferred clubroot resistance using genetic recombination techniques. This involved operably linking a promoter of a gene specifically expressed during clubroot fungal infection with an antifungal peptide, introducing it into broccoli, and then confirming the resistance. Use of the promoter has been filed for patent (Patent Document 1). However, the disease index of the plant obtained by introducing this set of promoter and gene was 1.36 to 1.50. Furthermore, the very clubroot resistance gene itself has not been used to confer resistance by genetic recombination techniques.

While there are many reports on markers for selection of clubroot resistance relating to Chinese cabbages as described above, the extent of their use in actual breeding is unclear. Crr1 is known to be positioned approximately at the center between BRMS-173 and BRMS-088 which are 4-cM away from each other. Specifically, since each of the markers of BRMS-173 and BRMS-088, which are linked to Crr1, are both approximately 2-cM away from Crr1, recombination has been confirmed to take place at a certain constant probability between the marker loci and the resistance gene locus. As in this case, when one cannot eliminate the possibility that recombination is taking place between the marker loci and the resistance gene locus, the progenies obtained after crossing or selection using two markers flanking a gene locus must be subjected to clubroot resistance assay to confirm the transmission of the resistance gene.

While there is information on the narrowed-down candidate Crr1 genes (Non-Patent Documents 15 to 18), none has been proven to be Crr1, and the sequence information has not been revealed either. A number of ORFs are present in the genomic region that has been narrowed down by map-based cloning, but it is unclear as to which of them is the Crr1 resistance gene. Furthermore, there have been attempts to elucidate all of the regions of the expressed genes by RT-PCR; however, the amplified clones were not all the same, and one could not determine which clone was Crr1. All of the amplified clones were compared and examined; and there were unpredictable situations such as the ill-functioning of splicing at the intron regions. Therefore, it was considered impossible to understand which regions correspond to the gene from the disclosed contents alone.

Prior art documents relating to the invention of this application are shown below.

### [Prior Art Documents]

### [Patent Documents]

[Patent Document 1] Japanese Patent Application Kokai Publication No. (JP-A) 2009-178090 (unexamined, published Japanese patent application)
[Patent Document 2] JP-A (Kokai) 2005-176619
[Patent Document 3] Japanese Patent No. 4366494
[Non-patent Documents]
[Non-patent Document 1] Yoshikawa, H., Bull. Natl. Res. Inst. Veg., Ornam. Plants & Tea Japan, (1993) 7:1-465.
[Non-patent Document 2] Williams, P. H., Phytopathology, (1966) 56: 624-626.
[Non-patent Document 3] Kuginuki Y. et al., Eur. J. Plant. Pathol, (1999) 105:327-332.
[Non-patent Document 4] Hatakeyama K. et al., Breed Sci., (2004) 54,197-201.
[Non-patent Document 5] Hatakeyama K. et al., Horticultural Research (Japan) (2008) 7 (supplementary volume2) 180.
[Non-patent Document 6] Kuginuki, Y. et al., Euphytica, (1997) 98: 149-154.
[Non-patent Document 7] Suwabe, K. et al., Theor. Appl. Genet., (2003) 107: 997-1002.
[Non-patent Document 8] Hirai, M. et al., Theor. Appl. Genet., (2003) 108: 639-643.
[Non-patent Document 9] Saito M. et al., Theor. Appl. Genet., (2009) 114:81-91.
[Non-patent Document 10] Matsumoto E. et al., Euphytica, (1998) 104:79-86.
[Non-patent Document 11] Hayashida N. et al., J. Jpn. Soc. Hortic. Sci., (2008) 77:150-154.
[Non-patent Document 12] Piao, Z. Y. et al., Theor. Appl. Genet., (2004) 108:1458-1465.
[Non-patent Document 13] Sakamoto K. et al., Theor. Appl. Genet., (2009) 117:759-767.
[Non-patent Document 14] Nagaoka T., et al., Theor. Appl. Genet., (2010) 120: 1335-1346.
[Non-patent Document 15] Matsumoto, S. and three others, "Molecular genetic analysis of Chinese cabbage clubroot resistance and application to breeding", [online], KAKEN, internet <URL: http://kaken.nii.ac.jp/en/p/19380008>
[Non-patent Document 16] Matsumoto, S., Kato, T. and five others, "(1) Cultivation of practical clubroot-resistant Chinese cabbage cultivars by marker selection; (2) DNA marker linked to clubroot resistance of the Chinese cabbage F1 cultivar "Akiriso", [online], internet <URL: http://www.nacos.com/jsb/06/06PDF/117th_611_612.pdf>
[Non-patent Document 17] Matsumoto, S., "Map Based Cloning of the Clubroot Resistance Gene, Crr1, in Brassica rapa L.", [online], September 9, 2008, Brassica2008-Lillehammer-Norway, CLUBROOT SESSION, internet <URL: http://www.brassica2008.no/clubroot.html>
[Non-patent Document 18] Matsumoto, S., and three others, "Isolation of a candidate gene (crr1) involved in clubroot resistance of Chinese cabbage", [online], September 28, 2008, Japanese Society for Horticultural Science, Internet <URL: http://www.jshs.jp/modules/tinyd4/index.php?id=7>

### Summary of the Invention

### [Problems to be Solved by the Invention]

An objective of the present invention is to provide efficient techniques for producing clubroot-resistant cruciferous plants by isolating clubroot resistance genes, and performing genetic recombination and marker selection.

### [Means for Solving the Problems]

The present inventors carried out dedicated research to solve the above-mentioned problems.

To promote efficient breeding, there is the method of cultivating by introducing resistance genes using genetic recombination techniques; and for species that can be crossed easily, there is the method of developing resistant cultivars by marker-assisted selection utilizing the genome information around the gene locus. The present inventors provide efficient methods for producing clubroot-resistant cruciferous plants by isolating clubroot resistance genes, and performing genetic recombination and marker selection.

Specifically, first, the present inventors successfully isolated a clubroot resistance gene. By linking the cDNA of the isolated Crr1 to the 35S cauliflower mosaic virus promoter or the lettuce ubiquitin promoter and expressing this in *Arabidopsis thaliana,* the inventors confirmed strong resistance in clubroot-susceptible *Arabidopsis thaliana.* By using this method, clubroot resistance may be conferred to species into which genes cannot be introduced by crossing, such as cabbage and broccoli belonging to *B. oleracea* and rapeseed of *B. napus.*

Furthermore, the cDNA or genomic DNA of the clubroot resistance gene was linked to an expression-regulatable promoter or a promoter unique to the clubroot resistance gene, and a cassette that enables expression of these genes in plants was produced. This was used to generate transformed cruciferous plants (such as cabbage, broccoli, and rapeseed). This is a novel production method, and there are no examples of using seeds of the obtained transformed plants to cultivate plants that become resistant due to clubroot resistance genes.

Furthermore, because the genomic structure of Crr1 was elucidated, it was revealed that there existed partial insertion or deletion of DNA sequences as compared with individuals without Crr1. These positions can be used as target markers to assess the presence and absence of resistance through DNA polymorphism. In this case, since the information used is a gene region, recombination does not occur between the marker loci and the resistance gene loci. Therefore, extremely accurate selection becomes possible.

Specifically, the present invention relates to the following:
[1] a polynucleotide having clubroot fungus resistance, which is any one of (a) to (d) below:
   (a) a polynucleotide encoding a protein comprising the amino acid sequence of SEQ ID NO: 2;
   (b) a polynucleotide comprising the coding region of the nucleotide sequence of SEQ ID NO: 1;
   (c) a polynucleotide encoding a protein comprising an amino acid sequence with one or more amino acid substitutions, deletions, additions, and/or insertions in the amino acid sequence of SEQ ID NO: 2; and
   (d) a polynucleotide that hybridizes under stringent conditions with a complementary strand of the nucleotide sequence of SEQ ID NO: 1;
[2] a vector in which the polynucleotide of [1] is operably linked downstream of a promoter region that enables expression in a plant cell;
[3] a transformed plant cell into which the vector of [2] has been introduced;
[4] a plant having clubroot fungus resistance activity, which is regenerated from the transformed cell of [3];
[5] a plant having clubroot fungus resistance activity, which is a progeny or a clone of the plant of [4];
[6] a propagation material of the plant having clubroot fungus resistance activity of [4] or [5];
[7] a method for assessing clubroot fungus resistance of a test plant or a test propagation medium, which comprises the step of detecting a DNA region comprising the nucleotide sequence of SEQ ID NO: 1 or SEQ ID NO:3, or a partial sequence or surrounding sequence of this nucleotide sequence;
[8] the assessment method of [7], which comprises the following steps of:
   (i) preparing a DNA sample from a test plant or a test propagation medium;
   (ii) amplifying a DNA region comprising the nucleotide sequence of SEQ ID NO: 1 or SEQ ID NO: 3, or a partial sequence or surrounding sequence of this nucleotide sequence from the DNA sample; and
   (iii) comparing the molecular weight or nucleotide sequence of a DNA fragment produced by amplifying the DNA region comprising the nucleotide sequence of SEQ ID NO: 1 or SEQ ID NO: 3, or a partial sequence or surrounding sequence of this nucleotide sequence, from the clubroot fungus-resistant plant or propagation medium with that of the DNA fragment amplified in step (ii);
[9] a method of selecting a plant or seed thereof having a clubroot fungus resistance gene by the assessment method of [7] or [8];
[10] a method for producing a plant or a seed thereof having clubroot fungus resistance activity, which comprises the following steps of:
   (i) introducing into a plant cell the vector of [2]; and
   (ii) regenerating a plant from the transformed plant cell which has been introduced with a vector in step (i) mentioned above;
[11] a method of conferring clubroot fungus resistance activity to a plant or seed thereof, which comprises the step of expressing the polynucleotide of [1] in a plant cell;
[12] the method of [11], which comprises the step of introducing into a plant cell the polynucleotide of [1] or the vector of [2];
[13] the method of [11], which comprises the following steps of:
   (a) crossing a plant comprising the polynucleotide of [1] with another plant; and
   (b) selecting a plant comprising the polynucleotide;
[14] the method of any one of [7] to [13], wherein the plant is a cruciferous plant;
[15] a plant or seed thereof which is obtained by the method of any one of [7] to [14];
[16] an artificially produced plant or seed thereof, which comprises the polynucleotide of [1] and has clubroot fungus resistance activity;
[17] the plant or the seed thereof of [15] or [16], wherein the plant is a cruciferous plant;
[18] a primer for detecting clubroot fungus resistance activity of a test plant, which comprises an oligonucleotide having a chain length of at least 15 nucleotides, and specifically hybridizes under stringent conditions with the nucleotide sequence of SEQ ID NO: 1 or the nucleotide sequence of SEQ ID NO: 3; and
[19] a probe for detecting clubroot fungus resistance activity of a test plant, which comprises an oligonucleotide having a chain length of at least 15 nucleotides, and specifically hybridizes under stringent conditions with the nucleotide sequence of SEQ ID NO: 1 or the nucleotide sequence of SEQ ID NO: 3.

### Brief Description of the Drawings

Fig. 1 depicts a detailed mapping in the neighborhood of the clubroot resistance gene locus Crr1 . BSA7 is a marker obtained by synteny analysis with *Arabidopsis thaliana,* and in an analysis using 1920 segregated populations, it cosegregated with Crr1.
Fig. 2 depicts a BAC clone assembled by chromosome walking from BSA7 near Crr1. A number of BAC clones containing the BSA7 sequence was isolated, and seven clones were used to cover the area between BSA7 and BSA2. The terminal sequences of the obtained BAC were used to develop markers, and the location of Crr1 was refined.

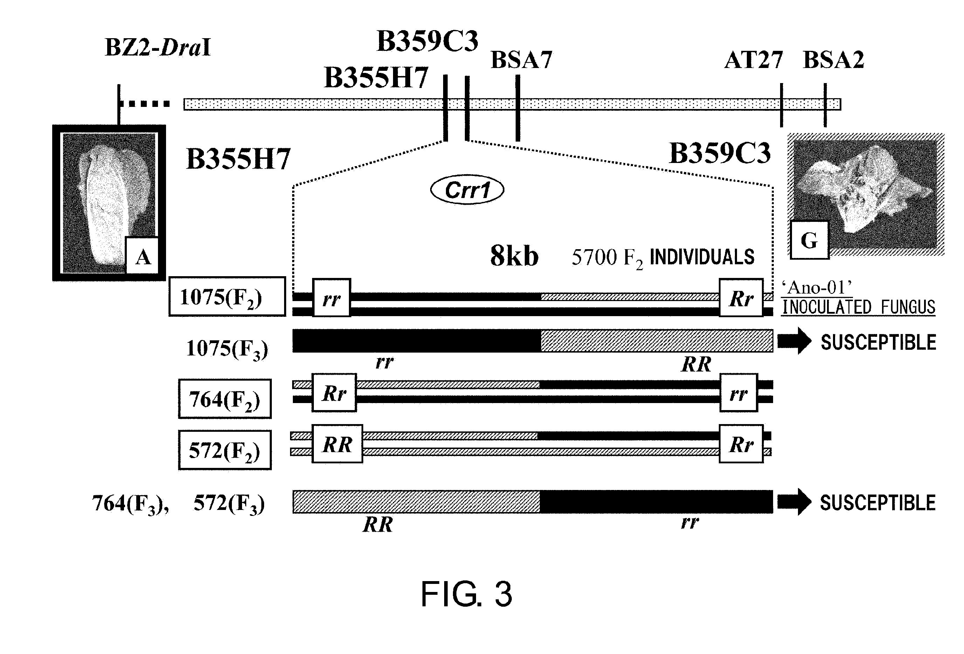

Fig. 3 depicts the marker genotypes of F₂ individuals in which recombination between nearby markers took place by homologous recombination near the Crr1 locus, and the presence of resistance in the F₃ generations of these individuals. The solid black region represents the susceptible variety, Chinese cabbage intermediate mother plant 7 (hereinafter, PL7), and the striped region represents the genome of the resistant line G004. The F₃ individuals 1075, 764, and 572 all show disease susceptibility, and did not carry the resistance gene. Therefore, Crr1 was strongly suggested not to be located at the BSA7-side of B359C3, nor at the BZ2-DraI-side of B359H7, but between B355H7 and B359C3.
Fig. 4 depicts the major (more than 10-bp long) insertion/deletion sequences and the translation regions (solid black regions) of Crr1 determined by comparing the Crr1-containing genomic regions of the resistant line G004 and the susceptible cultivar PL7. "In" indicates the sequence inserted in PL7 and "Del" indicates the sequence deleted in PL7 when compared to G004, and the number at the beginning indicates the number of nucleotides.
Fig. 5 shows the sequence of Crr1 (SEQ ID NO: 1) used in the complementarity test. The start codon and the stop codon are underlined.
Fig. 6 shows the amino acid sequence of Crr1 (SEQ ID NO: 2). Translation into 1224 amino acid residues was estimated.
Fig. 7 depicts the structure of the Crr1 protein estimated from the amino acid sequence.
Fig. 8 shows a photograph indicating the difference in expression of Crr1 determined by RT-PCR in the leaves and roots of the resistant line (R4-8-1) and the susceptible cultivar PL7. R: roots; L: leaves; V-ATP refers to a constitutively expressed gene (positive control).
Fig. 9 depicts the construct used in the complementarity test. Crr1 cDNA: Crr1 cDNA sequence; NPT II: kanamycin-resistance gene; LsUb-Pro: lettuce ubiquitin promoter; and LsUb-Ter: lettuce ubiquitin terminator.
Fig. 10 shows photographs showing the phenotype of *Arabidopsis thaliana* introduced with lettuce ubiquitin promoter (Up)::Crr1 cDNA. Individuals introduced with Crr1 showed resistance against Ann -01.
Fig. 11 shows a photograph indicating the results of determining the presence of clubroot resistance gene Crr1 by comparing the lengths of the amplified fragments of B359C3. Using each of the DNAs of A, F1, and PL9 as templates, fragments amplified by PCR with B359C3 were fractionated by agarose gel electrophoresis. A: individuals not carrying Crr1; F1 : individuals heterozygous for Crr1; PL9: individuals homozygous for Crr1; M: 100 bp ladder DNA size marker.
Fig. 12 depicts the results of comparing the nucleotide sequences of the resistant line G004 and the susceptible cultivar PL7. In the susceptible cultivar PL7, 357 bp have been found to be inserted 60 bp downstream of the start codon, and a stop codon has been found to exist in frame within exon 1. In the figure, the sequence indicated as G004 shows positions 2525 to 2707 of SEQ ID NO: 3. In the figure, the sequence indicated as PL7 shows positions 2602 to 3141 of SEQ ID NO: 13.
Fig. 13-1 shows the result of sequence comparison between the genomic sequence of the resistant line G004 (SEQ ID NO: 3) and the genomic sequence of the susceptible cultivar PL7 (SEQ ID NO: 13).
Fig. 13-2 is a continuation of Fig. 13-1.
Fig. 13-3 is a continuation of Fig. 13-2.
Fig. 13-4 is a continuation of Fig. 13-3.
Fig. 13-5 is a continuation of Fig. 13-4.
Fig. 13-6 is a continuation of Fig. 13-5.
Fig. 13-7 is a continuation of Fig. 13-6.
Fig. 13-8 is a continuation of Fig. 13-7.
Fig. 13-9 is a continuation of Fig. 13-8.

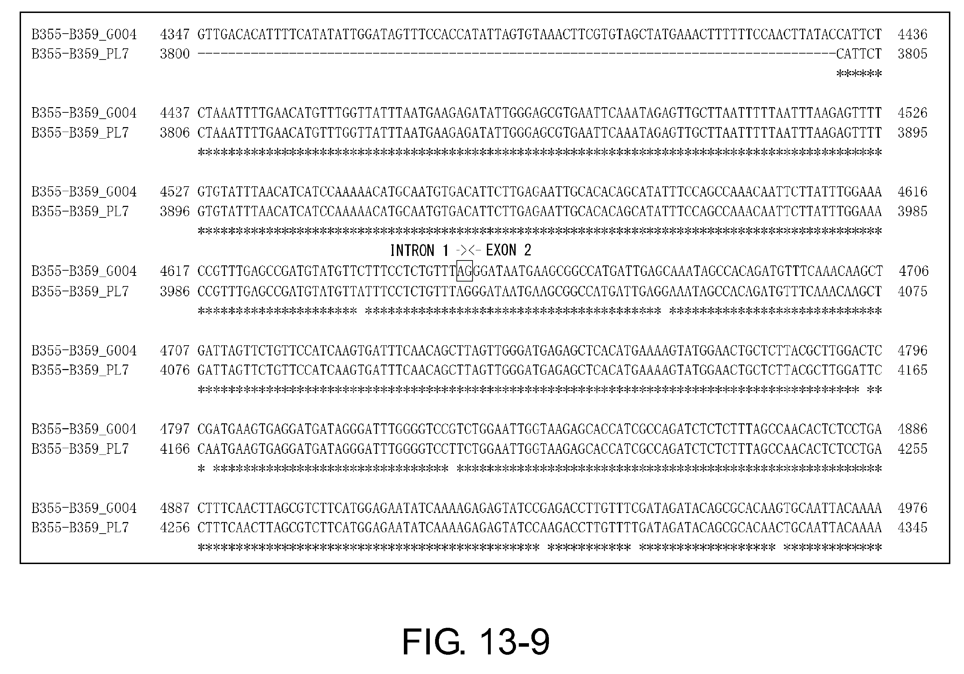

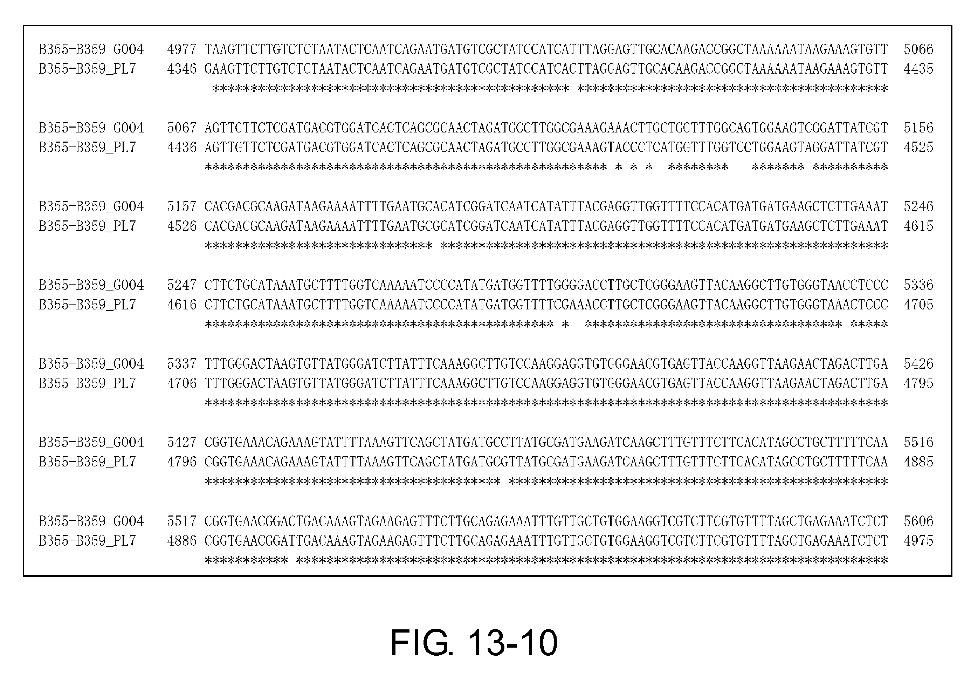

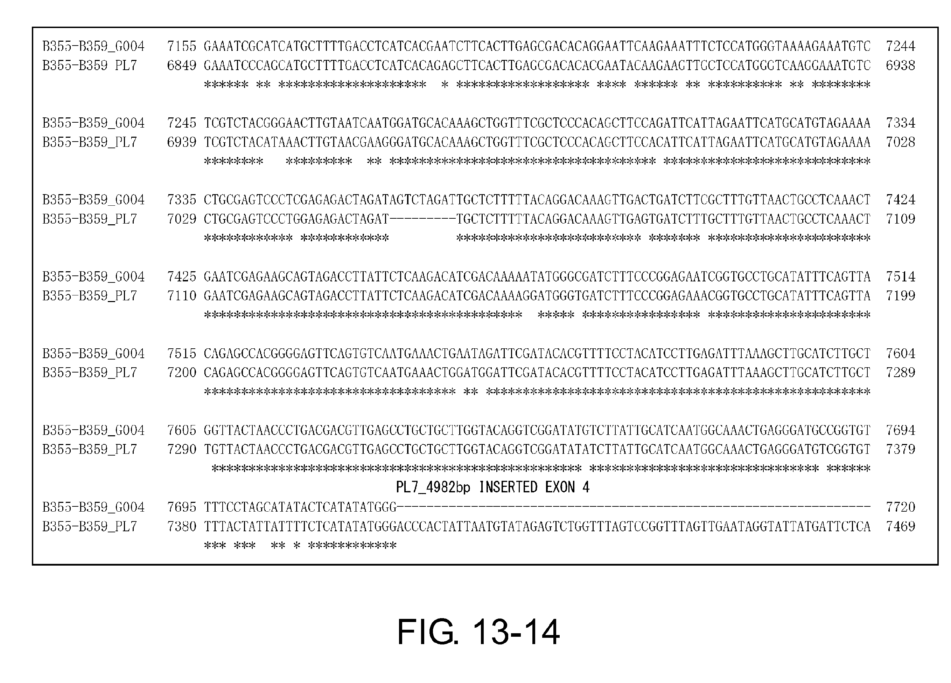

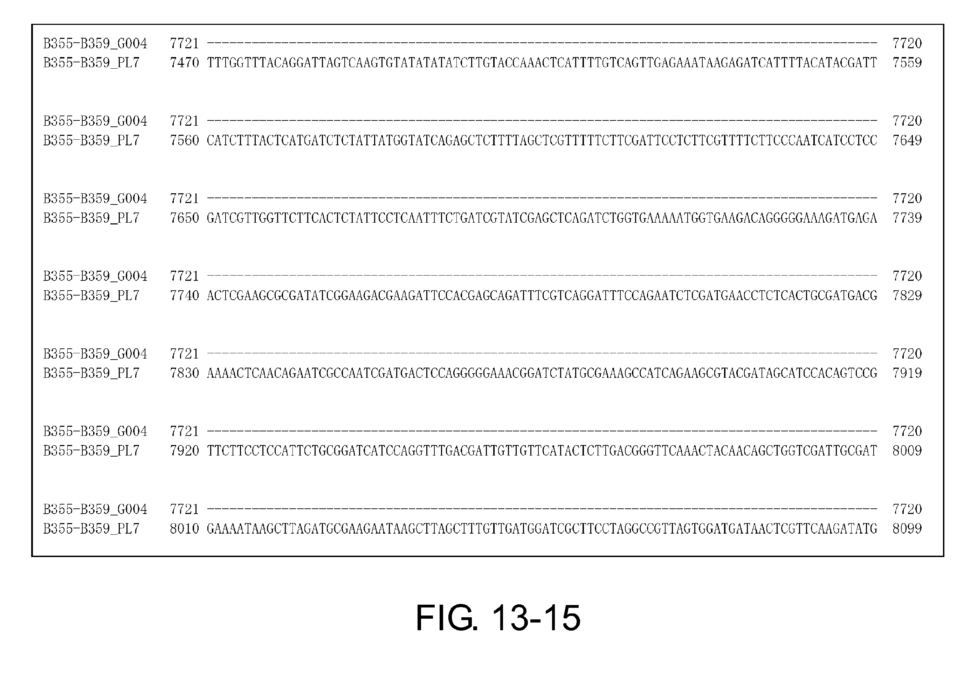

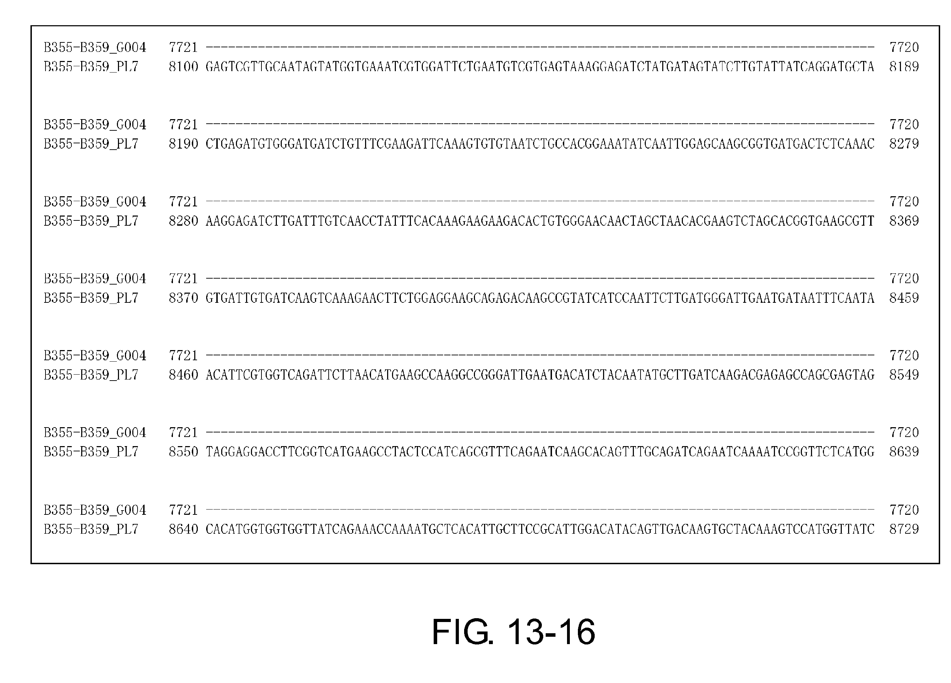

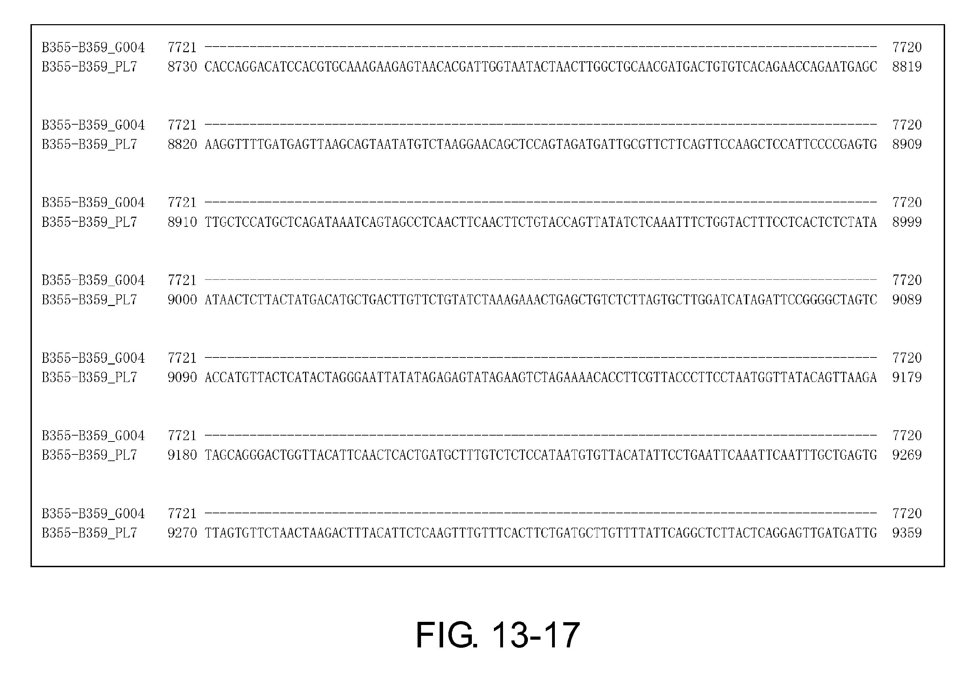

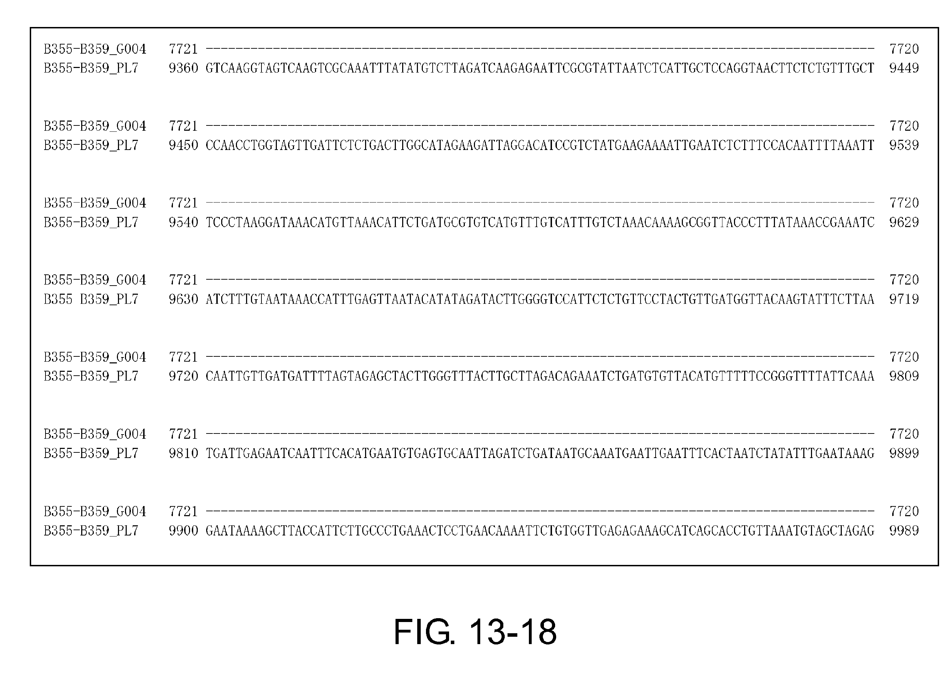

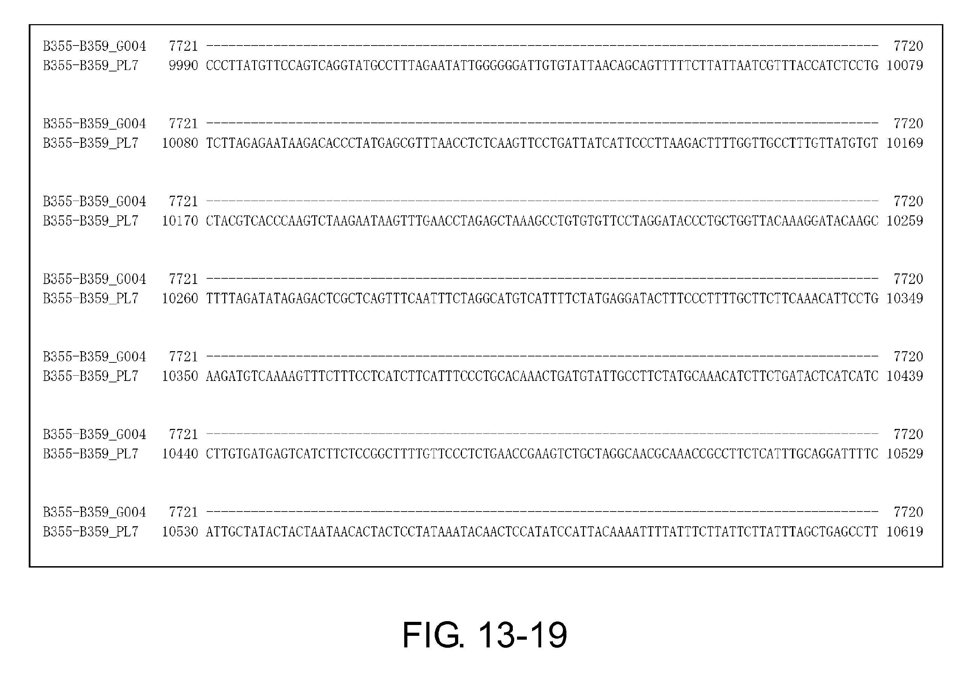

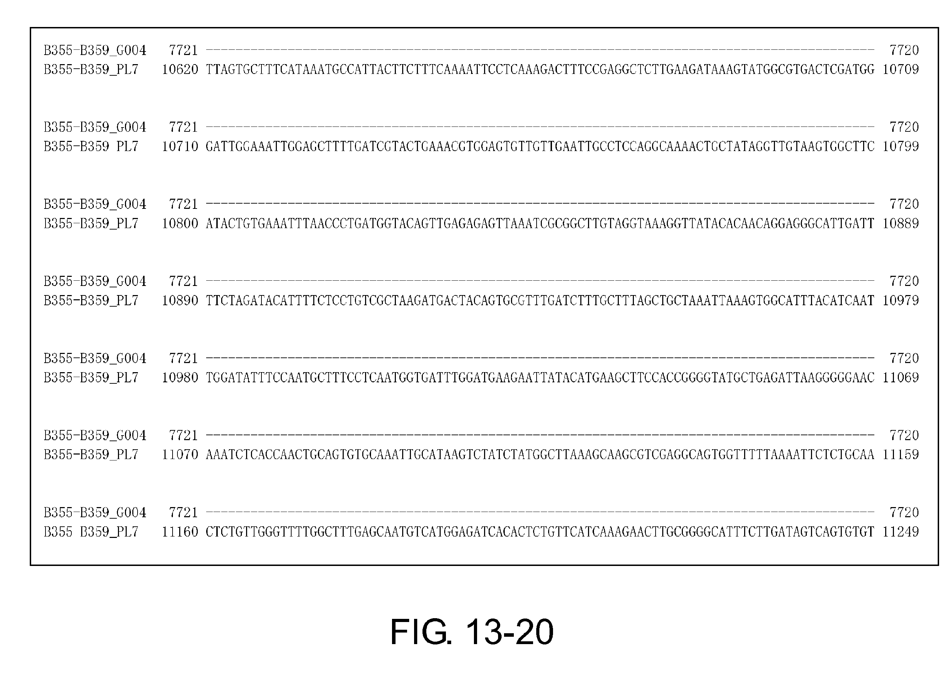

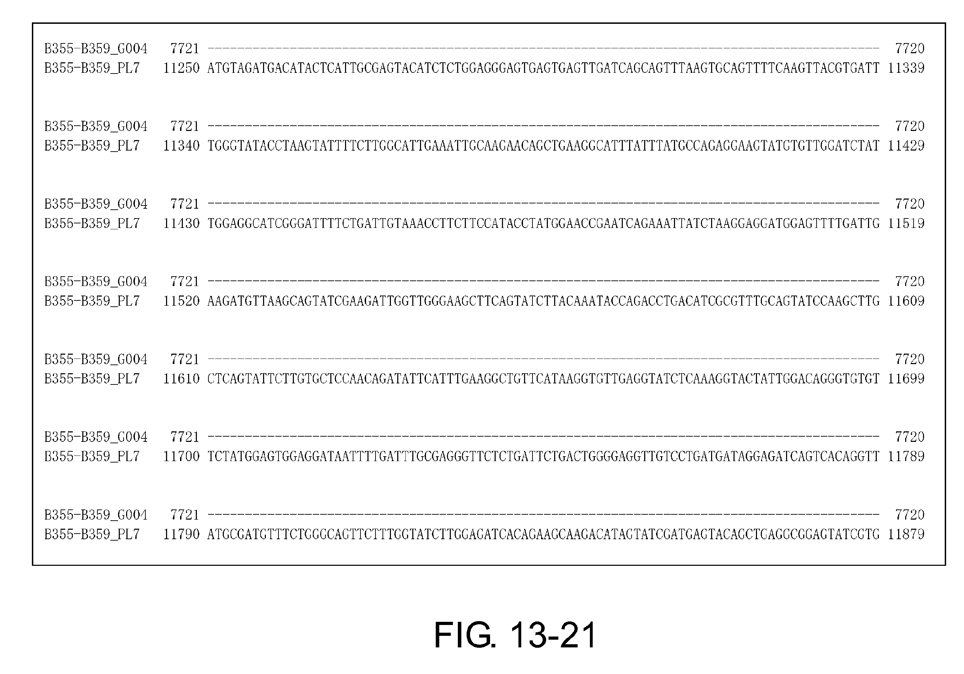

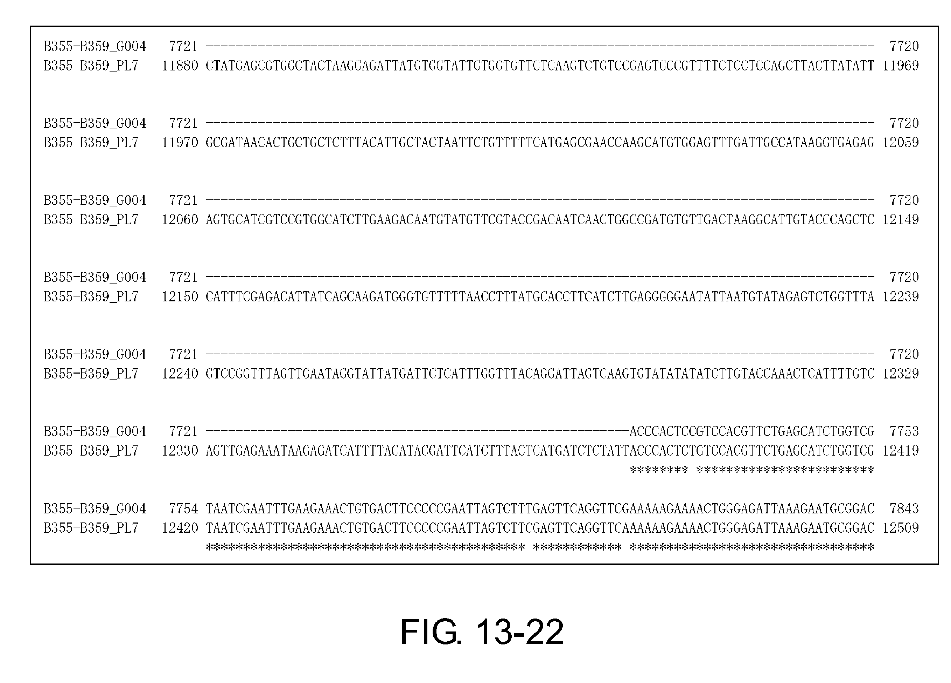

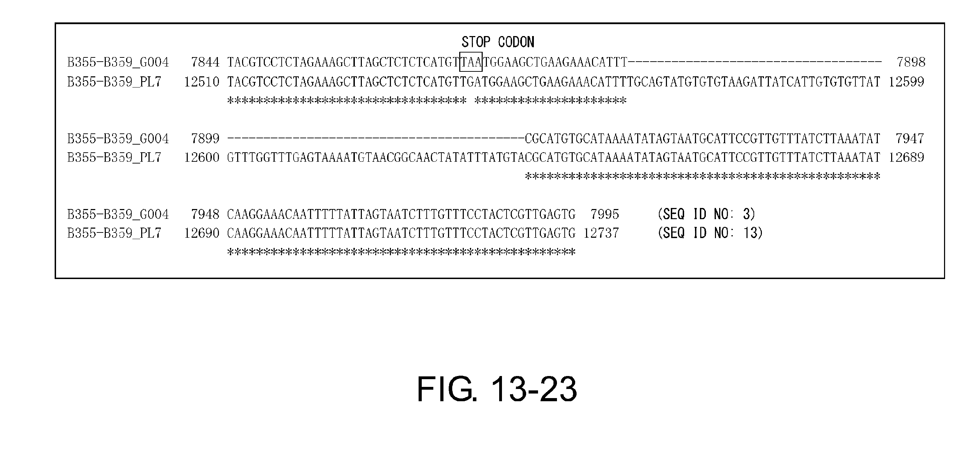

Fig. 13-10 is a continuation of Fig. 13-9.

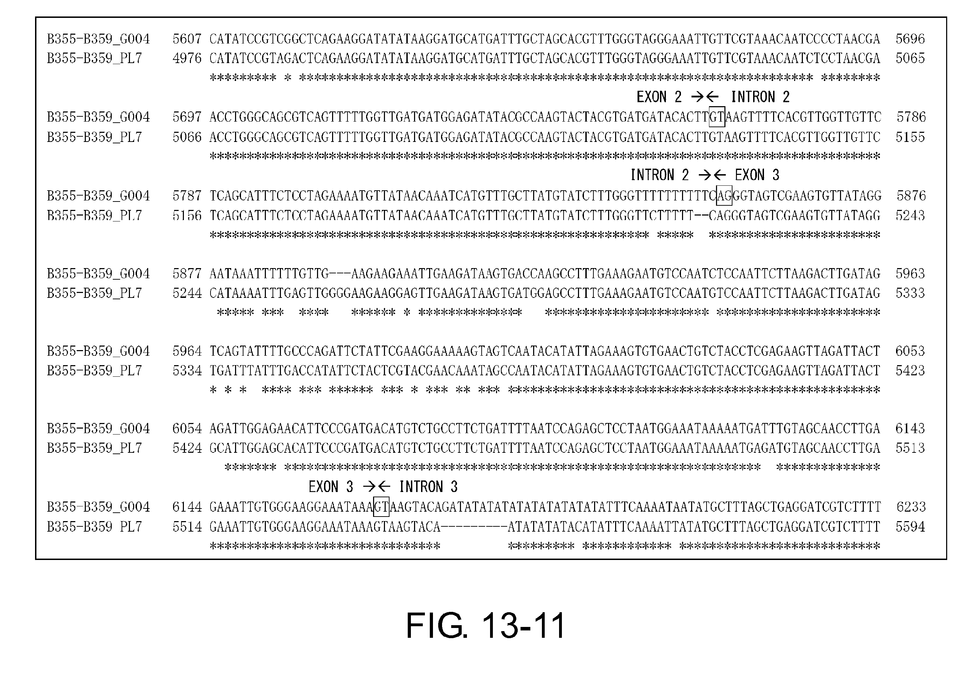

Fig. 13-11 is a continuation of Fig. 13-10.

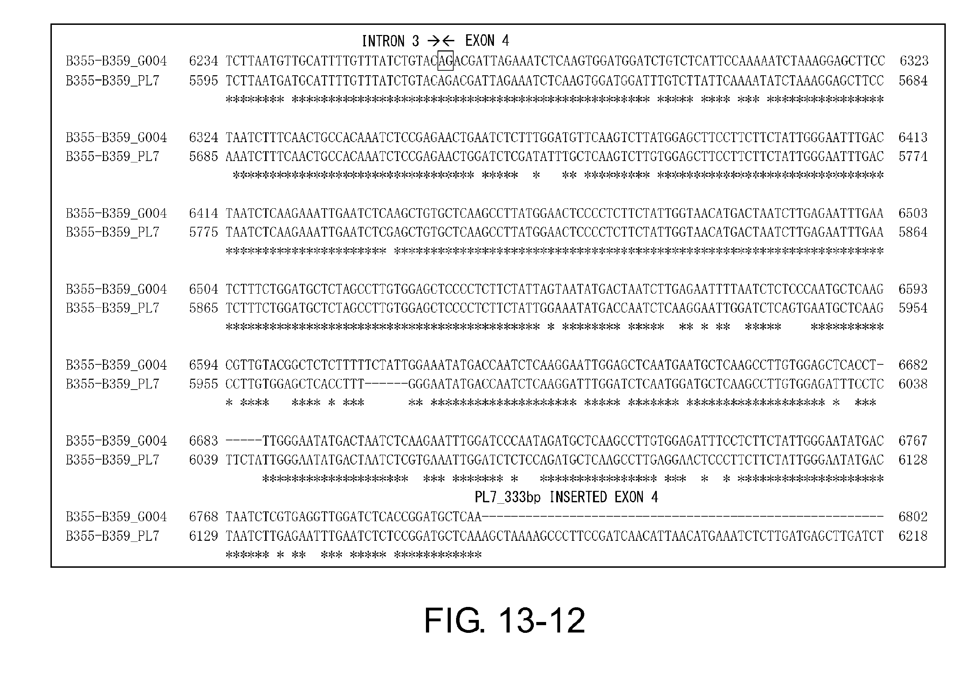

Fig. 13-12 is a continuation of Fig. 13-11.

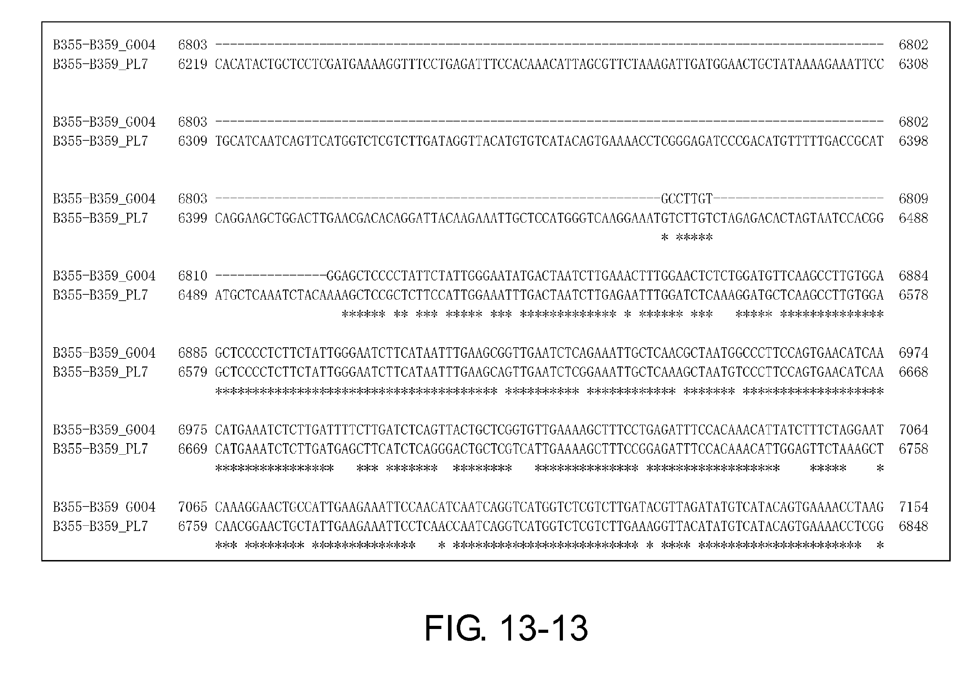

Fig. 13-13 is a continuation of Fig. 13-12.

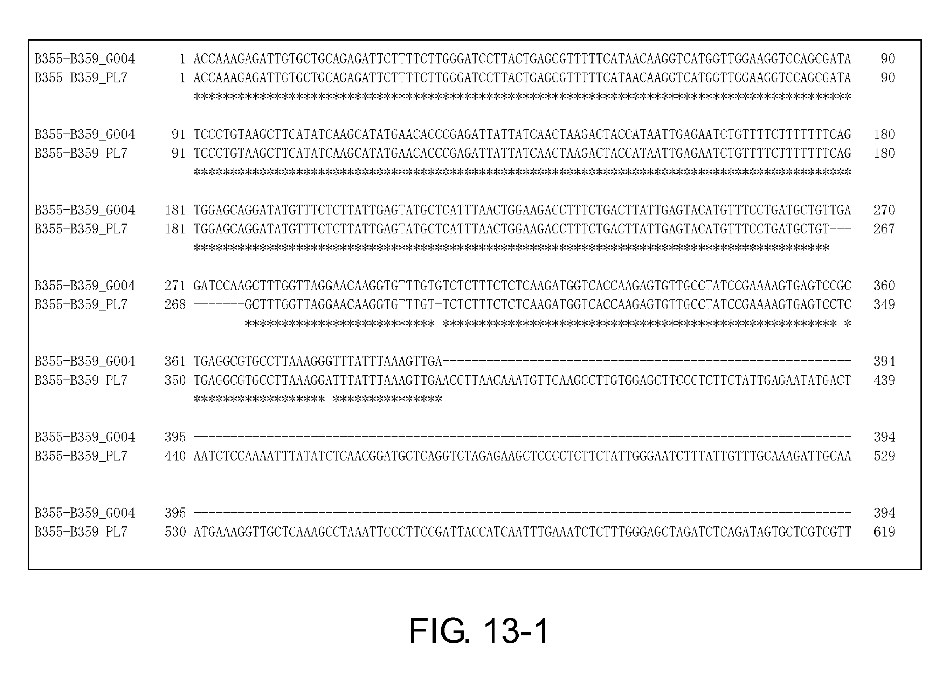

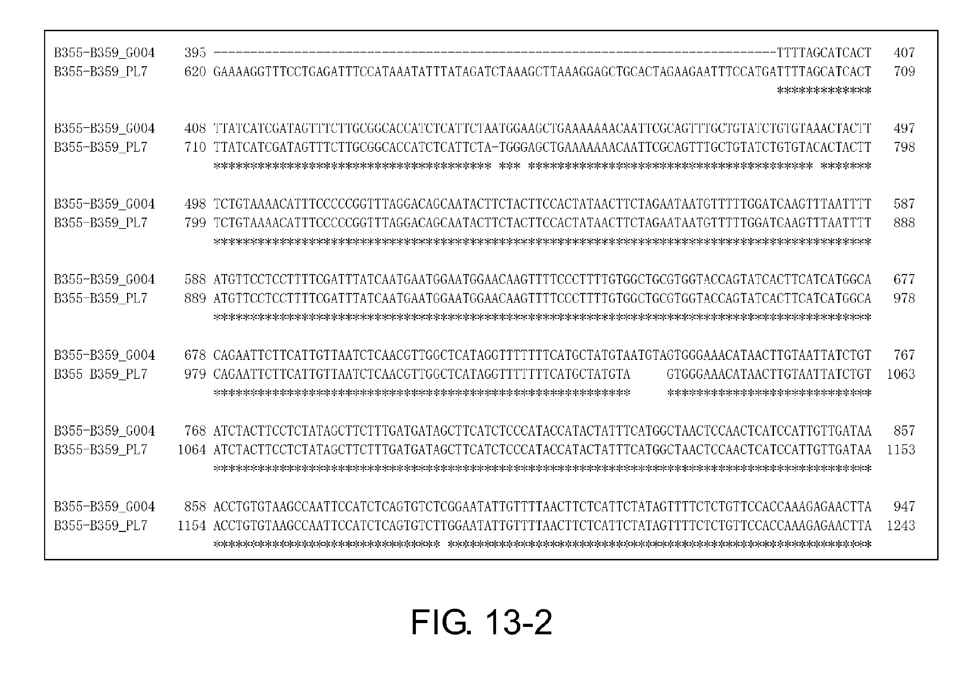

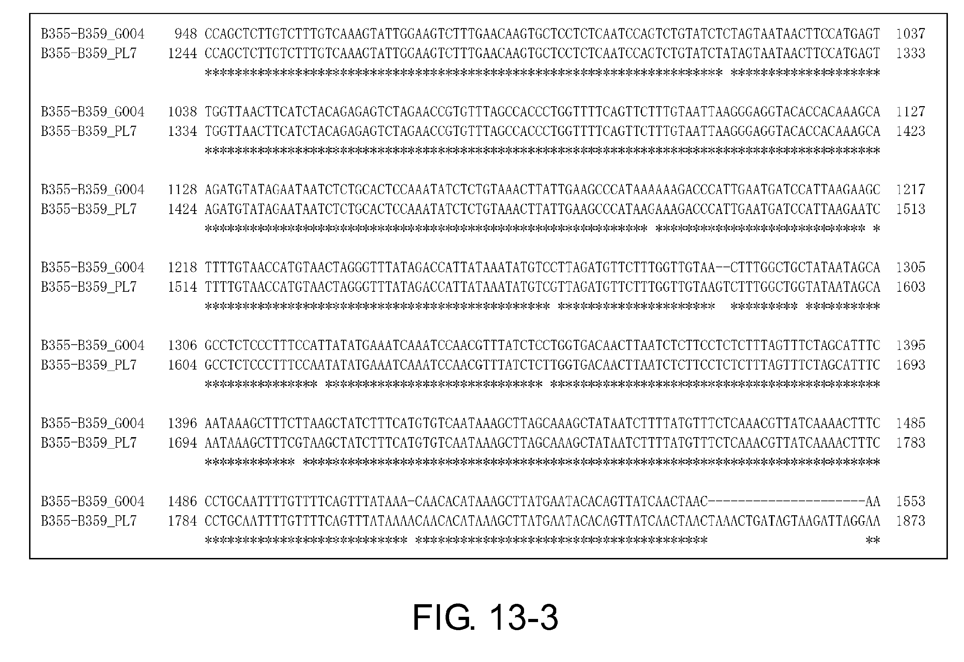

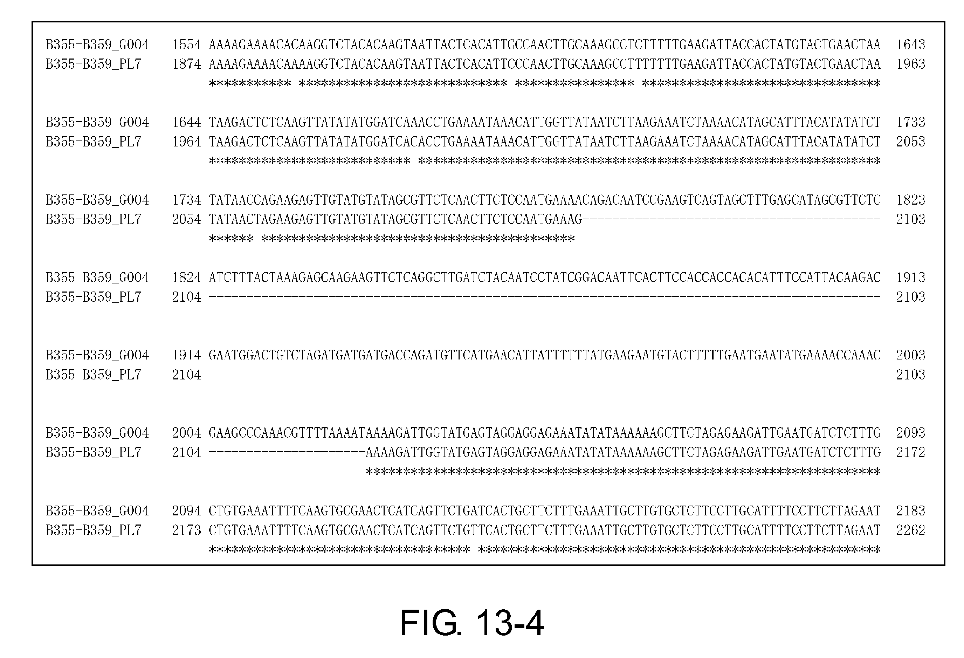

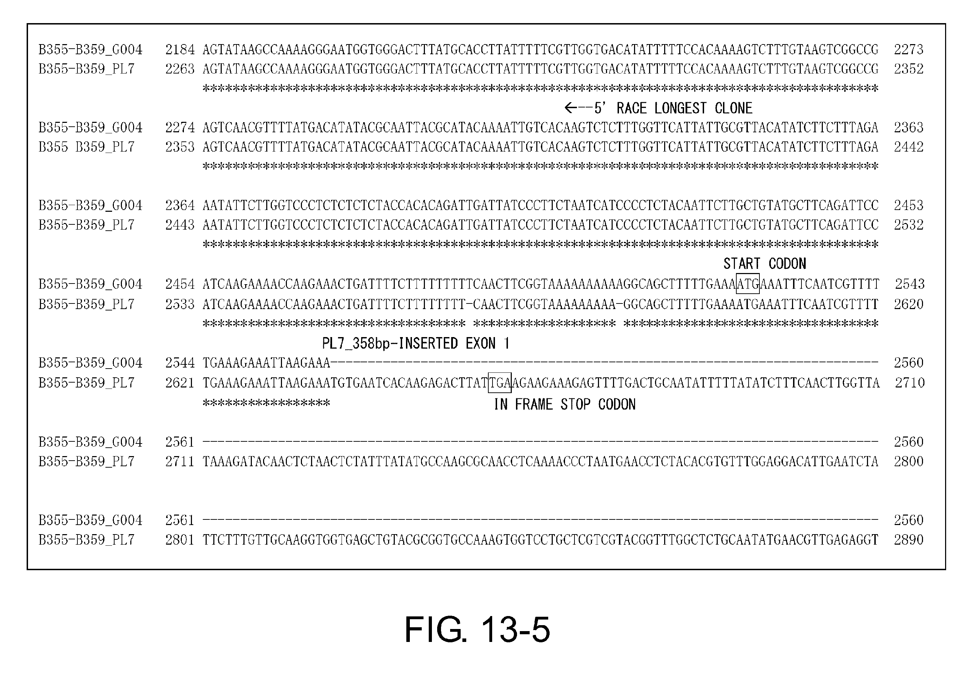

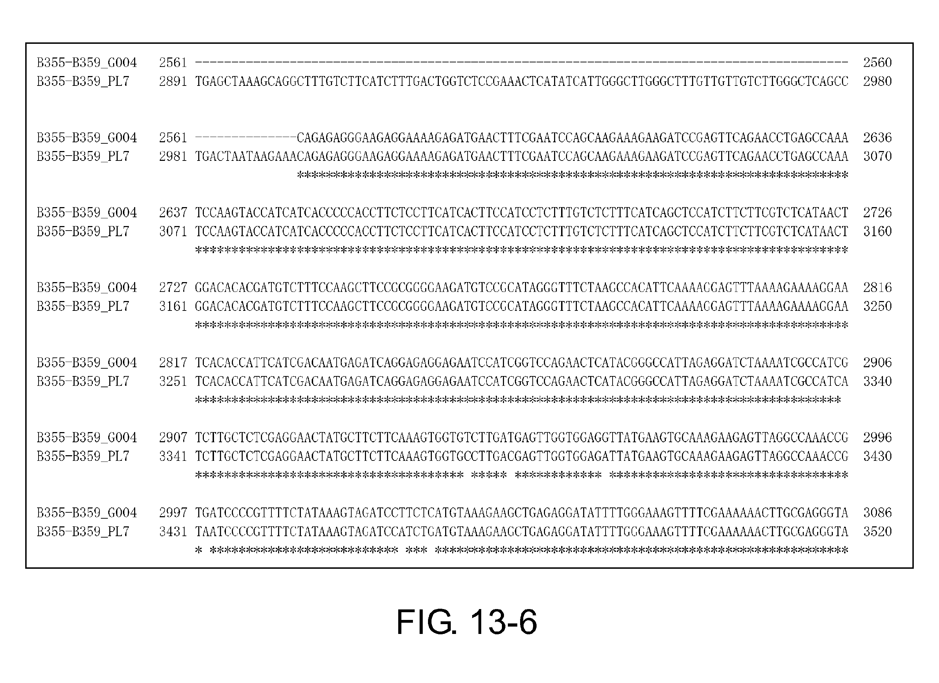

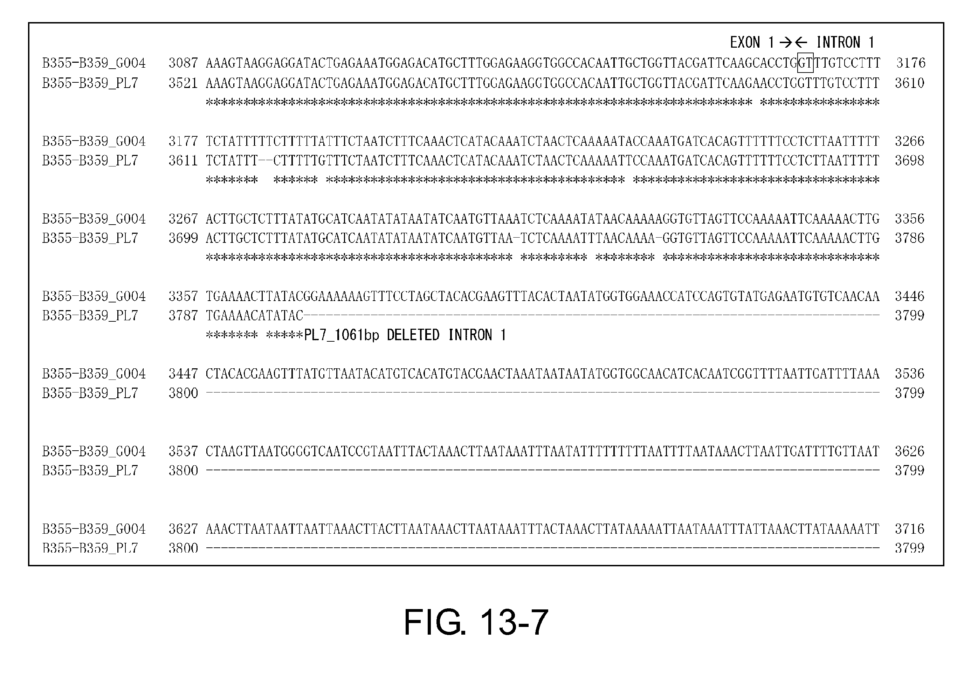

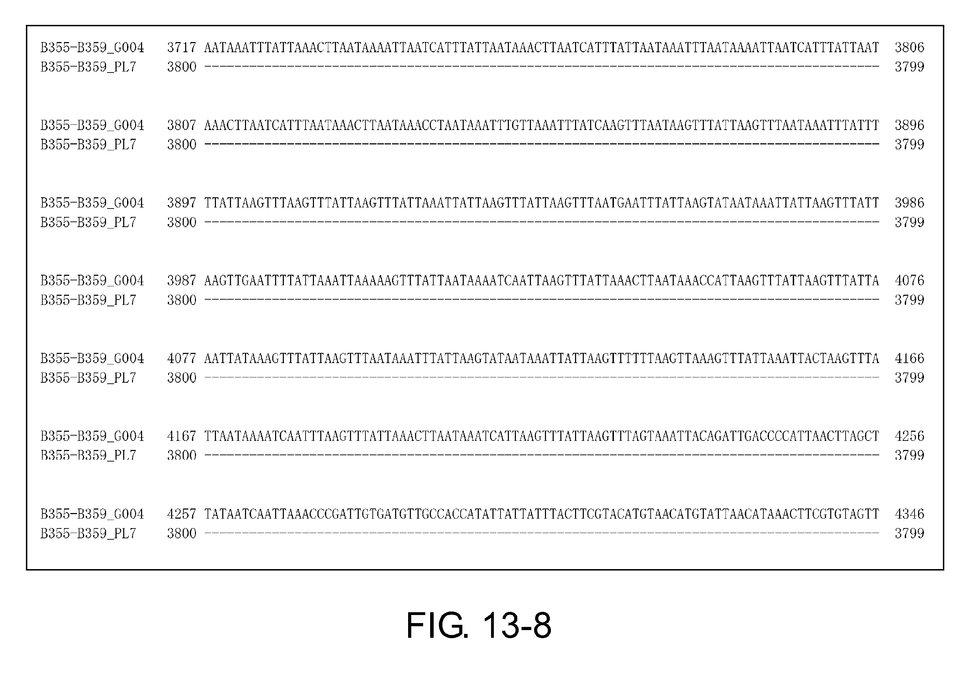

Fig. 13-14 is a continuation of Fig. 13-13.
Fig. 13-15 is a continuation of Fig. 13-14.
Fig. 13-16 is a continuation of Fig. 13-15.
Fig. 13-17 is a continuation of Fig. 13-16.
Fig. 13-18 is a continuation of Fig. 13-17.
Fig. 13-19 is a continuation of Fig. 13-18.
Fig. 13-20 is a continuation of Fig. 13-19.
Fig. 13-21 is a continuation of Fig. 13-20.
Fig. 13-22 is a continuation of Fig. 13-21.
Fig. 13-23 is a continuation of Fig. 13-22.

### Mode for Carrying Out the Invention

The present inventors isolated genes conferring clubroot fungus resistance. A preferred embodiment of the above-mentioned genes of the present invention includes, for example, the Crr1 gene of Chinese cabbage.

In the present invention, "having clubroot fungus resistance" not only means that the subject plants have resistance to clubroot fungus, but also refers to conferring to the subject plants resistance against clubroot fungus.

The genomic sequence of the Crr1 gene identified by the present invention is shown in SEQ ID NO: 3, the cDNA nucleotide sequence is shown in SEQ ID NO: 1, and the amino acid sequence encoded by the nucleotide sequence is shown in SEQ ID NO: 2.

Specifically, the present invention provides a polynucleotide having clubroot fungus resistance, which is described in any one of (a) to (d) below:
(a) a polynucleotide encoding a protein comprising the amino acid sequence of SEQ ID NO: 2;
(b) a polynucleotide comprising the coding region of the nucleotide sequence of SEQ ID NO: 1;
(c) a polynucleotide encoding a protein comprising an amino acid sequence with one or more amino acid substitutions, deletions, additions, and/or insertions in the amino acid sequence of SEQ ID NO: 2; and
(d) a polynucleotide that hybridizes under stringent conditions to a complementary strand of the nucleotide sequence of SEQ ID NO: 1 or SEQ ID NO: 3.

In the present invention, a polynucleotide of any one of (a) to (d) mentioned above may be described as a "polynucleotide of the present invention".

Furthermore, the term "polynucleotide" used in the present invention refers to a ribonucleotide or a deoxynucleotide, and indicates a polymer comprising multiple bases or base pairs. Single-stranded and double-stranded DNAs are included in polynucleotides. Polynucleotides include those that are not modified as well as those that are modified from the naturally-occurring state. Examples of modified bases include tritylated bases and unconventional bases such as inosine.

The term "nucleic acid" in the present invention means RNA or DNA. Furthermore, chemosynthetic nucleic acid analogs such as the so-called PNAs (peptide nucleic acids) are also included in the nucleic acid of the present invention. PNA is a molecule in which the pentose-phosphate backbone which is the basic backbone structure of a nucleic acid is substituted with a polyamide backbone composed of glycine units, and has a three-dimensional structure very similar to that of nucleic acids.

Furthermore, polynucleotides having clubroot fungus resistance of the present invention are not necessarily limited to polynucleotides comprising a nucleotide sequence specifically described in the Sequence Listing, or polynucleotides encoding a protein comprising an amino acid sequence specifically described in the Sequence Listing.

Proteins other than those described above are included in the proteins of the present invention, for example, when they are highly homologous (ordinarily, 70% or higher, preferably 80% or higher, more preferably 90% or higher, most preferably 95%, 96%, 97%, 98%, 99%, or higher homology) to the sequences described in the Sequence Listing, and maintain the functions (for example, clubroot fungus resistance) possessed by the proteins of the present invention.

Polynucleotides of the present invention include, for example, endogenous polynucleotides (homologs, etc.) in other organisms (plants), which correspond to a polynucleotide comprising the nucleotide sequence of SEQ ID NO: 1.

Furthermore, endogenous polynucleotides in other organisms, which correspond to a polynucleotide comprising the nucleotide sequence of SEQ ID NO: 1, are generally highly homologous to the polynucleotide of SEQ ID NO: 1. Highly homologous means a homology of 50% or higher, preferably 70% or higher, more preferably 80% or higher, and even more preferably 90% or higher (for example, 95% or higher, or even 96%, 97%, 98%, or 99% or higher). Such homology can be determined using the mBLAST algorithm (Altschul et al. (1990) Proc. Natl. Acad. Sci. USA 87: 2264-8; Karlin and Altschul (1993) Proc. Natl. Acad. Sci. USA 90: 5873-7).

Furthermore, when the polynucleotides are isolated from a living body, they are thought to hybridize under stringent conditions with the polynucleotide of SEQ ID NO: 1. Here, "stringent conditions" include, for example, conditions of "2x SSC, 0.1% SDS, 50°C", "2x SSC, 0.1% SDS, 42°C", and "1x SSC, 0.1% SDS, 37°C", and more stringent conditions include conditions of "2x SSC, 0.1% SDS, 65°C", "0.5x SSC, 0.1% SDS, 42°C", and "0.2x SSC, 0.1% SDS, 65°C". Based on the nucleotide sequence of SEQ ID NO: 1, those skilled in the art can appropriately obtain endogenous polynucleotides in other organisms that are equivalent to the polynucleotide of SEQ ID NO: 1.

The present invention also includes proteins encoded by the polynucleotides of the present invention. In the present invention, proteins encoded by the polynucleotides of the present invention may be described as "proteins of the present invention".

The term "proteins" used in the present invention means polymers formed from multiple amino acids. Therefore, proteins of the present invention also include the so-called "polypeptides" and "oligopeptides". Proteins of the present invention include those that are not modified as well as those that are modified from the naturally-occurring state. Modifications include acetylation, acylation, ADP-ribosylation, amidation, covalent attachment of flavin, covalent attachment of a heme moiety, covalent attachment of a nucleotide or nucleotide derivative, covalent attachment of a lipid or lipid derivative, covalent attachment of phosphatidylinositol, cross-linking, cyclization, disulfide bond formation, demethylation, formation of covalent cross-links, formation of cystine, formation of pyroglutamate, formylation, γ-carboxylation, glycosylation, GPI anchor formation, hydroxylation, iodination, methylation, myristoylation, oxidation, proteolytic processing, phosphorylation, prenylation, racemization, selenoylation, sulfation, transfer-RNA mediated addition of amino acids to proteins such as arginylation, and ubiquitination.

Proteins of the present invention can be produced by general chemical synthesis methods according to their amino acid sequences, and such methods include peptide synthesis methods by common liquid-phase methods and solid-phase methods. More specifically, such peptide synthesis methods may include the stepwise elongation method, in which each amino acid is successively synthesized one by one based on the amino acid sequence information to lengthen the chain, and the fragment condensation method, in which fragments containing several amino acids are synthesized in advance, and then each of these fragments are subjected to coupling reactions. Either method may be used for the synthesis of the proteins of the present invention.

Condensation methods used in such peptide synthesis methods can be carried out according to various types of methods, and examples include the azide method, mixed acid anhydride method, DCC method, active ester method, oxidation-reduction method, diphenylphosphoryl azide (DPPA) method, and Woodward method.

For solvents to be used in these various methods, generally used solvents can be suitably used. Such examples include dimethylformamide (DMF), dimethylsulfoxide (DMSO), hexaphosphoroamide, dioxane, tetrahydrofuran (THF), ethyl acetate, and mixed solvents thereof. During the above-mentioned peptide synthesis reaction, the carboxyl groups of amino acids and peptides that are not involved in the reaction can generally be protected by esterification, for example, as lower alkyl esters such as methyl ester, ethyl ester, or tertiary butyl ester, or as benzyl ester, *p*-methoxybenzyl ester, or *p*-nitrobenzyl ester aralkyl ester. Furthermore, the hydroxyl group of an amino acid having a functional group on its side chain, for example, Tyr, may be protected by an acetyl group, a benzyl group, a benzyloxycarbonyl group, a tertiary butyl group, or such, but such protection is not necessarily essential. Furthermore, for example, Arg can have its guanidino group protected by a suitable protecting group such as a nitro group, tosyl group, 2-methoxybenzenesulfonyl group, mesitylene-2-sulfonyl group, benzyloxycarbonyl group, isobornyloxycarbonyl group, or adamantyloxycarbonyl group.

Proteins of the present invention include, for example, proteins that are functionally equivalent to the proteins of the present invention. Herein, "functionally equivalent" means that the protein of interest has a biological or biochemical function (activity) similar or equivalent to that of a protein of the present invention. Examples of such functions include clubroot fungus resistance or clubroot fungus resistance activity.

The most common method for evaluating whether or not a certain polynucleotide encodes a protein having clubroot fungus resistance is, for example, the method of cultivating a plant that has been introduced with the polynucleotide and evaluating the plant's level of resistance to clubroot fungus.

Examples of methods for preparing a protein functionally equivalent to a certain protein that are well known to those skilled in the art include methods for introducing mutations into the amino acid sequence of a protein. For example, those skilled in the art can prepare a protein functionally equivalent to the above-mentioned proteins by introducing appropriate mutations into the amino acid sequence of SEQ ID NO: 2 using site-directed mutagenesis or such (Hashimoto-Gotoh, T. et al. (1995) Gene 152, 271-275; Zoller, MJ, and Smith, M.(1983) Methods Enzymol. 100, 468-500; Kramer, W. et al. (1984) Nucleic Acids Res. 12, 9441-9456; Kramer W, and Fritz HJ (1987) Methods. Enzymol. 154, 350-367; Kunkel, TA(1985) Proc. Natl. Acad. Sci. USA. 82, 488-492 and Kunkel (1988) Methods Enzymol. 85, 2763-2766). Amino acid mutations in a protein may also occur naturally. Regardless of whether they are artificial or naturally-occurring, proteins functionally equivalent to the above-mentioned proteins, which comprise an amino acid sequence in which one or more amino acid sequences are mutated in the amino acid sequence of SEQ ID NO: 2, are included in the proteins of the present invention.

The above-mentioned proteins are not limited as long as they maintain the functions possessed by the proteins of the present invention, and include, for example, a protein comprising an amino acid sequence with one or more amino acid additions, deletions, substitutions, or insertions in the amino acid sequence of SEQ ID NO: 2. The number of modified amino acids is not particularly limited as long as the modified proteins have the aforementioned functions, but are ordinarily 50 amino acids or less, preferably 30 amino acids or less, and more preferably ten amino acids or less (for example, five amino acids or less, or three amino acids or less). Alternatively, in the entire amino acid sequence, for example, modifications of 20% or less, or more specifically 10% or less (for example, 10, 9, 8, 7, 6, 5, 4, 3, 2, or 1% or less) of the amino acid residues are acceptable. That is, proteins containing amino acid sequences that have homology of preferably 80% or more, or more preferably 90% or more (for example, 90, 91, 92, 93, 94, 95, 96, 97, 98, or 99% or more) to the amino acid sequence of SEQ ID NO: 2 are also included in the proteins of the present invention.

Generally, to maintain protein function, amino acids to be substituted are preferably amino acids that have properties similar to those of the amino acids before substitution. Such amino acid residue substitutions are called conservative substitutions. For example, since Ala, Val, Leu, Ile, Pro, Met, Phe, and Trp are all classified as non-polar amino acids, their properties are similar to each other. Uncharged amino acids include Gly, Ser, Thr, Cys, Tyr, Asn, and Gln. Furthermore, acidic amino acids include Asp and Glu. Furthermore, basic amino acids include Lys, Arg, and His. Amino acid substitutions within each of these groups are acceptable.

The amino acid residues to be mutated are desirably mutated to other amino acids in which the properties of the amino acid side chain are conserved. Examples of amino acid side chain properties are: hydrophobic amino acids (A, I, L, M, F, P, W, Y, and V), hydrophilic amino acids (R, D, N, C, E, Q, G, H, K, S, and T), and amino acids having the following side chains: aliphatic side chains (G, A, V, L, I, and P); hydroxyl-containing side chains (S, T, and Y); sulfur-containing side chains (C and M); carboxylic acid- and amide-containing side chains (D, N, E, and Q); basic side chains (R, K, and H); and aromatic ring-containing side chains (H, F, Y, and W) (all amino acids are represented by one-letter codes in parentheses).

A protein having a modified amino acid sequence, in which one or more amino acid residues are deleted, added, and/or substituted with other amino acids in a certain amino acid sequence, is known to be able to retain its biological function (activity) (Mark, D. F. et al., Proc. Natl. Acad. Sci. USA (1984) 81, 5662-5666; Zoller, M. J. & Smith, M. Nucleic Acids Research (1982) 10, 6487-6500; Wang, A. et al., Science 224, 1431-1433; and Dalbadie-McFarland, G. et al., Proc. Natl. Acad. Sci. USA (1982) 79, 6409-6413).

When a specific amino acid sequence (for example SEQ ID NO: 2) is disclosed, those skilled in the art can appropriately select a protein of the present invention by appropriately producing a protein comprising the amino acid-modified sequence based on this amino acid sequence, and evaluating whether or not the protein has the desired function.

Proteins in which several amino acid residues have been added to an amino acid sequence of a protein of the present invention include fusion proteins containing these proteins. Fusion proteins are proteins in which such a protein is fused to another protein. A fusion protein can be prepared by a method that ligates a polynucleotide (for example, SEQ ID NO: 1) encoding a protein of the present invention (for example, SEQ ID NO: 2) to a polynucleotide encoding another protein such that their frames are in line, inserts this into an expression vector, and expresses it in a host; and techniques known to those skilled in the art can be used. The other peptide or polypeptide to be fused with a protein of the present invention is not particularly limited.

Examples of other proteins to be fused to the proteins of the present invention include, GST (glutathione-S-transferase), immunoglobulin constant region, β-galactosidase, MBP (maltose-binding protein), and such. Commercially available polynucleotides encoding these proteins can be fused with polynucleotides encoding the proteins of the present invention. A fusion protein can be prepared by expressing the fusion polynucleotide prepared in this way.

Other methods that are well known to those skilled in the art for preparing proteins that are functionally equivalent to a certain protein include, for example, a method that uses hybridization techniques (Sambrook, J. et al., Molecular Cloning 2nd ed., 9.47-9.58, Cold Spring Harbor Lab. press, 1989). More specifically, based on the polynucleotide (the nucleotide sequence of SEQ ID NO: 1) encoding a protein of the present invention or a portion thereof, those skilled in the art can generally isolate polynucleotides highly homologous thereto from polynucleotide samples derived from organisms of the same or different species, and then from these polynucleotides, isolate proteins functionally equivalent to the proteins of the present invention.

The present invention includes proteins that are encoded by a polynucleotide which hybridizes with the polynucleotide encoding a protein of the present invention, and which are functionally equivalent to a protein of the present invention.

Those skilled in the art can appropriately select hybridization conditions for isolating polynucleotides encoding proteins that are functionally equivalent to proteins of the present invention. The hybridization conditions are, for example, low-stringency conditions. "Low-stringency conditions" refers to washing after hybridization under conditions such as 42°C, 0.1 x SSC, 0.1% SDS, or preferably 50°C, 0.1 x SSC, 0.1% SDS. More preferable hybridization conditions include high-stringency conditions. High-stringency conditions are, for example, conditions of 65°C, 5 x SSC, and 0.1% SDS. Under these conditions, increasing the temperature is expected to result in efficient yield of DNAs having higher homology. However, multiple factors such as temperature and salt concentration are considered to be factors affecting the hybridization stringency, and those skilled in the art can achieve similar stringencies by appropriately selecting these factors.

In place of hybridization, a gene amplification technique (PCR) may be used to isolate a polynucleotide fragment highly homologous to a polynucleotide encoding a protein of the present invention by designing primers based on a portion of the polynucleotide (for example, SEQ ID NO: 1) encoding a protein of the present invention (Current protocols in Molecular Biology edit. Ausubel et al., (1987) Publish. John Wiley & Sons Section 6.1-6.4); and based on this polynucleotide, a protein functionally equivalent to a protein of the present invention can be obtained.

The proteins of the present invention may be in the form of a "mature" protein, or may be a part of a larger protein such as a fusion protein. The proteins of the present invention may contain leader sequences, pro-sequences, sequences which are useful in purification, such as multiple histidine residues, or additional sequences for securing stability during recombinant production.

Proteins functionally equivalent to proteins of the present invention, which are encoded by polynucleotides isolated by the above-mentioned hybridization techniques or gene amplification techniques, generally have high amino acid sequence homology with proteins of the present invention (for example, SEQ ID NO: 2). Proteins that are functionally equivalent to the proteins of the present invention, and have high amino acid sequence homology with these proteins are also included in the proteins of the present invention. High homology usually refers to an identity at the amino acid level of at least 50% or higher, preferably 75% or higher, more preferably 85% or higher, and even more preferably 95% or higher (for example, 96% or higher, 97% or higher, 98% or higher, or 99% or higher). Protein homology can be determined by following the algorithm described in the literature (Wilbur, W. J. and Lipman, D. J. Proc. Natl. Acad. Sci. USA (1983) 80, 726-730).

The amino acid sequence identity can be determined, for example, by the BLAST algorithm of Karlin and Altschul (Proc. Natl. Acad. Sci. USA 87, :2264-2268, 1990; Proc. Natl. Acad. Sci. USA, 90: 5873-5877, 1993). A program called BLASTX has been developed based on this algorithm (Altschul et al., J. Mol. Biol. 215:, 403-410, 1990). When amino acid sequences are analyzed by BLASTX, parameters are set, for example, at score = 50 and wordlength = 3. When using the BLAST and Gapped BLAST programs, the default parameters of each program are used. Specific procedures for these analytical methods are known (http://www.ncbi.nlm.nih.gov).

The proteins of the present invention can be prepared as a recombinant protein or as a naturally-occurring protein by methods known to those skilled in the art. Recombinant proteins can be prepared, for example, by incorporating a polynucleotide encoding a protein of the present invention (for example, the nucleotide sequence of SEQ ID NO: 1) into a suitable expression vector, harvesting transformants obtained by introducing this vector into suitable host cells, and obtaining their extracts and appropriately purifying them according to common methods generally used in the field of peptide chemistry such as ion exchange resin, partition chromatography, gel chromatography, affinity chromatography, high performance liquid chromatography (HPLC), and countercurrent distribution method.

When proteins of the present invention are expressed as fusion proteins with a glutathione S-transferase protein, or as recombinant proteins with multiple additions of histidines in host cells (for example, a plant cell or a microbial cell), the expressed recombinant proteins can be purified using a glutathione column or a nickel column. After the fused protein is purified, regions other than the protein of interest in the fused protein can be removed, as necessary, by cleavage with thrombin, factor Xa, or such.

Naturally derived proteins can be isolated by methods well known to those skilled in the art, for example, by purifying extracts of tissues or cells expressing the proteins of the present invention by subjecting them to an affinity column to which antibodies having affinity to the proteins of the present invention are bound. The antibodies that are used may be polyclonal antibodies or monoclonal antibodies.

Proteins of the present invention can be utilized, for example, in the production of antibodies that recognize the proteins of the present invention and such.

Polynucleotides of the present invention may be in any form as long as they can encode proteins of the present invention. That is, whether the polynucleotides are cDNAs synthesized from mRNAs, genomic DNAs, chemically synthesized DNAs, or such is not a concern. Furthermore, as long as a protein of the present invention is encoded, DNAs having an arbitrary nucleotide sequence based on genetic code degeneracy are also included.

Polynucleotides of the present invention can be prepared by methods known to those skilled in the art. For example, they can be prepared by producing a cDNA library from cells expressing the proteins of the present invention, then performing hybridization using a portion of the polynucleotides of the present invention (for example, the nucleotide sequence of SEQ ID NO: 1) as a probe. The cDNA library may be prepared, for example, by a method described in the literature (Sambrook, J. et al., Molecular Cloning, Cold Spring Harbor Laboratory Press (1989)), or a commercially available DNA library may be used. Alternatively, polynucleotides can be produced by preparing RNAs from cells expressing a protein of the present invention, synthesizing cDNAs using reverse transcriptase, and then synthesizing oligoDNAs based on the polynucleotides of the present invention (for example, the nucleotide sequence of SEQ ID NO: 1), and performing PCR reactions using them as primers to amplify the cDNAs encoding the proteins of the present invention.

By determining the nucleotide sequence of the obtained cDNA, the translation region encoded by the cDNA can be determined, and the amino acid sequence of the protein of the present invention can be obtained. Furthermore, the obtained cDNA can also be used as a probe for screening a genomic DNA library to isolate genomic DNAs.

Specifically, the following processes may be carried out. First, mRNAs are isolated from cells, tissues, or organs expressing a protein of the present invention. mRNAs are isolated using known methods, for example, by preparing total RNAs using guanidine ultracentrifugation methods (Chirgwin, J. M. et al., Biochemistry (1979) 18, 5294-5299), AGPC methods (Chomczynski, P. and Sacchi, N., Anal. Biochem. (1987) 162, 156-159), or such, and then purifying mRNAs from the total RNAs using an mRNA Purification Kit (Pharmacia) or such. The mRNAs can also be prepared directly by using the QuickPrep mRNA Purification Kit (Pharmacia).

cDNAs are synthesized from the obtained mRNAs using reverse transcriptase. cDNAs may be synthesized using the AMV Reverse Transcriptase First-strand cDNA Synthesis Kit (Seikagaku Corporation) and such. Alternatively, by using the primers and such described herein, cDNAs may be synthesized and amplified following the 5'-RACE method (Frohman, M. A. et al., Proc. Natl. Acad. Sci. USA (1988) 85, 8998-9002; Belyavsky, A. et al., Nucleic Acids Res. (1989) 17, 2919-2932) that uses the 5'-Ampli FINDER RACE Kit (manufactured by Clontech) and polymerase chain reaction (PCR). A polynucleotide fragment of interest is prepared from the obtained PCR products and linked to a vector. A recombinant vector is produced from this and introduced into *E. coli* and such, and colonies are selected to prepare a desired recombinant vector. The nucleotide sequence of the polynucleotide of interest can be confirmed through known methods such as the dideoxynucleotide chain termination method.

Furthermore, when producing polynucleotides of the present invention, the nucleotide sequences having higher expression efficiency can be designed by considering the codon usage frequency in the host used for expression (Grantham R. et al., Nucleic Acids Research (1981) 9, r43-74). Furthermore, polynucleotides of the present invention can be modified by commercially available kits or known methods. Examples of the modification include digestion with restriction enzymes, insertion of a synthetic oligonucleotide or a suitable DNA fragment, addition of a linker, and insertion of the initiation codon (ATG) and/or a stop codon (TAA, TGA, or TAG).

Furthermore, the present invention provides vectors into which polynucleotides of the present invention have been inserted.

In addition to the above-mentioned vectors used for recombinant protein production, vectors of the present invention include vectors for expressing polynucleotides of the present invention in plant cells for the production of transformed plants. Examples of preferred embodiments of the present invention include vectors in which a polynucleotide of the present invention is operably linked downstream of a promoter region that enables expression in plant cells. For example, they can contain a promoter sequence that enables transcription in plant cells and a terminator sequence containing a polyadenylation site necessary for stabilization of transcription products. A vector used for transformation of a plant cell is not particularly limited as long as it allows expression of an inserted gene in the cell. For example, a vector having a promoter for constitutively expressing a gene in plant cells, and a vector having a promoter that is inducibly activated by an external stimulus may be used.

A promoter for constitutively expressing a protein of the present invention may be, for example, the ubiquitin promoter from lettuce, the 35S promoter from cauliflower mosaic virus, the actin promoter from rice, or the ubiquitin promoter from maize.

Promoters for inducible expression include, for example, promoters known to be expressed by exogenous factors including bacterial or viral infection or invasion, low temperature, elevated temperature, dryness, UV light radiation, and application of specific compounds. Examples of such promoters include the rice chitinase gene promoter and tobacco PR protein gene promoter which are expressed by bacterial or viral infection or invasion; the rice "lip19" gene promoter induced by low temperature; the rice "hsp80" gene and "hsp72" gene promoter induced by high temperature; the *Arabidopsis thaliana* "rab16" gene promoter induced by dryness; the parsley chalcone synthase gene promoter induced by UV light radiation; the maize alcohol dehydrogenase gene promoter induced by anaerobic conditions; and such. Also, the rice chitinase gene promoter and tobacco PR protein gene promoter are induced by specific compounds such as salicylic acid, and "rab16" is also induced by application of the phytohormone abscisic acid.

Those skilled in the art can appropriately produce vectors carrying desired polynucleotides using general genetic engineering techniques. Usually, various commercially available vectors can be used.

Vectors of the present invention are also useful for retaining polynucleotides of the present invention in host cells, and expressing proteins of the present invention.

Polynucleotides of the present invention are generally carried by (inserted into) suitable vectors and then introduced into host cells. The vectors are not particularly limited as long as the inserted polynucleotide is stably maintained. For example, when using *E. coli* as a host, pBluescript vector (manufactured by Stratagene) and such are preferable as cloning vector, but various commercially available or known vectors can be used. Expression vectors are particularly useful when using vectors for the purpose of producing proteins of the present invention. Expression vectors are not particularly limited as long as they can express proteins in test tubes, *E. coli,* cultured cells, or individual plants. For example, such vectors are pBEST vector (manufactured by Promega) for expression in test tubes, pET vector (manufactured by Invitrogen) for *E. coli,* pME18S-FL3 vector (GenBank Accession No. AB009864) for cultured cells, and pME18S vector (Mol. Cell Biol. 8, 466-472 (1988)) for individual organisms. Insertion of a polynucleotide of the present invention into vectors can be performed by standard methods such as ligase reactions using restriction enzyme sites.

The above-mentioned host cells are not particularly limited, and various host cells can be used depending on the purpose. Cells used for expressing the proteins of the present invention include bacterial cells (for example, *Streptococcus, Staphylococcus, E. coli, Streptomyces,* and *Bacillus subtilis*), insect cells (for example, *Drosophila* S2 and *Spodoptera* SF9), animal cells (for example, CHO, COS, HeLa, C127, 3T3, BHK, HEK293, Bowes melanoma cell), and plant cells. Vectors can be introduced into host cells using known methods such as the calcium phosphate precipitation method, electroporation method (Current protocols in Molecular Biology edit. Ausubel et al. (1987) Publish. John Wiley & Sons Section 9.1-9.9), lipofection method (manufactured by GIBCO-BRL), and microinjection method.

To secrete host cell-expressed proteins into the lumen of endoplasmic reticulum, periplasmic space, or extracellular environment, suitable secretion signals can be incorporated into the proteins of interest. These signals may be endogenous or heterogenous to the proteins of interest.

When the proteins of the present invention are secreted into culture media, the media are collected. When the proteins of the present invention are produced inside cells, the cells are first lysed, and then the proteins are collected.

The proteins of the present invention can be collected and purified from recombinant cell cultures using known methods, including ammonium sulfate or ethanol precipitation, acidic extraction, anion or cation exchange chromatography, phosphocellulose chromatography, hydrophobic interaction chromatography, affinity chromatography, hydroxyapatite chromatography, and lectin chromatography.

Methods for expressing a polynucleotide of the present invention in a plant include the method of incorporating a polynucleotide of the present invention into a suitable vector and then introducing this into a living body by methods such as the electroporation method, agrobacterium method, liposome method, cationic liposome method, and such.

General genetic engineering procedures such as insertion of a polynucleotide of the present invention into a vector can be carried out according to conventional procedures (Molecular Cloning, 5.61-5.63). Administration into a plant may be performed by an *ex vivo* method or an *in vivo* method. A method for introducing a polynucleotide of the present invention into a plant is, for example, an *Agrobacterium-*mediated method for introducing a gene.

Furthermore, using a technique described later, one can produce a transformed plant into which a polynucleotide of the present invention has been introduced, and a protein of the present invention can be prepared from this plant.

Furthermore, by using recombinant proteins obtained as described above, one can prepare antibodies that bind to them. For example, one can prepare polyclonal antibodies by immunizing animals such as rabbits with purified proteins of the present invention or a partial peptide thereof, collecting blood after a certain period of time, and removing blood clots. Further, one can prepare monoclonal antibodies by fusing antibody-producing cells of animals immunized with the above-mentioned proteins or peptides with bone tumor cells, isolating single-clone cells (hybridoma) producing the antibodies of interest, and obtaining antibodies from the cells. The antibodies thus obtained can be used for purification and detection of the proteins of the present invention. The present invention includes antibodies that bind to the proteins of the present invention. By using these antibodies, it is possible to determine the location where the proteins of the present invention are expressed in plants or determine whether or not a plant species expresses a protein of the present invention.

The present invention relates to transformed plant cells into which a vector of the present invention described above has been introduced. When transformed plants having clubroot resistance activity are produced using the polynucleotides of the present invention, a polynucleotide encoding a protein of the present invention is inserted into a suitable vector, the vector is introduced into a plant cell, and an obtained transformed plant cell is regenerated.

By introducing a protein of the present invention into an arbitrary plant species and expressing it, it is possible to confer clubroot resistance activity to those plants or their seeds. The time required for this transformation is very short as compared to gene transfer by conventional crossing. It is also advantageous since it does not involve other phenotypic changes.

Cells into which a vector of the present invention is introduced include, in addition to the above-mentioned cells used for producing recombinant proteins, plant cells used for generating transformed plants.

In the present invention, the "plants" are not particularly limited, but are preferably plants which may become infected with clubroot fungus, for example, cruciferous plants. Specific examples include Chinese cabbage (Hakusai, *Brassica rapa* L. Pekinensis group), turnip, Bok choy, Nabana (*Brassica napus* L., *Brassica rapa* L. Oleifera Group), Nozawa-na (*Brassica rapa* L. Hakabura Group), Tsukena (*Brassica rapa* L. Perviridis Group), cabbage, broccoli, cauliflower, rapeseed, daikon radish (*Raphanus sativus* L. Daikon Group), and such. The "plant cells" include, in addition to cells within plants, plant cells in various forms including cultured cells (for example, suspension culture cells), protoplasts, shoot primordia, multiple shoots, hairy roots, sections of leaves, calli, and such.

Vectors can be introduced into plant cells by using various methods known to those skilled in the art, such as polyethylene glycol methods, electroporation, *Agrobacterium-*mediated methods, and particle gun methods. Plants can be regenerated from transformed plant cells using methods known to those skilled in the art according to the type of plant cell. Several techniques have already been established, such as the method of introducing genes into protoplasts using polyethylene glycol and regenerating the plant, the method of introducing genes into protoplasts using electric pulse and regenerating the plant, the method of introducing genes directly into cells by the particle gun method and regenerating the plant, and the method of introducing genes via *Agrobacterium* and regenerating the plant; and they are widely used in the technical field of the invention of this application. These methods can be suitably used in the present invention.

For efficient selection of plant cells transformed by introduction of a vector of the present invention, preferably the above-mentioned vector of the present invention contains a suitable selection marker gene or is introduced into the plant cell together with a plasmid vector containing a selection marker gene. Examples of selection marker genes used for this purpose include the hygromycin phosphotransferase gene for resistance to the antibiotic hygromycin, neomycin phosphotransferase gene for resistance to kanamycin or gentamycin, and acetyl transferase gene for resistance to the herbicide phosphinothricin.

Plant cells introduced with a recombinant vector are placed in a known selection medium containing a suitable agent for selection according to the type of the introduced selection marker gene, and the cells are cultured. This way, transformed cultured plant cells can be obtained.

Plants can be regenerated by redifferentiating transformed plant cells. The method of redifferentiation differs depending on the type of plant cells, and examples include the method of Takasaki et al. (Breeding Sci. 47: 127-134 (1997)) for Japanese mustard spinach (*Brassica rapa* Perviridis Group) which can be crossed with Chinese cabbage, the method of Fujimura et al. (Plant Tissue Culture Lett. 2: 74 (1995)) for rice, and the methods of Shillito et al. (Bio/Technology 7: 581 (1989)) and Gorden-Kamm et al. (Plant Cell 2: 603 (1990)) for maize.

Once a transformed plant that has a polynucleotide of the present invention integrated into the genome is obtained, it is possible to obtain a progeny from the plant by sexual or asexual reproduction. It is also possible to obtain propagation materials (breeding materials such as seeds, fruits, panicles, tubers, root tubers, stubs, calluses, and protoplasts) from the plant or a progeny or clone thereof, and mass-produce the plant based on such material. Thus, the present invention includes plant cells into which a polynucleotide of the present invention has been introduced, plants containing these cells, progenies and clones of these plants, as well as propagation materials and breeding materials of the plants, their progenies and clones. Plants produced in this manner or their seeds are expected to have clubroot resistance activity.

The present invention provides methods for assessing clubroot fungus resistance in a test plant or a test propagation medium. Specifically, the present invention provides a method for assessing clubroot fungus resistance in a test plant or a test propagation medium, which contains the step of detecting a DNA region containing the nucleotide sequence of SEQ ID NO: 1 or SEQ ID NO:3 or a partial sequence or surrounding sequence thereof For example, a method including steps of (i) to (iii) below may be carried out:
(i) preparing a DNA sample from a test plant or a test propagation medium;
(ii) amplifying a DNA region comprising the nucleotide sequence of SEQ ID NO: 1 or SEQ ID NO: 3, or a partial sequence or surrounding sequence thereof, from the DNA sample; and
(iii) comparing the molecular weight or the nucleotide sequence of a DNA fragment produced by amplifying the DNA region comprising the nucleotide sequence of SEQ ID NO: 1 or 3, or a partial sequence or surrounding sequence thereof, from the clubroot fungus-resistant plant or propagation medium with that of the DNA fragment amplified in step (ii).

In the present invention, "assessing clubroot resistance" includes not only assessment of clubroot resistance in cultivars cultivated so far, but also assessment of clubroot resistance in novel cultivars produced by crossing or genetic recombination techniques.

Methods for assessing clubroot resistance of plants or propagation media of the present invention comprise detecting whether or not a plant or a propagation medium retains a functional clubroot resistance gene. Whether or not a plant or a propagation medium retains a functional clubroot resistance gene can be evaluated by detecting differences in the molecular weight or nucleotide sequence of regions in the cDNA or genomic DNA region that correspond to the clubroot resistance gene.

An embodiment is a method that compares the molecular weight of a DNA region that corresponds to the clubroot resistance gene in test plants and test propagation media (for example, a DNA region comprising the nucleotide sequence of SEQ ID NO: 1 or SEQ ID NO: 3, or a partial sequence or surrounding sequence of the nucleotide sequence), with that of the same DNA region in a plant or a propagation medium having clubroot resistance.

First, a DNA sample is prepared from a test plant or a propagation medium. Next, a DNA region that corresponds to the clubroot resistance gene (for example, a DNA region comprising the nucleotide sequence of SEQ ID NO: 1 or 3, or a partial sequence or surrounding sequence of this nucleotide sequence) is amplified from the DNA sample. Furthermore, the molecular weight of the DNA fragment produced by amplifying the DNA region of the clubroot resistance gene in a clubroot-resistant cultivar is compared with that of the DNA fragment amplified from the DNA sample; and when the molecular weight is significantly lower than that of the clubroot-resistant cultivar, the clubroot resistance of the test plant or propagation medium is determined to be decreased.

Specifically, first, the DNA region of a clubroot resistance gene of the present invention is amplified using methods such as the PCR method. The term "clubroot resistance gene" in the present invention refers to a part corresponding to the cDNA region (DNA region of SEQ ID NO: 1) or genomic DNA region (the DNA region of SEQ ID NO: 3) of a clubroot resistance gene, and the amplified range may be the cDNA region or full-length genomic DNA, or a part of the genomic DNA. The above-mentioned assessment method comprises using the nucleotide sequence of SEQ ID NO: 1 or SEQ ID NO: 3, or a partial sequence or surrounding sequence of the nucleotide sequence as DNA marker.

PCR can be performed by those skilled in the art by appropriately selecting the reaction conditions and such. The amplified DNA products can be labeled using primers labeled with an isotope such as ³²P, a fluorescent dye, biotin, or such during PCR. Alternatively, amplified DNA products can be labeled by adding substrate bases labeled with an isotope such as ³²P, fluorescent dye, or biotin to PCR reaction solutions, and performing PCR. Furthermore, labeling can also be carried out by adding to the amplified DNA fragments, substrate bases labeled with an isotope such as ³²P, a fluorescent dye, or biotin using Klenow enzyme or such after PCR reaction.

The labeled DNA fragment obtained this way is denatured by heating or such, and electrophoresed in polyacrylamide gels containing a denaturant such as urea or SDS. In the present invention, SDS-PAGE which uses SDS as denaturant is an advantageous separation technique, and SDS-PAGE can be performed according to Laemmli's method (Laemmli (1970) Nature 227, 680-685). After electrophoresis, mobility of the DNA fragment is analyzed and detected by autoradiography using X-ray films, fluorescence detection scanner, and the like. Even when labeled DNAs are not used, bands can be detected by staining the electrophoresed gel with ethidium bromide or by using the silver staining method. For example, clubroot resistance can be determined by amplifying DNA fragments from the clubroot-resistant cultivars and test plants using the polynucleotides of SEQ ID NOs: 9 to 12 as primers, and comparing their molecular weights.

Moreover, the clubroot resistance in a plant or propagation medium can be assessed by directly determining the nucleotide sequence of a test-plant DNA region that corresponds to a DNA of the present invention, and comparing it to the nucleotide sequences of clubroot-resistant cultivars. Clubroot resistance can be assessed in the following way. For example, in the range corresponding to the genomic region of SEQ ID NO: 3 in a test plant or propagation medium, when the 10 bp of positions 268 to 277 are deleted, 314 bp are inserted at position 395, the 5 bp of positions 740 to 744 are deleted, 3 bp are inserted at position 1286, 21 bp are inserted at position 1552, the 241 bp of positions 1784 to 2024 are deleted, 357 bp are inserted at position 2561, the 1061 bp of positions 3370 to 4430 are deleted, 326 bp are inserted at position 6803, 4981 bp are inserted at position 7721, or 76 bp are inserted at position 7898, the test plant or propagation medium has a clubroot-susceptible phenotype (decreased clubroot resistance). Alternatively, clubroot resistance can be assessed in the following way. When the genomic region of SEQ ID NO: 3 is amplified in a test plant or propagation medium, if the nucleotide sequence of SEQ ID NO: 13 is detected, the test plant or propagation medium has a clubroot-susceptible phenotype (decreased clubroot resistance).

The above-mentioned assessment step is not limited to assessment using gels such as agarose or polyacrylamide, and assessment methods that may be used by those skilled in the art such as assessment by capillary-type electrophoresis apparatus, or assessment using single nucleotide polymorphisms (SNPs) may be used.

Furthermore, the present invention also provides methods for selecting plants or seeds thereof having the clubroot resistance gene by an above-mentioned assessment method of the present invention.

As described above, a method for producing transformed plants, which comprises the step of introducing polynucleotides or vectors of the present invention to plant cells, and regenerating a plant from the plant cells is also included in the present invention. Specifically, the present invention relates to methods for producing plants or seeds thereof having clubroot resistance activity, which comprise the following steps of:
(i) introducing a vector of the present invention into plant cells; and
(ii) regenerating a plant from the transformed cells introduced with a vector in step (i) mentioned above.

As described above, the method of conferring clubroot resistance activity to plants or seeds thereof, which comprise the step of expressing a polynucleotide of the present invention in the cells of a plant, is also included in the present invention. For example, this method can be carried out by introducing a polynucleotide of the present invention or a vector of the present invention into plant cells. An example is a method comprising the following steps of:
(a) crossing a plant having a polynucleotide of the present invention with another plant; and
(b) selecting a plant having the aforementioned polynucleotide.

Plants or seeds thereof having clubroot resistance are produced by these methods. More specifically, by the methods of the present invention, for example, a plant sensitive to clubroot can be converted to a clubroot-resistant plant.

Plants that can be conferred resistance by methods of the present invention are not particularly limited, and resistance can be conferred to any plant. Examples include cruciferous plants. Specific examples include Chinese cabbage, turnip, Bok-choy, Nabana, Nozawa-na, Tsukena, cabbage, broccoli, cauliflower, rapeseed, daikon radish, and such, but are not limited thereto.

Furthermore, the present invention provides plants and seeds thereof produced by the above-mentioned methods of the present invention. For example, artificially produced plants or seeds thereof that carry polynucleotides of the present invention and have clubroot resistance activity are also included in the present invention.

Furthermore, the present invention provides oligonucleotides that have a chain length of at least 15 nucleotides and are complementary to the nucleotide sequence of SEQ ID NO: 1 or SEQ ID NO: 3 or their complementary sequences.

Herein, the term "complementary sequence" refers to the sequence of the other strand relative to the sequence of one strand in a double-stranded DNA consisting ofA:T and G:C base pairs. Furthermore, the term "complementary" is not limited to the case of a completely complementary sequence in a region of at least 15 consecutive nucleotides, and may have at least 70%, preferably at least 80%, more preferably 90%, and even more preferably 95% or higher nucleotide sequence identity. Such DNAs are useful as probes for detecting the polynucleotides of the present invention or selecting plants (plant cells) having the polynucleotides of the present invention, or as primers for amplifying the polynucleotides of the present invention.

Specifically, the present invention provides primers and probes for detecting clubroot resistance activity in a test plant, which comprise an oligonucleotide having a chain length of at least 15 nucleotides and specifically hybridizes under stringent conditions with the nucleotide sequence of SEQ ID NO: 1.

Furthermore, the present invention provides reagents containing an oligonucleotide to be used in the methods for producing the plants of the present invention or seeds thereof More specifically, reagents of the present invention contain the following oligonucleotides:
(a) oligonucleotide primers for amplifying the full-length sequences or partial sequences of the nucleotide sequences of SEQ ID NOs: 1 and 3; and
(b) oligonucleotide probes which have a chain length of at least 15 nucleotides and hybridize under stringent conditions with the nucleotide sequences of SEQ ID NOs: 1 and 3.

Primers or probes of the present invention can be synthesized by any method based on the nucleotide sequences constituting them. The length of the nucleotide sequence complementary to the genomic DNA of the primers or probes of the present invention is usually 15 to 100, generally 15 to 50, and preferably 15 to 30. Methods for synthesizing an oligonucleotide having the nucleotide sequence based on an obtained nucleotide sequence are well known. Furthermore, in oligonucleotide synthesis, an arbitrary modification can be introduced to an oligonucleotide using nucleotide derivatives modified with fluorescent dye, biotin and the like. Alternatively, methods for binding fluorescent dye or the like to synthetic oligonucleotides are also known.

Furthermore, the present invention provides a kit to be used in the various methods of the present invention. In preferred embodiments of the kits of the present invention, at least one type of oligonucleotide in (a) or (b) described above is included. Kits of the present invention may appropriately include in their package, positive or negative standard samples, instructions describing the method of use, and such.

Polynucleotides or vectors of the present invention can be used, for example, in the production of plants or seeds having clubroot fungus resistance activity. Plants or seeds thereof having clubroot fungus resistance activity can be produced by expressing in desired plants or seeds thereof polynucleotides or vectors of the present invention.

Therefore, the present invention relates to agents for conferring clubroot fungus resistance activity, which comprise a polynucleotide or vector of the present invention as the active ingredient. The term "agent for conferring clubroot fungus resistance activity" in the present invention refers to a pharmaceutical agent having the effect of conferring clubroot fungus resistance activity to all or a part of a plant or its seed, and refers to a substance or a composition (mixture) comprising a polynucleotide or a vector of the present invention as the active ingredient.

In the pharmaceutical agents of the present invention, in addition to polynucleotides or vectors which are the active ingredients, for example, sterilized water, physiological saline solution, plant oil, surfactants, lipids, solubilizing agents, buffers, preservatives, and such may be mixed in as necessary.

All prior art references cited in this specification are incorporated herein by reference.

### Examples

Hereinafter, the present invention is specifically described with reference to the Examples; however, the present invention should not be construed as being limited thereto. Reference examples have been disclosed (Suwabe, K., Tomita, N., Fukuoka, H., Suzuki, T., Mukai, Y., and Matsumoto, S., "Genetic refinement of the Chinese cabbage clubroot resistance gene locus Crr1 and synteny analysis with Arabidopsis thaliana using a physical map", Breeding Research, supplement 1·2, (2005) 154).

### Reference Example 1. Isolation of a BAC library carrying Crr1

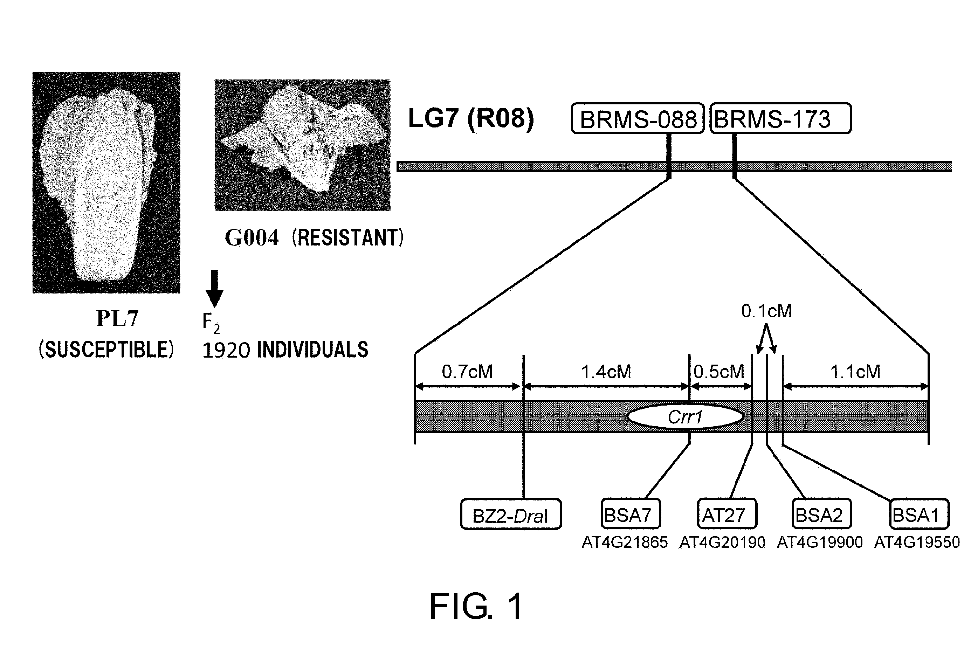

To isolate clubroot resistance genes by map-based cloning, a BAC library of resistant line G004 derived from the European fodder turnip "Siloga" was constructed. This library had an average insert length of 67.4 kb and a size of approximately 38,400 clones, and it was equivalent to 4.7-times the genome of *B. rapa* which is estimated to be 550 MB (Arumuganathan K, Earle ED., Plant Mol Biol Rep, (1991) 9 (3): 208-219). To isolate BAC clones carrying Crr1, chromosome walking was performed starting from BSA7, which is the marker most closely linked to Crr1 among the linked markers (Fig. 1, Suwabe, K. et al., Genetics, (2006) 173: 309-319).

After 96 arbitrary E. *coli* cells were selected and cultured overnight in a liquid medium, and replicas of E. *coli* were taken. These 96 E. *coli* were combined into one, and their plasmid DNA was extracted. 384 of such a plasmid DNA pool which combines 96 E. *coli* into one were produced. The first screening examined whether the BSA7 fragments contained in the 384 samples were amplified. For the pools in which amplification was confirmed by PCR, replicas of the initial 96 E. *coli* were cultured, and E. *coli* (clones) carrying the BSA7 sequence were identified by secondary screening. Plasmid of each clone was extracted, their terminal sequences were determined, and then PCR primers were designed using those terminal sequences as targets. Whether each terminal sequence was amplified was examined using the designed primers in all BAC clones possibly containing BSA7, and the manner in which each of the BAC clones overlapped was elucidated. From the BAC clones obtained this way, the BAC clone positioned at the very end was identified.

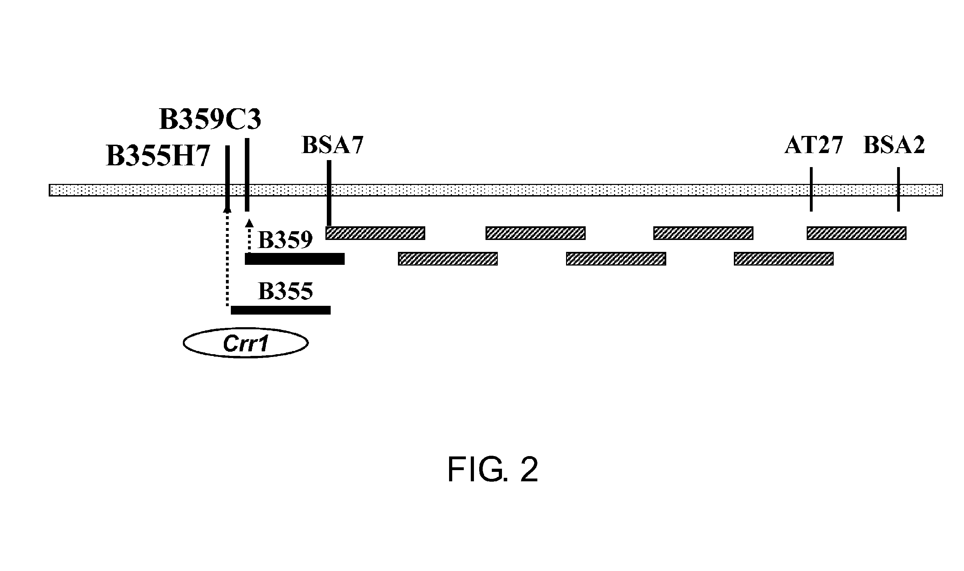

Primary screening was performed again using both terminal sequences of the identified BAC clone, and a number of BAC clones that overlapped starting from BSA7 were identified (Fig. 2). Furthermore, screening was repeated using the terminal sequences of the obtained clones, and ultimately BSA7 and BSA2 were covered. This region could be covered by a minimum of seven clones. Meanwhile, on the opposite side, B355H7 carrying the BSA7 marker sequence and a B359C3 clone that overlapped with it were obtained.

### Reference Example 2. Narrowing down the location of Crr1 using an individual in which the markers near Crr1 are recombined

Starting from BSA7, the region around Crr1 was covered with BAC clones, and by comparing the terminal sequences of these BACs between the susceptible cultivars PL7 and G004, multiple markers indicating polymorphism were obtained. From comparison of the marker genotype obtained from the terminal sequences of the BAC clones and the presence of the resistance gene, the markers produced from the terminal sequences of B355H7 and B359C3 were the markers cloest to Crr1.

The marker genotypes of the G004 type and PL7 type were denoted by RR and rr, respectively, and the heterozygous type was denoted by Rr. To narrow down the Crr1 location individuals with recombination between markers near Crr1 were screened from PL7, G004, and F₂ individuals. From approximately 5,700 F₂ individuals, those in which genomic recombination of the susceptible type and resistant type had taken place between BRMS-088 and BRMS-173 were selected. That is, three individuals (No. 1075, No. 764, and No. 572) in which recombination took place between markers of B355H7 and B359C3 were investigated in detail. The marker genotypes of each individual are as shown below.

| | B355H7 | B359C3 |
|---|---|---|
| F₂ No. 1075 | rr | Rr |
| F₂ No. 764 | Rr | rr |
| F₂ No. 572 | RR | Rr |

These three F₂ plants were self-reproduced to obtain F₃ seeds. The heterotype loci in the F₂ generation were separated into three types: RR, Rr, and rr in the F₃ generation. Clubroot resistance of the F₃ generation was assayed using the F₃ seeds and the clubroot fungus isolate "Ano-01". Specifically, it was investigated whether plant individuals whose B355H7-B359C3 marker genotype is the rr-RR type in No. 1075, and the RR-rr-type in No. 764 and No.572 are resistant (Fig. 3). Clubroot resistance assay was performed as described below according to the method of Yoshikawa *et. al* (Yoshikawa, H., Bull. Natl. Res. Inst. Veg., Ornam. Plants & Tea Japan, (1993) 7:1-465).

Horticultural culture soil was placed up to half the height from the bottom of a 9-cm-diameter jiffy pot, and a groove having a width of about 2 cm was made; and 10 g of infected soil, which was prepared so that 5 x 10⁶ resting spores of the clubroot fungus isolate "Ano-01" were present per 1 g of dry soil, was placed into the groove. Next, ten F₃ seeds were sown in each jiffy pot; and the plants were cultivated for six weeks in a phytotron with settings of approximately 20,000 lux illuminance, 16-hours day length, 23°C temperature during the light period, and 18°C temperature during the dark period. Then, the roots were washed. To evaluate the degree of damage of the roots, four levels of disease index were implemented in (0: knobs are not found at all; 1: tiny knobs are attached to the lateral roots; 2: serial knobs are attached to the lateral roots, or the symptoms are intermediate between damage levels 1 and 3; and 3: relatively large knobs are attached to the main root, or the main root is enlarged). Of the four stages, 0, 1, and 2 were judged to be "resistant" as there is certain resistance against clubroot; and 3 was judged to be "susceptible" with no resistance. Results of the resistance assay showed that none of the F₃ individuals of the three lineages had resistance, and their phenotype was determined to be the susceptible type.

Furthermore, DNA was extracted from each individual used in the resistance assay, the genotype of the nearby markers was determined, and the location of Crr1 was narrowed down based on the relationship between the marker genotypes and the presence of the resistance genes. In the F₃ individuals of No. 1075, individuals that have the RR-type B359C3 showed susceptibility. This strongly suggests that Crr1 does not exist towards the side of AT27 beyond B359C3, and the results with No. 764 and No. 572 strongly suggest that Crr1 does not exist towards the BZ2-DraI side beyond B355H7. Therefore, the resistance gene, Crr1, was estimated to be present within the approximately 8 kb region between B355H7 and B359C3 (Fig. 3).

### [Example 1] Estimation of the Crr1 candidate gene

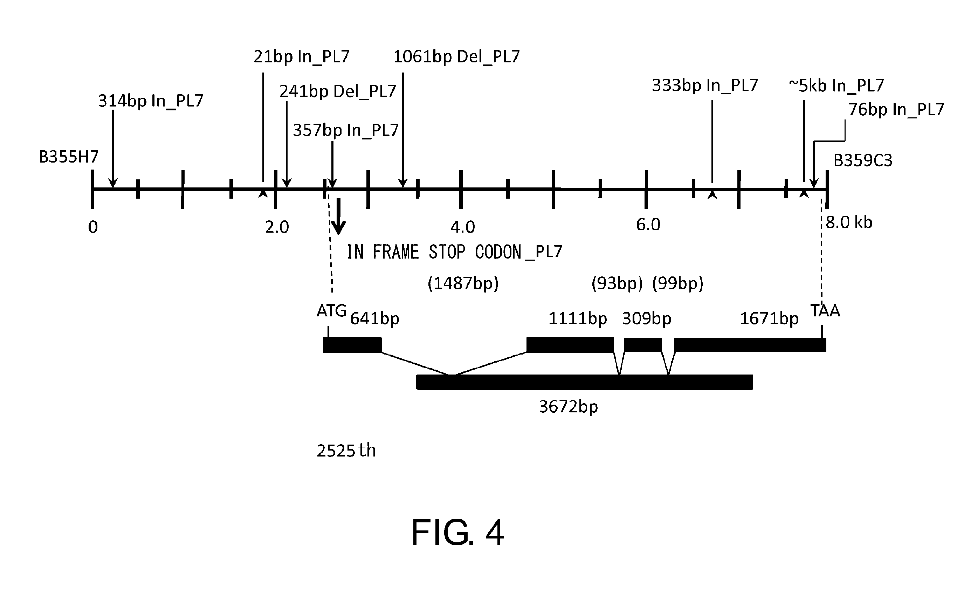

Shotgun clones of B355H7 were produced to determine the DNA sequence of the approximately 8-kb region between B355H7 and B359C3. The DNA sequences of the extracted plasmid fragments were determined according to a standard method using T7 and Reverse Primer, and a DNA Sequence Assembly Software, SEQUENCHER ver. 2 (Hitachi Software Engineering, Tokyo), was used to produce a single sequence. In this region, search of open reading frames (ORF) that encode proteins was done using a genetic information processing software, GENETYX (Genetyx, Tokyo). Based on the determined G004 sequence information, primers were designed at suitable positions, DNA fragments were amplified using the susceptible PL7 as a template, and the nucleotide sequence of this region in PL7 was determined. The DNA sequences were compared between PL7 and G004, and the inserted and deleted sequences as well as the presence of single nucleotide polymorphisms were investigated. As a result, the length of the DNA fragment between B355H7 and B359C3 in the resistant line G004 was 7,995 bp, and four ORFs were presumed to be present in this sequence (Fig. 4, SEQ ID NO: 3).

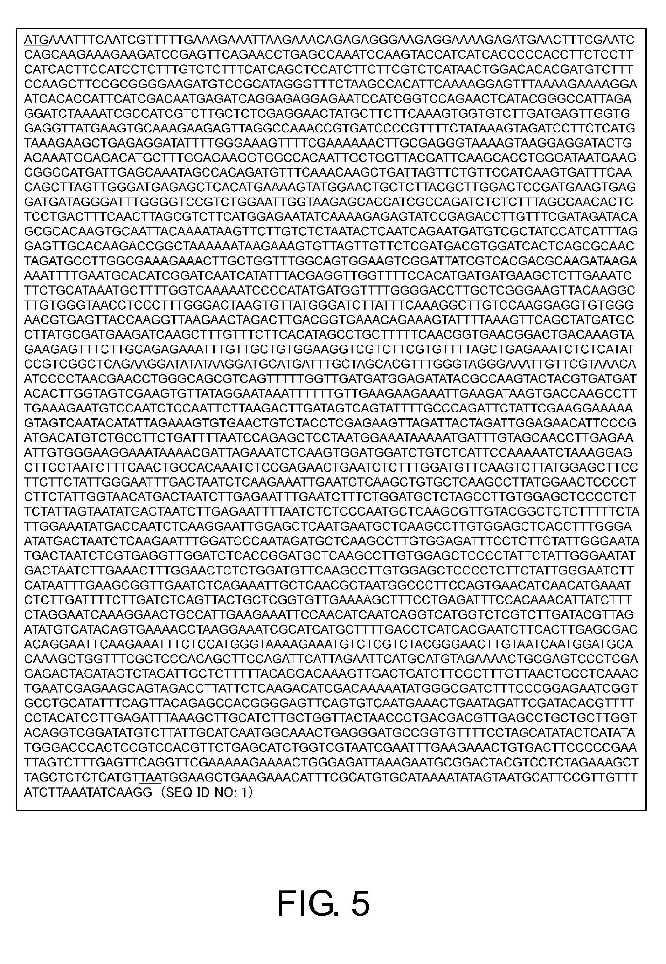

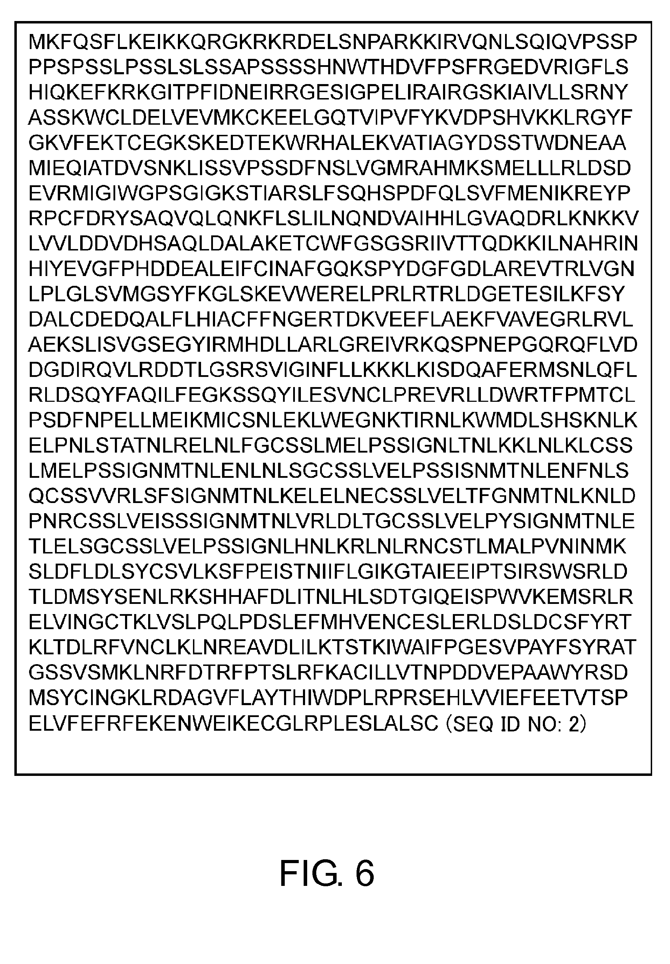

To investigate whether the estimated ORF regions are transcribed, a single-stranded cDNA was synthesized from poly(A)+ RNA extracted from the roots of resistant material An4-8-1 containing Crr1, then the primers, Crr1-Fsm (5'-TCC [CCCGGG] AAAATGAAATTTCAATCGTTTTTG-3' / SEQ ID NO:9; [ ] indicates the SmaI site) and Crr1-R (5'-CCTTGATATTTAAGATAAACAACGGAATG-3' / SEQ ID NO: 10) were used to amplify the cDNA region of the Crr1 gene. The cDNA nucleotide sequence was determined, and the amino acid translation region was estimated. As a result, a DNA fragment having nearly the same length as the fragment containing the four ORFs was found. The part starting from the ATG start codon first found in the nucleotide sequence of this cDNA to immediately before the TAA stop codon had 3672 bp, and was estimated to encode 1224 amino acids (Fig. 5, Fig. 6).

The genomic DNAs were compared to estimate the intron and exon portions. As a result, a structure in which four ORFs are linked into one by splicing was found. The splicing regions which separate the introns from the exons all followed the GT-AG rule.

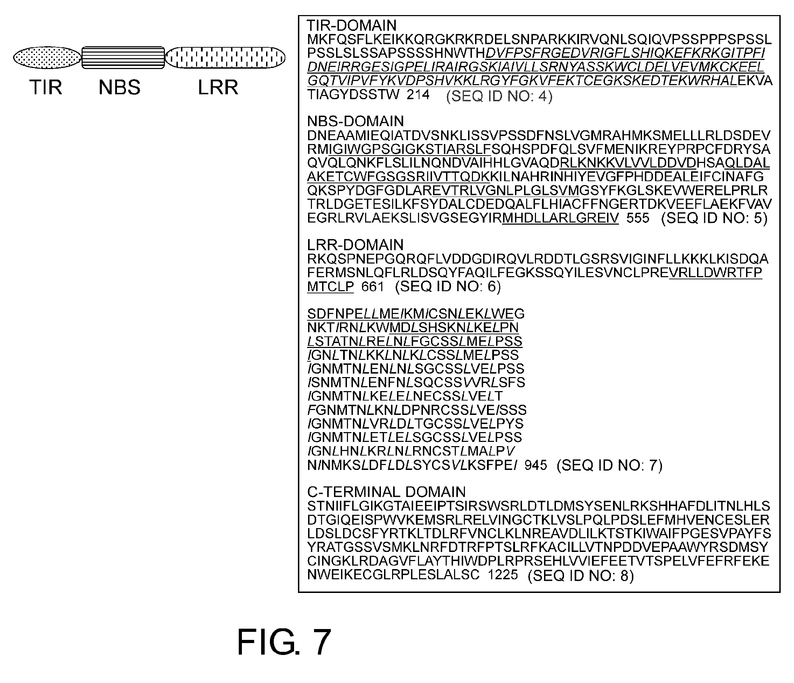

Furthermore, when the ends (the translation initiation and termination sites) of the transcription products were determined from the genomic sequences of resistant cultivars by performing 5' race and 3' race analyses, sequences encoding amino acids other than the four ORFs were not found, and this suggests that the four ORFs are transcribed as a single cDNA (SEQ ID NO: 3). The estimated amino acid sequence demonstrated the NIR-NBS-LRR structure which is a sequence commonly shared by disease resistant genes (Fig. 7).

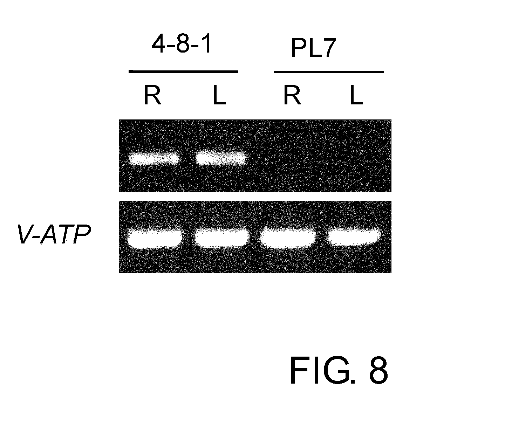

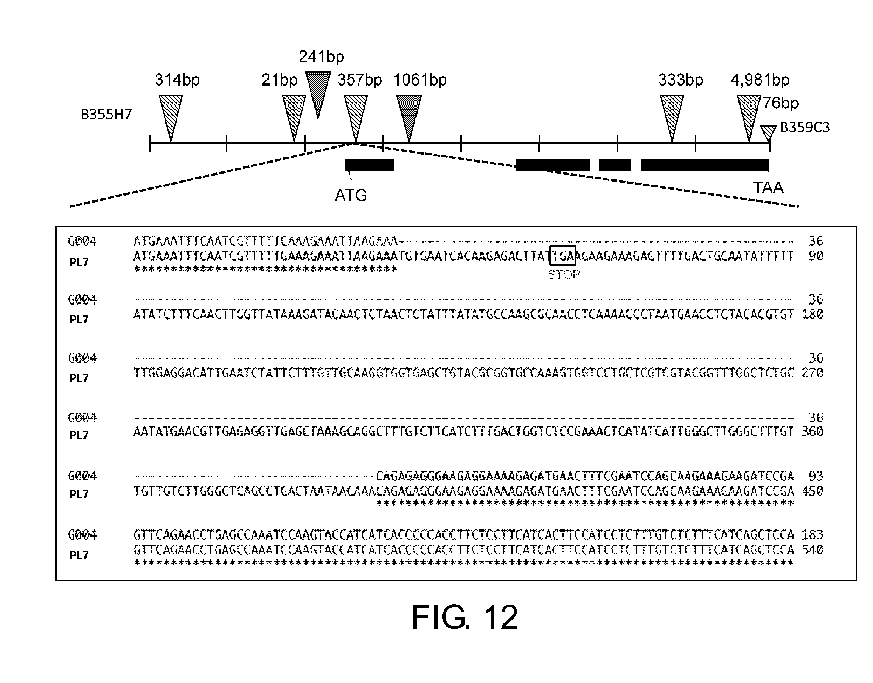

In comparison with this region in the susceptible cultivar PL7 (SEQ ID NO: 13), many insertion and deletion sequences and single nucleotide polymorphisms were found. Insertions and deletions of 100 bp or more were found in seven places. In particular, comparison of the 5' side showed that insertion of 357 bp approximately 60 bp downstream of the start codon in PL7 placed an in-frame stop codon in exon 1 (Fig. 12), and on the 3' side, a long insertion sequence of approximately 5 kb was present in PL7. Furthermore, the terminal sequence of B359C3 had a 78-bp insertion. Between them, 200 bp or longer exons were found in four places (SEQ ID NO: 3). The four estimated ORFs were defined with the region having a sequence of 641 bp as exon 1, and exons 1, 2, 3, and 4 in order. To investigate how the expression of the ORF regions differs between the resistant and susceptible cultivars, RT-PCR was performed by targeting the first exon regions in the resistant line An4-8-1 and the susceptible cultivar PL7. A primer set was designed using the sequences in exon 1. Using the cDNAs derived from the roots and leaves of the resistant line An4-8-1 and the susceptible PL7 as templates, amplification was carried out using the designed primers. V-ATP which is expressed constitutively in the cells was used as a positive control. As a result, amplification of DNAs at the intended chain length was confirmed in both of the roots and leaves in An4-8-1. However, in PL7, such amplification could not be confirmed in either the roots or the leaves. Therefore, the clubroot-resistance candidate gene was found to be expressed in the resistant line, but not expressed in the susceptible cultivar (Fig. 8).

### [Example 2] Proof of Crr1 by complementarity experiments

### (1) Construction of vectors for introduction into plant cells

### - Construction of a vector using the lettuce ubiquitin gene as the promoter

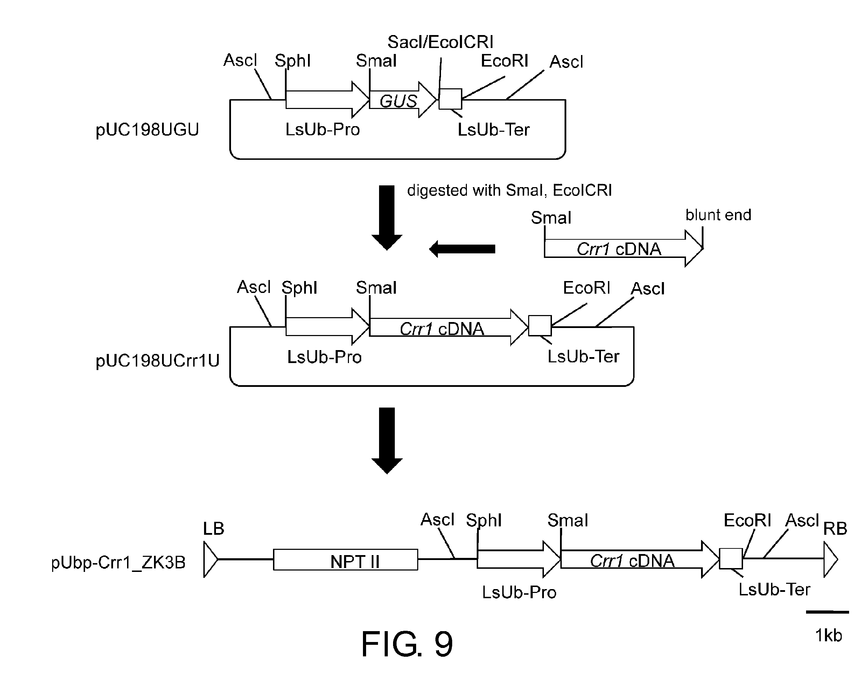

A plasmid pUC198UGU (received from Dr. H. Fukuoka) inserted with a GUS gene between the promoter and terminator of the ubiquitin gene cloned from lettuce was cleaved with SmaI and EcoICRI, and a Crr1 cDNA sequence treated in advance with SmaI was inserted to construct pUC198UCrr1U (Fig. 9). pUbp-Crr1_ZK3B was constructed by excising the lettuce ubiquitin promoter - Crr1 cDNA - lettuce ubiquitin terminator cassette using AscI, and inserting it into a binary vector pZK3B (received from Dr. M. Kuroda at the National Agriculture and Food Research Organization (NARO) / Agricultural Research Center (ARC)) which has been modified from pPZP202 (Hajdukiewicz P. et al., Plant Mol Biol, (1994) 25: 989-994) (Fig. 9).

The constructed binary vector was transformed into the agrobacterium GV3101 strain, and *Arabidopsis thaliana* Col-0 was transformed by the Flower-dip method. The seeds (T1) of *Arabidopsis thaliana* harvested from the agrobacterium-inoculated line (T0) carrying the constructed vector were seeded into a selection medium containing kanamycin, resistant individuals were selected, and a number of viable lines were obtained. Plants that can be cultivated on a selection medium were potted and self-propagated to obtain next-generation seeds (T2). These T2 seeds were tested under clubroot resistance assay.

### (2) Clubroot resistance assay using transformed Arabidopsis thaliana

Clubroot assay of *Arabidopsis thaliana* was performed by modifying the method of Jubault M. et al., Theor. Appl. Genet.,(2008) 117:191-202. Kanamycin-resistant plants were transferred to a horticultural culture soil (TM-1, TAKII) at nine individuals per pot, and three days later, 2 mL of a dormant spore solution (1.0 x 10⁶ spores/mL) was used to drench the base of the plant. The plants were cultivated under conditions of 22°C and 14-hour day length; and three weeks after inoculation, the degree of symptoms at the root was investigated. The degree of disease development was evaluated at four levels: 0 (no symptoms); 1 (small knobs in the lateral roots); 2 (slightly large knobs in the lateral roots); and 3 (large knobs in the main root and enlargement of the hypocotyl).

As a result, when the untransformed columbia lines were subjected to clubroot fungus isolate Ano-01, out of the total 18 lines except for one, 17 lines showed marked root enlargement or thickening and were judged to be at level 3 in the disease index (Table 1: degrees of clubroot resistance in transformed *Arabidopsis thaliana* introduced with the Crr1 candidate gene).

| | NUMBER OF EMERGED LINES ACCORDING TO THE DISEASE INDEX ^{1),2)} | | | | RATIO OF DISEASED LINES (%) ⁴⁾ | AVERAGE DISEASE INDEX ⁵⁾ | NUMBER OF EMERGED LINES ACCORDING TO THE DISEASE INDEX ^{1),3)} | | | | RATIO OF DISEASED LINES (%) ⁴⁾ | AVERAGE DISEASE INDEX ⁵⁾ |
|---|---|---|---|---|---|---|---|---|---|---|---|---|
| NAME OF LINE | 0 | 1 | 2 | 3 | | | 0 | 1 | 2 | 3 | | |
| Col-0 (non-transformant) | 0 | 0 | 0 | 9 | 100.0 | 3.0 | 0 | 0 | 1 | 8 | 100.0 | 2.9 |
| UpCrr1_02 | 9 | 0 | 0 | 0 | 0.0 | 0.0 | 9 | 0 | 0 | 0 | 0.0 | 0.0 |
| UpCrr1_04 | 9 | 0 | 0 | 0 | 0.0 | 0.0 | 8 | 1 | 0 | 0 | 11.1 | 0.1 |
| UpCrr1_09 | 2 | 4 | 1 | 0 | 71.4 | 0.9 | 5 | 2 | 0 | 2 | 44.4 | 0.9 |
| UpCrr1_11 | 4 | 5 | 0 | 0 | 55.6 | 0.6 | 4 | 1 | 1 | 3 | 55.6 | 1.3 |
| UpCrr1_15 | 6 | 3 | 0 | 0 | 33.3 | 0.3 | 7 | 0 | 0 | 2 | 22.2 | 0.7 |
| UpCrr1_17 | 9 | 0 | 0 | 0 | 0.0 | 0.0 | 9 | 0 | 0 | 0 | 0.0 | 0.0 |

| | | | | | | | | | | | | |
|---|---|---|---|---|---|---|---|---|---|---|---|---|
| 1) 0 (no symptoms); 1 (small knobs in the lateral roots); 2 (slightly large knobs in the lateral roots); 3 (large knobs in the main root and enlargement of hypocotyl) 2) Date of inoculation: 2010.2.4; Date of examination: 2010.2.25-26 3) Date of inoculation: 2010.3.19; Date of examination: 2010.4.12 4) (total number of lines showing disease index 1, 2, or 3 / total number of individuals) x 100 5) (number of lines with disease index 0 x 0 + number of lines with disease index 1 x 1 + number of lines with disease index 2 x 2 + number of lines with disease index 3 x 3) / total number of individuals | | | | | | | | | | | | |

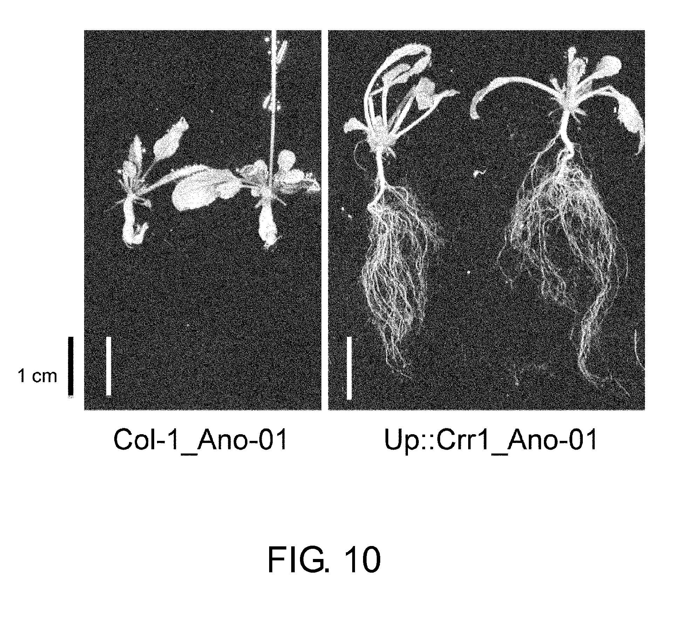

In these lines, a pigment presumed to be anthocyanin accumulated in the leaves, changing the color of the leaves to a pale red color, and the growth on the ground was markedly inhibited (Fig. 10). In two independent experiments, three lines of transformants, which are UpCrr1_02, UpCrr1_04, and UpCrr1_17, produced large amounts of white healthy roots, the symptoms of clubroot were not observed at all, and the number of diseased lines as well as the disease index were both 0. Furthermore, the above-ground part also showed growth equivalent to the normally cultivated line, and it was evident that resistance against clubroot was clearly acquired. In the section that was inoculated on March 19, 2010, although several lines judged to have disease index 3 were observed in some of UpCrr1_09, UpCrr1_11, and UpCrr1_15, but there were more individuals with no disease development at all. When the candidate gene was used to transform the clubroot-susceptible *Arabidopsis thaliana* var. *Columbia,* clubroot-resistant *Arabidopsis thaliana* was obtained. From the above, the cloned gene was revealed to have a function of conferring clubroot resistance and was determined to be Crr1.

### [Example 3] Effect of selection when insertion/deletion sequences found between the clubroot-resistant and -susceptible lines were used as selection markers

In marker selection using BRMS-173 and BRMS-088, recombination between the marker locus and the Crr1 loc took place in some individuals, albeit at a low probability. To avoid this, one can imagine using the resistance gene itself as marker. As shown in SEQ ID NO: 3, when the resistant and susceptible lines were compared for their Crr1 gene sequences, there were many insertion/deletion sequences. These can be used to examine their utility as markers for selecting resistant individuals.

Between the resistant line G004 and the susceptible cultivar PL7, an 86-bp insertion/deletion sequence is present near the C terminus of Crr1. This insertion/deletion sequence was used as a target, and the primer set B359C3 (forward primer: CTCTCTCATGTTAATGGAAGCTGA / SEQ ID NO: 11; and reverse primer: CACTCAACGAGTAGGAAACAAAGA / SEQ ID NO: 12) was constructed. The test material was an F₂ population derived by crossing the susceptible A line with "Hakusai Parental Line No.9" (hereinafter, abbreviated as PL9) having two resistance genes, Crr1 and Crr2.

For the resistance assay, clubroot fungus isolates "Wakayama-01" and "No. 5" were inoculated. Since the "Wakayama-01" and "No. 5" isolates damage a considerably large number of Chinese cabbage cultivars, they are fungi with a wide host range. To confer resistance against "Wakayama-01" and "No. 5", both clubroot resistance genes, Crr1 and Crr2, must be carried in homozygous forms (Suwabe, K. et al., Theor. Appl. Genet., (2003) 107: 997-1002).

Resistance assay was performed according to the method of Yoshikawa (1993) (Yoshikawa, H., Bull. Natl. Res. Inst. Veg., Ornam. Plants & Tea Japan, (1993) 7:1-465). The F₂ crossed seeds were seeded into diseased soils separately containing the "Wakayama-01" and "No. 5" fungi; and six weeks later, the disease index was determined based on the resistance indicator.

Furthermore, DNAs were extracted from all of the tested individuals, PCR was performed using the primers of B359C3 and BRMS-096, amplified fragments were fractionated by agarose gel electrophoresis, and three marker genotypes, i.e., resistant homozygous-type, heterozygous-type, and susceptible homozygous-type, were detected. Since Crr1 and Crr2 are located on separate linkage groups, the marker genotypes of B359C3 and BRMS-096 are independent. Therefore, these two marker genotypes were combined to produce six types of marker genotypes. The relationship between these six marker genotypes and their disease indices was carefully examined, and the utility of B359C3 as a marker was examined.

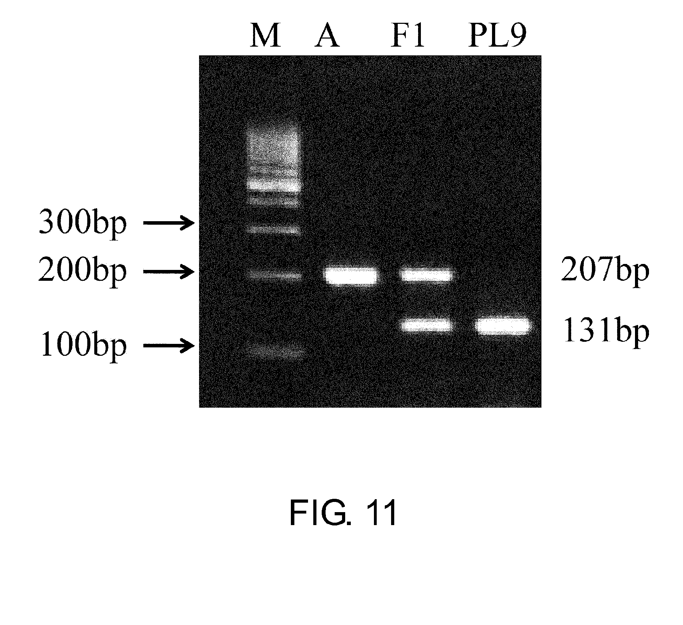

As a result, 131 bp were amplified in the resistant line PL9, whereas in the susceptible cultivar A, 207 bp containing a 68-bp insertion sequence was amplified. Furthermore, regarding BRMS-096 linked to Crr2, 220 bp were amplified in PL9 and 200 bp were amplified in the A line, and these differences were also clearly distinguishable by 2% agarose gel electrophoresis (Fig. 11). Data were obtained from 115 individuals inoculated with the Wakayama-01 isolate and 92 individuals inoculated with the No. 5 isolate. In the experiment using the Wakayama-01, among the 115 individuals, the number of individuals appearing to have resistance with a disease index of 0, 1, or 2 were 4, 7, and 6 individuals, respectively, which accounts for approximately ten percent of the whole. On the other hand, 98 individuals were susceptible to the disease, and this exceeded 90 percent. Apart from the disease index, when the number of emergences was examined by the marker genotype, of ten individuals that had the resistant homozygous type (RR,RR) as both of the two marker genotypes, 4, 3, and 3 had a disease index of 0, 1, or 2, respectively, and they were all resistant individuals. The average disease index was 0.9. The disease indices of the other five marker genotypes were mostly 3. Similar results were obtained in tests using the "No.5" isolate.

Resistance against "Wakayama-01" and "No. 5" appeared only in individuals having both Crr1 and Crr2 in homozygous forms. B359C3 is a marker which has a 69-bp insertion/deletion sequence between the PL9 and A lines, and the PCR-amplified DNA fragments can be easily distinguished between the resistant line (PL9), the heterozygous individuals, and the susceptible line (PL7) by agarose gel electrophoresis. Furthermore, since BRMS-096 used to detect Crr2 mostly cosegregates with Crr2, individuals carrying the two markers in the resistant homozygous forms are predicted to show resistance. In fact, in experiments using "Wakayama-01" and "No. 5", while more than 80% of the tested F₂ individuals were susceptible, individuals that have both of the two markers in the resistant homozygous type did not show susceptibility. Therefore, B359C3 was revealed to be a highly precise marker for selection of Crr1.

To date, there have been reports regarding detection of QTL related to clubroot resistance and markers linked to resistance genes, but when used as selection markers, there are two problems. The first one is that the distance between marker and the gene is close for use as selection marker. When the distance between the marker and the gene is 1 cM, breeders using the marker must be prepared to see recombination taking place in 1% or so in 100 individuals. BRMS-088 and BRMS-173, which are SSR markers linked to Crr1, were useful as selection markers, but since both of the markers are approximately 2 cM away from Crr1, even though the probability was low, emergence ofrecombinant individuals was a problem. Since Crr1 is located between BRMS-088 and BRMS-173, recombinant individuals could be excluded by using both markers. However, developing two markers that flank a gene of interest in this manner is not easy, except for crops whose entire nucleotide sequence has been disclosed such as rice. B359C3 allows amplification of the intron sequence of Crr1 by PCR. Therefore, one hardly has to worry about the occurrence of recombinant individuals. The results are summarized in Table 2 (number of emerged individuals at each disease index in the inoculation test using the clubroot fungus isolate "Wakayama-01") and Table 3 (number of emerged individuals at each disease index in the inoculation test using the clubroot fungus isolate "No. 5"), showing the relationship of the genotypes of the novel Crr1-linked marker B359C3 and the Crr2-linked marker BRMS-096 with the degree of resistance. Two experimental results support the findings.

**Table 2**

| B359C3 *¹ | BRMS-096 *¹ | DISEASE INDEX *² | | | | | AVERAGE DISEASE INDEX *³ |
|---|---|---|---|---|---|---|---|
| | | 0 | 1 | 2 | 3 | SUBTOTAL | |
| *rr* | *rr* | 0 | 0 | 0 | 5 | 5 | 3.0 |
| *rr* | *Rr* | 0 | 0 | 0 | 9 | 9 | 3.0 |
| *rr* | *RR* | 0 | 0 | 1 | 3 | 4 | 2.8 |
| *Rr* | *rr* | 0 | 0 | 0 | 14 | 14 | 3.0 |
| *Rr* | *Rr* | 0 | 2 | 0 | 33 | 35 | 2.9 |
| *Rr* | *RR* | 0 | 2 | 0 | 11 | 13 | 2.7 |
| *RR* | *rr* | 0 | 0 | 0 | 12 | 12 | 3.0 |
| *RR* | *Rr* | 0 | 0 | 2 | 11 | 13 | 2.8 |
| *RR* | *RR* | 4 | 3 | 3 | 0 | 10 | 0.9 |

**Table 3**

| B359C3 *¹ | BRMS-096 *¹ | DISEASE INDEX *² | | | | | AVERAGE DISEASE INDEX *³ |
|---|---|---|---|---|---|---|---|
| | | 0 | 1 | 2 | 3 | SUBTOTAL | |
| *rr* | *rr* | 0 | 0 | 0 | 5 | 5 | 3.0 |
| *rr* | *Rr* | 0 | 0 | 0 | 12 | 12 | 3.0 |
| *rr* | *RR* | 0 | 0 | 0 | 3 | 3 | 3.0 |
| *Rr* | *rr* | 0 | 0 | 0 | 12 | 12 | 3.0 |
| *Rr* | *Rr* | 0 | 1 | 1 | 21 | 23 | 2.9 |
| *Rr* | *RR* | 0 | 3 | 2 | 1 | 6 | 1.7 |
| *RR* | *rr* | 0 | 0 | 0 | 8 | 8 | 3.0 |
| *RR* | *Rr* | 1 | 0 | 2 | 12 | 15 | 2.7 |
| *RR* | *RR* | 3 | 4 | 1 | 0 | 8 | 0.8 |

The signs in Tables 2 and 3 above are shown below.
*1: Crr1 and Crr2 are located on separate chromosomes, and B359C3 and BRMS-096 are DNA markers that are linked to the Crr1 gene and the Crr2 gene, respectively. rr: susceptible parent, Rr: heterozygous type, RR: resistant parent type
*2: disease indices; 0: no symptoms; 1: small knobs in the lateral roots; 2: serial knobs in the lateral roots; and 3: knobs in the main root
*3: average disease index: ∑(disease index * number of individuals)/(total number of individuals)

Furthermore, it has been reported that there are genes around the gene of interest that are undesirable in terms of breeding, and their link could not be broken which results in linkage drag (Fukuoka S, et al., Science, (2009) 325(5943): 998-1001). When there is a linkage drag, the information of the region around the gene is necessary. At present, whether there is a gene that causes a linkage drag to Crr1 is unclear; however, if the sequence information of Crr1 is available, a linkage drag can be removed when present.

The second problem is that while the distance to the resistance gene is close, since the marker genotype of the individual carrying the gene of interest is the same as that of the individual to be introduced with the gene, it cannot be used as a marker. More specifically, depending on the susceptible cultivars and lineages, some may not carry Crr1 but may have marker genotypes completely identical to those of BRMS-088 and BRMS-173, and these cannot be used as selection markers. The gene region encoding Crr1 has been elucidated, and comparison with susceptible lines has become possible. Comparison of this region in resistant PL6 and susceptible PL7 showed many insertions/deletions and single nucleotide polymorphisms, and construction of markers has become efficient and easy. Even for cultivars and lines in which markers could not be used so far because they had the same marker genotype, it has become possible to construct markers easily by using the Crr1 region sequence as a reference. As described above, by using the Crr1 sequence, a marker producing polymorphism could be easily constructed regardless of the type of cultivar or lineage, and the obtained marker has a highly precise selection ability and practically does not produce any recombinant individuals.

### Industrial Applicability

By the present invention, a clubroot resistance gene has been isolated, and it becomes possible to efficiently produce cruciferous plants with clubroot resistance by genetic recombination using the gene and marker selection. In particular, when performing marker selection, recombination between marker and gene does not occur when Crr1 or its surrounding sequences is used as marker; therefore, it has become possible to obtain a technique with very high selection effect. Furthermore, with common linkage markers, there are cases where marker selection may not be performed because the marker genotype coincides between the resistant and susceptible individuals. However, this time, since the entire nucleotide sequence of the resistance gene has been elucidated, differences in nucleotide sequence could be invariably found between the resistant and susceptible individuals, and development of markers became possible without difficulty. Accordingly, it is thought that there will be less need to perform cultivation that depends on chemosynthetic agrochemicals, and to rely on materials for promoting decrease of clubroot. Specifically, not only can the cost and labor to the farmers be decreased significantly, but continuous cropping will also become possible in the fields, and efficient farm management is expected to become possible. Furthermore, decrease in the use of chemosynthetic agrochemicals is desirable for the health of consumers as well.

## Claims

1. A polynucleotide having clubroot fungus resistance, which is any one of (a) to (d) below:
(a) a polynucleotide encoding a protein comprising the amino acid sequence of SEQ ID NO: 2;
(b) a polynucleotide comprising the coding region of the nucleotide sequence of SEQ ID NO: 1;
(c) a polynucleotide encoding a protein comprising an amino acid sequence with one or more amino acid substitutions, deletions, additions, and/or insertions in the amino acid sequence of SEQ ID NO: 2; and
(d) a polynucleotide that hybridizes under stringent conditions with a complementary strand of the nucleotide sequence of SEQ ID NO: 1.

2. A vector in which the polynucleotide of claim 1 is operably linked downstream of a promoter region that enables expression in a plant cell.

3. A transformed plant cell into which the vector of claim 2 has been introduced.

4. A plant having clubroot fungus resistance activity, which is regenerated from the transformed cell of claim 3.

5. A plant having clubroot fungus resistance activity, which is a progeny or a clone of the plant of claim 4.

6. A propagation material of the plant having clubroot fungus resistance activity of claim 4 or 5.

7. A method for assessing clubroot fungus resistance of a test plant or a test propagation medium, which comprises the step of detecting a DNA region comprising the nucleotide sequence of SEQ ID NO: 1 or SEQ ID NO: 3, or a partial sequence or surrounding sequence of this nucleotide sequence.

8. The assessment method of claim 7, which comprises the following steps of:
(i) preparing a DNA sample from a test plant or a test propagation medium;
(ii) amplifying a DNA region comprising the nucleotide sequence of SEQ ID NO: 1 or SEQ ID NO: 3, or a partial sequence or surrounding sequence of this nucleotide sequence from the DNA sample; and
(iii) comparing the molecular weight or nucleotide sequence of a DNA fragment produced by amplifying the DNA region comprising the nucleotide sequence of SEQ ID NO: 1 or SEQ ID NO: 3, or a partial sequence or surrounding sequence of this nucleotide sequence, from the clubroot fungus-resistant plant or propagation medium with that of the DNA fragment amplified in step (ii).

9. A method of selecting a plant or seed thereof having a clubroot fungus resistance gene by the assessment method of claim 7 or 8.

10. A method for producing a plant or a seed thereof having clubroot fungus resistance activity, which comprises the following steps of:
(i) introducing into a plant cell the vector of claim 2; and
(ii) regenerating a plant from the transformed plant cell into which a vector has been introduced in step (i) mentioned above.

11. A method of conferring clubroot fungus resistance activity to a plant or seed thereof, which comprises the step of expressing the polynucleotide of claim 1 in a plant cell.

12. The method of claim 11, which comprises the step of introducing into a plant cell the polynucleotide of claim 1 or the vector of claim 2.

13. The method of claim 11, which comprises the following steps of:
(a) crossing a plant comprising the polynucleotide of claim 1 with another plant; and
(b) selecting a plant comprising the polynucleotide.

14. The method of any one of claims 7 to 13, wherein the plant is a cruciferous plant.

15. A plant or seed thereof which is obtained by the method of any one of claims 7 to 14.

16. An artificially produced plant or seed thereof, which comprises the polynucleotide of claim 1 and has clubroot fungus resistance activity.

17. The plant or the seed thereof of claim 15 or 16, wherein the plant is a cruciferous plant.

18. A primer for detecting clubroot fungus resistance activity of a test plant, which comprises an oligonucleotide having a chain length of at least 15 nucleotides, and specifically hybridizes under stringent conditions with the nucleotide sequence of SEQ ID NO: 1 or the nucleotide sequence of SEQ ID NO: 3.

19. A probe for detecting clubroot fungus resistance activity of a test plant, which comprises an oligonucleotide having a chain length of at least 15 nucleotides, and specifically hybridizes under stringent conditions with the nucleotide sequence of SEQ ID NO: 1 or the nucleotide sequence of SEQ ID NO: 3.
